(19) Europäisches Patentamt
European Patent Office
Office européen des brevets

(11) EP 1 911 828 B1

(12) **EUROPÄISCHE PATENTSCHRIFT**

(45) Veröffentlichungstag und Bekanntmachung des Hinweises auf die Patenterteilung:
**01.09.2010 Patentblatt 2010/35**

(51) Int Cl.:
***C09K 19/32*** *(2006.01)* ***C09K 19/34*** *(2006.01)*

(21) Anmeldenummer: **07018099.7**

(22) Anmeldetag: **14.09.2007**

(54) **Flüssigkristallanzeige**

Liquid crystal display

Affichage à cristaux liquides

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **12.10.2006 DE 102006048274**

(43) Veröffentlichungstag der Anmeldung:
**16.04.2008 Patentblatt 2008/16**

(73) Patentinhaber: **Merck Patent GmbH**
**64293 Darmstadt (DE)**

(72) Erfinder:
- **Bernatz, Georg, Dr.**
  **64289 Darmstadt (DE)**
- **Taugerbeck, Andreas, Dr.**
  **64285 Darmstadt (DE)**

(56) Entgegenhaltungen:
**EP-A- 1 215 195**
**EP-A- 1 378 557**
**WO-A-02/34739**
**WO-A-02/094805**
**JP-A- 2005 015 473**
**US-A- 5 780 629**
**US-A1- 2003 006 398**
**US-A1- 2006 054 859**
**US-A1- 2006 103 804**
**US-A1- 2007 122 565**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 911 828 B1

## Beschreibung

Die vorliegende Erfindung betrifft eine Flüssigkristallanzeige des PSA-(polymer sustained alignment) Typs, sowie neue Flüssigkristallmedien und neue polymerisierbare Verbindungen zur Verwendung in PSA-Anzeigen.

Die derzeit verwendeten Flüssigkristallanzeigen (FK-Anzeigen) sind meist solche des TN-Typs (twisted nematic). Diese weisen allerdings den Nachteil einer starken Blickwinkelabhängigkeit des Kontrastes auf. Daneben sind sogenannte VA-Anzeigen (vertical alignment) bekannt, die einen breiteren Blickwinkel aufweisen. Die FK-Zelle einer VA-Anzeige enthält eine Schicht eines FK-Mediums zwischen zwei transparenten Elektroden, wobei das FK-Medium üblicherweise einen negativen Wert der dielektrischen (DK-) Anisotropie aufweist. Die Moleküle der FK-Schicht sind im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle parallel zu den Elektrodenflächen statt. Weiterhin sind OCB-Anzeigen (optically compensated bend) bekannt, die auf einem Doppelbrechungseffekt beruhen und eine FK-Schicht mit einer sogenannten "bend"-Orientierung und üblicherweise positiver (DK-) Anisotropie aufweisen. Bei Anlegen einer elektrischen Spannung findet eine Umorientierung der FK-Moleküle senkrecht zu den Elektrodenflächen statt. Darüber hinaus enthalten OCB-Anzeigen normalerweise einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber TN-Anzeigen einen weiteren Blickwinkel und kürzere Schaltzeiten.

In VA-Anzeigen des neueren Typs ist die einheitliche Ausrichtung der FK-Moleküle auf mehrere kleinere Domänen innerhalb der FK-Zelle beschränkt. Zwischen diesen Domänen, auch als Tilt-Domänen (engl. "tilt domains") bezeichnet, existieren Disklinationen. VA-Anzeigen mit Tilt-Domänen weisen, verglichen mit herkömmlichen VA-Anzeigen, eine größere Blickwinkelunabhängigkeit des Kontrastes und der Graustufen auf. Außerdem sind solche Anzeigen einfacher herzustellen, da eine zusätzliche Behandlung der Elektrodenoberfläche zur einheitlichen Orientierung der Moleküle im eingeschalteten Zustand, wie z.B. durch Reiben, nicht mehr notwendig ist. Stattdessen wird die Vorzugsrichtung des Kipp- oder Tiltwinkels (engl. "pretilt") durch eine spezielle Ausgestaltung der Elektroden kontrolliert. In den sogenannten MVA-Anzeigen (multidomain vertical alignment) wird dies üblicherweise dadurch erreicht, dass die Elektroden Erhebungen oder Vorsprünge (engl. "protrusions") aufweisen, die einen lokalen pretilt verursachen. Als Folge werden die FK-Moleküle beim Anlegen einer Spannung in verschiedenen, definierten Regionen der Zelle in unterschiedliche Richtungen parallel zu den Elektrodenflächen orientiert. Dadurch wird ein "kontrolliertes" Schalten erreicht und das Entstehen störender Disklinationslinien vermieden. Diese Anordnung verbessert zwar den Blickwinkel der Anzeige, führt aber zu einer Verringerung ihrer Lichtdurchlässigkeit. Ein Weiterentwicklung von MVA verwendet Protrusions nur auf einer Elektroden-Seite, die gegenüberliegende Elektrode weist hingegen Schlitze (engl. "slits") auf, was die Lichtdurchlässigkeit verbessert. Die geschlitzten Elektroden erzeugen beim Anlegen einer Spannung ein inhomogenes elektrisches Feld in der FK-Zelle, so dass weiterhin ein kontrolliertes Schalten erreicht wird. Zur weiteren Verbesserung der Lichtdurchlässigkeit können die Abstände zwischen den slits und protrusions vergrößert werden, was jedoch wiederum zu einer Verlängerung der Schaltzeiten führt. Beim sogenannten PVA (Patterned VA) kommt man ganz ohne Protrusions aus, indem man beide Elektroden auf den gegenüberliegenden Seiten durch Schlitze strukturiert, was zu einem erhöhten Kontrast und verbesserter Lichtdurchlässigkeit führt, aber technologisch schwierig ist und das Display empfindlicher gegen mechanische Einflüsse macht (Klopfen, engl. "tapping", etc.). Für viele Anwendungen, wie beispielsweise Monitore und vor allem TV-Bildschirme, ist jedoch eine Verkürzung der Schaltzeiten sowie eine Verbesserung des Kontrastes und der Luminanz (Transmission) der Anzeige gefragt.

Eine Weiterentwicklung stellen die sogenannten PSA-Anzeigen (polymer sustained alignment) dar. Darin wird dem FK-Medium eine geringe Menge (zum Beispiel 0.3 Gew.%, typischerweise <1 Gew.%) einer polymerisierbaren Verbindung zugesetzt, welche nach Einfüllen in die FK-Zelle bei angelegter elektrischer Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als "reaktive Mesogene" (RM) bezeichnet, zur FK-Mischung erwiesen. Es sind zwei Anwendungen zu erwähnen, die sogenannten PSA-VA-Anzeigen und PSA-OCB-Anzeigen. Wie man in Testzellen nachweisen kann, führt das PSA-Verfahren zu einem pretilt in der Zelle. Bei PSA-OCB-Anzeigen kann man daher erreichen, dass die Bend-Struktur stabilisiert wird, so dass man ohne Offset-Spannung auskommt oder diese reduzieren kann. Im Falle von PSA-VA-Anzeigen wirkt sich dieser Pretilt positiv auf die Schaltzeiten aus. Für PSA-VA-Anzeigen kann ein Standard-MVA- bzw -PVA Pixel- und Elektroden-Layout verwendet werden. Darüberhinaus kann man aber beispielsweise mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt.

PSA-VA-Anzeigen enthaltend FK-Mischungen mit polymerisierbaren Verbindungen oder RMs sind beispielsweise in JP 10-036847 A, EP 1 170 626 A2, EP 1 378 557 A1, EP 1 498 468 A1, US 2004/0191428 A1, US 2006/0066793 A1 und US 2006/0103804 A1 beschrieben. PSA-OCB-Anzeigen enthaltend FK-Mischungen mit polymerisierten RMs oder Polymeren sind beispielsweise in T.-J- Chen et al., Jpn. J. Appl. Phys. 45, 2006, 2702-2704 und S. H. Kim, L.-C-

Chien, Jpn. J. Appl. Phys. 43, 2004, 7643-7647 beschrieben.

Es hat sich jedoch gezeigt, dass bei Verwendung in PSA-Anzeigen die aus dem Stand der Technik bekannten FK-Mischungen und RMs noch einige Nachteile aufweisen. So eignet sich bei weitem nicht jedes beliebige lösliche Monomer oder RM für PSA-Anzeigen, und es erscheint schwierig, geeignetere Auswahlkriterien als eben das direkte PSA-Experiment mit pretilt-Messung zu finden. Noch kleiner wird die Auswahl, wenn eine Polymerisation mittels UV-Licht ohne den Zusatz von Photoinitiatoren gewünscht ist, was für bestimmte Anwendungen von Vorteil sein kann.

Es besteht somit immer noch ein großer Bedarf nach PSA-Anzeigen, insbesondere vom VA- und OCB-Typ, sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Insbesondere besteht ein großer Bedarf nach PSA-Anzeigen bzw. -Materialien mit einem hohen spezifischen Widerstand bei gleichzeitig großem Arbeitstemperaturbereich, kurzen Schaltzeiten auch bei tiefen Temperaturen und niedriger Schwellenspannung, die eine Vielzahl von Graustufen, einen hohen Kontrast und einen weiten Blickwinkel ermöglichen, sowie hohe Werte der "voltage holding ratio" (HR) nach UV-Belastung aufweisen.

Der Erfindung lag die Aufgabe zugrunde, PSA-Anzeigen bereitzustellen, die die oben angegebenen Nachteile nicht oder nur in geringerem Maße aufweisen, die Einstellung eines Pretilt-Winkels ermöglichen und vorzugsweise gleichzeitig sehr hohe spezifische Widerstände, niedrige Schwellenspannungen und niedrige Schaltzeiten besitzen.

Überraschend wurde nun gefunden, dass diese Aufgabe gelöst werden kann, indem man erfindungsgemäße PSA-Anzeigen verwendet, die eine polymerisierte Verbindung mit einem Biarylstrukturelement der Formel I gemäß Anspruch 1 enthalten. Dies konnte in Verbindung mit einem FK-Medium mittels Pretilt-Messungen in VA-Tilt-Messzellen nachgewiesen werden. Insbesondere konnte ein Pretilt ohne den Zusatz von Photoinitiator erreicht werden.

US 2006/054859 A1, WO 02/94805 A1, WO 02/34739 A, US 2003/006398 A1, JP 2005-015473 A, EP-A-1215195 und US 5,780,629 offenbaren chirale, polymerisierbare Verbindungen mit einem Biarylstrukturelement. Racemate von chiralen polymerisierbaren Verbindungen, deren Verwendung in PSA-Anzeigen oder neue chirale polymerisierbare Verbindungen gemäß den Ansprüchen der vorliegenden Anmeldung werden durch diese Dokumente jedoch weder offenbart noch nahegelegt.

Gegenstand der Erfindung ist somit eine Flüssigkristall (FK-) Anzeige des PSA- (polymer sustained alignment) Typs, enthaltend eine FK-Zelle bestehend aus zwei Substraten, wobei mindestens ein Substrat lichtdurchlässig ist und mindestens ein Substrat eine Elektrodenschicht aufweist, sowie einer zwischen den Substraten befindlichen Schicht eines niedermolekularen FK-Mediums enthaltend eine oder mehrere polymerisierte Verbindungen, wobei die polymerisierte(n) Verbindung(en) erhältlich sind durch Polymerisation einer oder mehrerer polymerisierbarer Verbindungen zwischen den Substraten der FK-Zelle im FKMedium unter Anlegen einer elektrischen Spannung, dadurch gekennzeichnet, dass mindestens eine der polymerisierbaren Verbindungen ein Racemat von Verbindungen enthaltend ein Biarylstrukturelemeht der Formel I ist

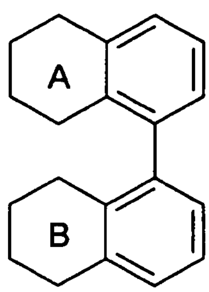

I

weiches an mindestens einer Position, optional über eine organische Gruppe oder eine Abstandsgruppe, mit einer oder mehreren polymerisierbaren Gruppen verknüpft ist, und worin A und B jeweils unabhängig voneinander einen aromatischen oder ganz oder teilweise gesättigten Ring bedeuten, wobei in den einzelnen Ringen auch eine oder mehrere CH-Gruppen durch N und/oder eine oder mehrere $CH_2$-Gruppen durch O und/oder S so ersetzt sein können, dass O- und/oder S-Atome nicht direkt miteinander verknüpft sind, und wobei die einzelnen Ringe auch ein- oder mehrfach substituiert sein können.

Ein weiterer Gegenstand der Erfindung sind neue FK-Medien enthaltend eine oder mehrere polymerisierbare oder polymerisierte Verbindungen oder Racemate gemäß einem oder mehreren den Ansprüche 1 bis 10 und 15 bis 18.

Ein weiterer Gegenstand der Erfindung sind neue polymerisierbare Verbindungen oder Racemate gemäß einem der Ansprüche 15 bis 18.

Die vor- und nachstehend gezeigten Biarylstrukturelemente und diese enthaltenden polymerisierbaren und polymerisierten Verbindungen sind chiral und können sowohl als optisch aktive Form, also als reine Enantiomere oder

als beliebige Mischung beider Enantiomere, als auch als deren Racemat eingesetzt werden. Bevorzugt ist die Verwendung der Racemate. Die Verwendung der Racemate besitzt gegenüber der Verwendung von reinen Enantiomeren einige Vorteile, wie beispielsweise ein deutlich geringerer Syntheseaufwand und geringere Materialkosten.

In den vor- und nachstehend gezeigten Formeln und Unterfomeln ist das Biarylgrundgerüst der Formel I

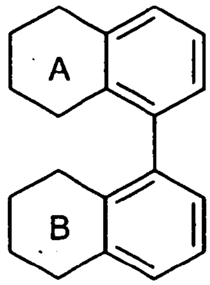

I

vorzugsweise ausgewählt aus folgenden Formeln

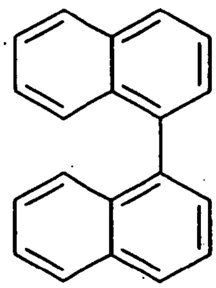

Ia

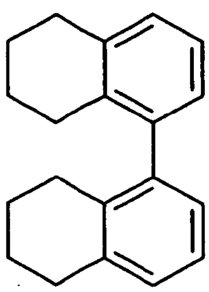

Ib

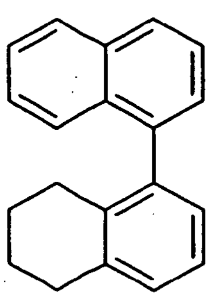

Ic

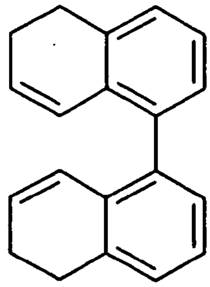

Id

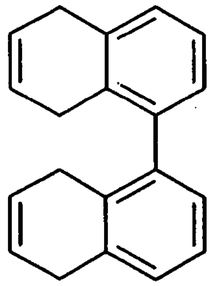

Ie

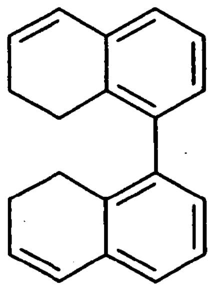

If

Besonders bevorzugt sind Grundgerüste der Formel Ia und Ib.

Besonders bevorzugt sind polymerisierbare Verbindungen enthaltend ein Strukturelement der Formel I, welches an einer oder mehreren Positionen über eine Abstandsgruppe mit einer oder mehreren polymerisierbaren Gruppen verknüpft ist, und vorzugsweise eine oder keine polymerisierbare Gruppe enthält, welche direkt mit dem Strukturelement der Formel I verknüpft ist.

Ferner bevorzugt sind polymerisierbare Verbindungen enthaltend ein Strukturelement der Formel I, welches an lediglich einer Position direkt mit einer polymerisierbaren Gruppe verknüpft ist.

Ferner bevorzugt sind polymerisierbare Verbindungen enthaltend ein Strukturelement der Formel I, welches an zwei Positionen direkt mit einer polymerisierbaren Gruppe verknüpft ist.

Ferner bevorzugt sind polymerisierbare Verbindungen enthaltend ein Strukturelement der Formel I, welches an mehr als zwei Positionen direkt mit einer polymerisierbaren Gruppe verknüpft ist.

In einer bevorzugten Ausführungsform der Erfindung sind die polymerisierbaren Verbindungen ausgewählt aus folgender Formel

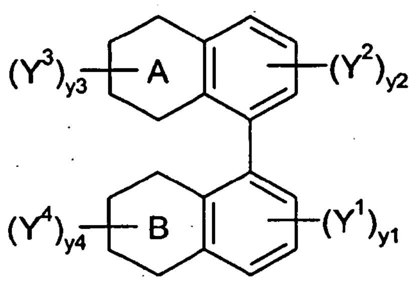

I1

worin

A und B jeweils unabhängig voneinander anelliertes Benzol, Cyclohexan oder Cyclohexen,

$Y^{1-4}$ jeweils unabhängig voneinander H, Halogen, $SF_5$, $NO_2$, einen Kohlenstoffrest oder Kohlenwasserstoffrest, wobei auch einer oder mehrere der Reste $Y^{1-4}$ mit einem benachbarten Rest $Y^{1-4}$ und/oder mit dem Biarylgrundgerüst ein aliphatisches oder aromatisches, mono- oder polycyclisches und gegebenenfalls kondensiertes Ringsystem bilden können, und worin mindestens einer der Reste $Y^{1-4}$ eine polymerisierbare Gruppe P enthält oder bedeutet,

y1, y2 1, 2 oder 3, und

y3, y4 1, 2, 3 oder 4

bedeuten, wobei in jedem der Ringe in Formel I1 auch eine oder mehrere CH-Gruppen durch N und/oder eine oder mehrere $CH_2$-Gruppen-durch O und/oder S so ersetzt sein können, dass O- und/oder S-Atome nicht direkt miteinander verknüpft sind.

Bevorzugte erfindungsgemäße polymerisierte Biarylverbindungen entsprechen den vor- und nachstehend gezeigten Formeln für polymerisierbare Biarylverbindungen, z.B. Formel 11 und deren Unterformeln, worin die polymerisierbare Gruppe P durch die entsprechende, aus der Polymerisationsreaktion entstehende Verknüpfung zur Polymerhauptkette ersetzt ist. So ist beispielsweise eine Gruppe P der Formel $CH_2$=CH-COO- (Acrylat) in der entsprechenden polymerisierten Verbindung durch die Struktur

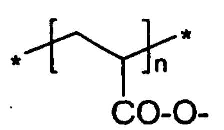

zu ersetzen, wobei n die Zahl der Monomereinheiten (Polymerisationsgrad) bedeutet. Der Einfachheit halber werden im Folgenden lediglich die Formeln für polymerisierbare Verbindungen gezeigt. Die erfindungsgemäßen polymerisierten Biarylverbindungen sollen als von diesen Formeln mitumfasst gelten, ohne dass dies jedesmal explizit genannt ist.

In den vor- und nachstehend angegebenen Formeln gelten folgende Bedeutungen:

Der Begriff "Abstandsgruppe" (engl. "spacer" oder "spacer group"), vor-und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible organische Gruppe, die in einer polymerisierbaren mesogenen Verbindung ("RM") die mesogene Gruppe und die polymerisierbare(n) Gruppe(n) miteinander verbindet.

Der Begriff "mesogene Gruppe" bedeutet eine Gruppe mit der Fähigkeit, flüssigkristallines (FK-) Phasenverhalten zu induzieren. Verbindungen enthaltend mesogene Gruppen müssen nicht notwendigerweise selbst eine FK-Phase aufweisen. Es ist auch möglich, dass sie FK-Phasenverhalten lediglich in Mischungen mit anderen Verbindungen zeigen, oder wenn die mesogenen Verbindungen oder deren Mischungen polymerisiert werden. Der Einfachheit

halber wird der Begriff "Flüssigkristall" vor- und nachstehend sowohl für mesogene als auch für FK-Materialien verwendet. Ein Überblick über die Definitionen findet sich auch in Pure Appl. Chem. 73(5), 888 (2001) und C. Tschierske, G. Pelzl und S. Diele, Angew. Chem. 2004, 116, 6340-6368.

Der Begriff "organische Gruppe" bedeutet eine Kohlenstoff- oder Kohlenwasserstoffgruppe.

Der Begriff "Kohlenstoffgruppe" bedeutet eine ein- oder mehrbindige organische Gruppe enthaltend mindestens ein Kohlenstoffatom, wobei diese entweder keine weiteren Atome enthält (wie z.B. -C≡C-), oder gegebenenfalls ein oder mehrere weitere Atome wie beispielsweise N, O, S, P, Si, Se, As, Te oder Ge enthält (z.B. Carbonyl etc.). Der Begriff "Kohlenwasserstoffgruppe" bedeutet eine Kohlenstoffgruppe, die zusätzlich ein oder mehrere H-Atome und gegebenenfalls ein oder mehrere Heteroatome wie beispielsweise N, O, S, P, Si, Se, As, Te oder Ge enthält.

"Halogen" bedeutet F, Cl, Br oder I.

Eine Kohlenstoff- oder Kohlenwasserstoffgruppe kann eine gesättigte oder ungesättigte Gruppe sein. Ungesättigte Gruppen sind beispielsweise Aryl-, Alkenyl- oder Alkinylgruppen. Ein Kohlenstoff- oder Kohlenwasserstoffrest mit mehr als 3 C-Atomen kann geradkettig, verzweigt und/oder cyclisch sein, und kann auch Spiroverküpfungen oder kondensierte Ringe aufweisen.

Die Begriffe "Alkyl", "Aryl", "Heteroaryl" etc. umfassen auch mehrbindige Gruppen, beispielsweise Alkylen, Arylen, Heteroarylen etc.

Der Begriff "Aryl" bedeutet eine aromatische Kohlenstoffgruppe oder eine davon abgeleitete Gruppe. Der Begriff "Heteroaryl" bedeutet "Aryl" gemäß vorstehender Definition, enthaltend ein oder mehrere Heteroatome.

Bevorzugte Kohlenstoff- und Kohlenwasserstoffgruppen sind gegebenfalls substituiertes Alkyl, Alkenyl, Alkinyl, Alkoxy, Alkylcarbonyl, Alkoxycarbonyl, Alkylcarbonyloxy und Alkoxycarbonyloxy mit 1 bis 40, vorzugsweise 1 bis 25, besonders bevorzugt 1 bis 18 C-Atomen, gegebenfalls substituiertes Aryl oder Aryloxy mit 6 bis 40, vorzugsweise 6 bis 25 C-Atomen, oder gegebenfalls substituiertes Alkylaryl, Arylalkyl, Alkylaryloxy, Arylalkyloxy, Arylcarbonyl, Aryloxycarbonyl, Arylcarbonyloxy und Aryloxycarbonyloxy mit 6 bis 40, vorzugsweise 6 bis 25 C-Atomen.

Weitere bevorzugte Kohlenstoff- und Kohlenwasserstoffgruppen sind $C_1$-$C_{40}$ Alkyl, $C_2$-$C_{40}$ Alkenyl; $C_2$-$C_{40}$ Alkinyl, $C_3$-$C_{40}$ Allyl, $C_4$-$C_{40}$ Alkyldienyl, $C_4$-$C_{40}$ Polyenyl, $C_6$-$C_{40}$ Aryl, $C_6$-$C_{40}$ Alkylaryl, $C_6$-$C_{40}$ Arylalkyl, $C_6$-$C_{40}$ Alkylaryloxy, $C_6$-$C_{40}$ Arylalkyloxy, $C_6$-$C_{40}$ Heteroaryl, $C_4$-$C_{40}$ Cycloalkyl, $C_4$-$C_{40}$ Cycloalkenyl, etc. Besonders bevorzugt sind $C_1$-$C_{22}$ Alkyl, $C_2$-$C_{22}$ Alkenyl, $C_2$ -$C_{22}$ Alkinyl, $C_3$-$C_{22}$ Allyl, $C_4$-$C_{22}$ Alkyldienyl, $C_6$-$C_{12}$ Aryl, $C_6$-$C_{20}$ Arylalkyl und $C_6$-$C_{20}$ Heteroaryl.

Weitere bevorzugte Kohlenstoff- und Kohlenwasserstoffgruppen sind geradkettige, verzweigte oder cyclische Alkylreste mit 1 bis 40, vorzugsweise 1 bis 25 C-Atomen, welche unsubstituiert oder durch F, Cl, Br, I oder CN ein- oder mehrfach substituiert sind, und worin ein mehrere nicht benachbarte $CH_2$-Gruppen jeweils unabhängig voneinander durch-C($R^x$)=C($R^x$)-, -C≡C-, -N($R^x$)-, -O-, -S-, -CO-, -CO-O-, -O-CO-, -O-CO-O-so ersetzt sein können, daß O- und/oder S-Atome nicht direkt miteinander verknüpft sind.

$R^x$ bedeutet H, Halogen, eine geradkettige, verzweigte oder cyclische Alkylkette mit 1 bis 25 C-Atomen, in der auch ein oder mehrere nicht benachbarte C-Atome durch -O-, -S-, -CO-, -CO-O-, -O-CO-, -O-CO-O-ersetzt sein können, wobei auch ein oder mehrere H-Atome durch Fluor ersetzt sein können, oder eine optional substituierte Aryl-, Aryloxy-, Heteroaryl- oder Heteroaryloxygruppe mit 5 bis 40 C-Atomen.

Bevorzugte Alkylgruppen sind beispielsweise Methyl, Ethyl, n-Propyl, Isopropyl, n-Butyl, Isobutyl, s-Butyl, t-Butyl, 2-Methylbutyl, n-Pentyl, s-Pentyl, Cyclopentyl, n-Hexyl, Cyclohexyl, 2-Ethylhexyl, n-Heptyl, Cycloheptyl, n-Octyl, Cyclooctyl, n-Nonyl, n-Decyl, n-Undecyl, n-Dodecyl, Dodecanyl, Trifluoromethyl, Perfluoro-n-butyl, 2,2,2-Trifluoroethyl, Perfluorooctyl, Perfluorohexyl etc.

Bevorzugte Alkenylgruppen sind beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl etc.

Bevorzugte Alkinylgruppen sind beispielsweise Ethinyl, Propinyl, Butinyl, Pentinyl, Hexinyl, Octinyl etc.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Bevorzugte Aminogruppen sind beispielsweise Dimethylamino, Methylamino, Methylphenylamino, Phenylamino, etc.

Arylgruppen können monocyclisch oder polycyclisch sein, d.h. sie können einen Ring (beispielsweise Phenyl) oder zwei oder mehr Ringe aufweisen, welche auch kondensiert (beispielsweise Naphthyl) oder kovalent verknüpft sein können (beispielsweise Biphenyl), oder eine Kombination von kondensierten und verknüpften Ringen beinhalten. Bevorzugt sind vollständig konjugierte Arylgruppen.

Bevorzugte Arylgruppen sind beispielsweise Phenyl, Biphenyl, Triphenyl, [1,1':3',1'']Terphenyl-2'-yl, Naphthyl,

Anthracen, Binaphthyl, Phenanthren, Pyren, Dihydropyren, Chrysen, Perylen, Tetracen, Pentacen, Benzpyren, Fluoren, Inden, Indenofluoren, Spirobifluoren, etc.

Bevorzugte Heteroarylgruppen sind beispielsweise 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzothiophen, Benzothiadiazothiophen, oder Kombinationen dieser Gruppen. Die Heteroarylgruppen können auch mit Alkyl, Alkoxy, Thioalkyl, Fluor, Fluoralkyl oder weiteren Aryl- oder Heteroarylgruppen substituiert sein.

Die Aryl-, Heteroaryl-, Kohlenstoff- und Kohlenwasserstoffreste weisen gegebenfalls einen oder mehrere Substituenten auf, welche vorzugsweise ausgewählt sind aus der Gruppe enthaltend Silyl, Sulfo, Sulfonyl, Formyl, Amin, Imin, Nitril, Mercapto, Nitro, Halogen, $C_{1-12}$ Alkyl, $C_{6-12}$ Aryl, $C_{1-12}$ Alkoxy, Hydroxy, oder Kombinationen dieser Gruppen.

Bevorzugte Substituenten sind beispielsweise löslichkeitsfördernde Gruppen wie Alkyl oder Alkoxy, elektronenziehende Gruppen wie Fluor, Nitro oder Nitril, oder Substituenten zur Erhöhung der Glastemperatur (Tg) im Polymer, insbesondere voluminöse Gruppen wie z.B. t-Butyl oder gegebenfalls substituierte Arylgruppen.

Bevorzugte Substituenten, im Folgenden auch als "L" bezeichnet, sind beispielsweise F, Cl, Br, I, -CN, $-NO_2$, -NCO, -NCS, -OCN, -SCN,$-C(=O)N(R^x)_2$, $-C(=O)Y^1$, $-C(=O)R^x$, $-N(R^x)_2$, worin $R^x$ die oben angegebene Bedeutung hat und $Y^1$ Halogen bedeutet, optional substituiertes Silyl, Aryl mit 4 bis 40, vorzugsweise 6 bis 20 C Atomen, und geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin ein oder mehrere H-Atome gegebenfalls durch F oder Cl ersetzt sein können.

Besonders bevorzugte Substituenten L sind beispielsweise F, Cl, CN, $NO_2$, $CH_3$, $C_2H_5$, $OCH_3$, $OC_2H_5$, $COCH_3$, $COC_2H_5$, $COOCH_3$, $COOC_2H_5$, $CF_3$, $OCF_3$, $OCHF_2$, $OC_2F_5$, ferner Phenyl.

Die polymerisierbare Gruppe P ist eine Gruppe, die für eine Polymerisationsreaktion, wie beispielsweise die radikalische oder ionische Kettenpolymerisation, Polyaddition oder Polykondensation, oder für eine polymeranaloge Umsetzung, beispielsweise die Addition oder Kondensation an eine Polymerhauptkette, geeignet ist. Besonders bevorzugt sind Gruppen für die Kettenpolymerisation, insbesondere solche enthaltend eine C-C-Doppelbindung oder C-C-Dreifachbindung, sowie zur Polymerisation unter Ringöffnung geeignete Gruppen wie beispielsweise Oxetan- oder Epoxygruppen

Bevorzugte Gruppen P sind ausgewählt aus $CH_2=CW^1-COO-$, $CH_2=CW^1-CO-$,

CO-, 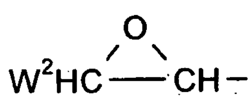 ,

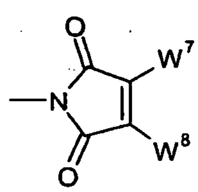 ,

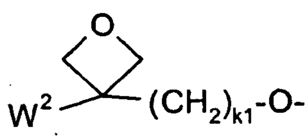 ,

$CH_2=CW^2-(O)_{k1}-$, $CH_3-CH=CH-O-$, $(CH_2=CH)_2CH-OCO-$, $(CH_2=CH-CH_2)_2CH-OCO-$, $(CH_2=CH)_2CH-O-$, $(CH_2=CH-CH_2)_2N-$, $(CH_2=CH-CH_2)_2N-CO-$, $HO-CW^2W^3-$, $HS-CW^2W^3-$, $HW^2N-$, $HO-CW^2W^3-NH-$, $CH_2=CW^1-CO-NH-$,

$CH_2$=CH-$(COO)_{k1}$-Phe-$(O)_{k2}$-, $CH_2$=CH-$(CO)_{k1}$-Phe-$(O)_{k2}$-, Phe-CH=CH-, HOOC-, OCN-, und $W^4W^5W^6$Si-, worin $W^1$ H, F, Cl, CN, $CF_3$, Phenyl or Alkyl mit 1 bis 5 C-Atomen, insbesondere H, Cl oder $CH_3$ bedeutet, $W^2$ und $W^3$ jeweils unabhängig voneinander H oder Alkyl mit 1 bis 5 C-Atomen, insbesondere H, Methyl, Ethyl oder n-Propyl bedeuten, $W^4$, $W^5$ und $W^6$ jeweils unabhängig voneinander Cl, Oxaalkyl oder Oxacarbonylalkyl mit 1 bis 5 C-Atomen bedeuten, $W^7$ und $W^8$ jeweils unabhängig voneinander H, Cl oder Alkyl mit 1 bis 5 C-Atomen bedeuten, Phe 1,4-Phenylen bedeutet, welches optional mit ein oder mehreren Resten L wie oben definiert ist, und $k_1$ und $k_2$ jeweils unabhängig voneinander 0 oder 1 bedeuten.

Besonders bevorzugte Gruppen P sind $CH_2$=CH-COO-, $CH_2$=C($CH_3$)-COO-, $CH_2$=CH-, $CH_2$=CH-O-, $(CH_2=CH)_2$CH-OCO-, $(CH_2=CH)_2$CH-O-,

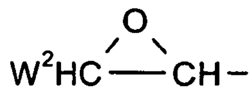

und

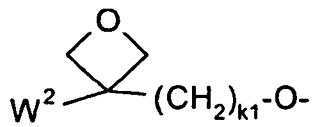
,

insbesondere Vinyl, Acrylat, Methacrylat, Oxetan und Epoxy.

Bevorzugte Abstandsgruppen Sp sind ausgewählt aus der Formel Sp'-X', so daß der Rest "P-Sp-" der Formel "P-Sp'-X'-" entspricht, wobei

| | |
|---|---|
| Sp' | Alkylen mit 1 bis 20, vorzugsweise 1 bis 12 C-Atomen bedeutet, welches optional durch F, Cl, Br, I oder CN ein- oder mehrfach substituiert ist, und worin auch eine oder mehrere nicht benachbarte $CH_2$-Gruppen jeweils unabhängig voneinander so durch -O-, -S-,-NH-, -$NR^0$-, -Si$R^0R^{00}$-, -CO-, -COO-, -OCO-, -OCO-O-, -S-CO-, -CO-S-, -$NR^0$-CO-O-, -O-CO-$NR^0$-, -$NR^0$-CO-$NR^0$-, -CH=CH- oder -C≡C-ersetzt sein können, daß O- und/oder S-Atome nicht direkt miteinander verknüpft sind, |
| X' | -O-, -S-, -CO-, -COO-, -OCO-, -O-COO-, -CO-$NR^0$-, -$NR^0$-CO-, -$NR^0$-CO-$NR^0$-, -$OCH_2$-, -$CH_2O$-, -$SCH_2$-, -$CH_2S$-, -$CF_2O$-, -$OCF_2$-, -$CF_2S$-, -$SCF_2$-, -$CF_2CH_2$-, -$CH_2CF_2$-, -$CF_2CF_2$-, -CH=N-, -N=CH-, -N=N-,-CH=$CR^0$-, -$CY^2$=$CY^3$-, -C≡C-, -CH=CH-COO-, -OCO-CH=CH- oder eine Einfachbindung bedeutet, |
| $R^0$ und $R^{00}$ | jeweils unabhängig voneinander H oder Alkyl mit 1 bis 12 C-Atomen bedeuten, und |
| $Y^2$ und $Y^3$ | jeweils unabhängig voneinander H, F, Cl oder CN bedeuten. |

X' ist vorzugsweise -O-, -S -CO-, -COO-, -OCO-, -O-COO-, -CO-$NR^0$-,-$NR^0$-CO-, -$NR^0$-CO-$NR^0$- oder eine Einfachbindung.

Typische Abstandsgruppen Sp' sind beispielsweise -$(CH_2)_{p1}$-, -$(CH_2CH_2O)_{q1}$-$CH_2CH_2$-, -$CH_2CH_2$-S-$CH_2CH_2$-, -$CH_2CH_2$-NH-$CH_2CH_2$- oder -$(SiR^0R^{00}\text{-}O)_{p1}$-, worin p1 eine ganze Zahl von 1 bis 12 ist, q1 eine ganze Zahl von 1 bis 3 ist, und $R^0$ und $R^{00}$ die oben angegebenen Bedeutungen besitzen.

Besonders bevorzugte Gruppen -X'-Sp'- sind -$(CH_2)_{p1}$-, -O-$(CH_2)_{p1}$-, -OCO-$(CH_2)_{p1}$-, -OCOO-$(CH_2)_{p1}$-.

Besonders bevorzugte Gruppen Sp' sind beispielsweise jeweils geradkettiges Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen und Butenylen.

Besonders bevorzugt sind polymerisierbare Verbindungen der folgenden Formeln

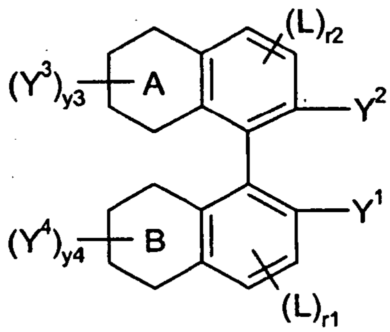

I1a

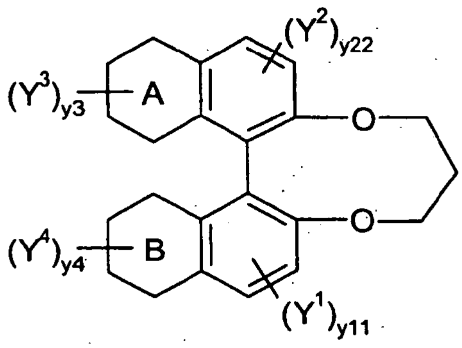

I1b

worin L, A, B, $Y^{1-4}$, y3 und y4 die oben angegebene Bedeutung besitzen, y11 und y22 jeweils unabhängig voneinander 0 oder 1 bedeuten, und r1 und r2 jeweils unabhängig voneinander 0, 1 oder 2 bedeuten, und $Y^1$ und $Y^2$ vorzugsweise eine polymerisierbare Gruppe enthalten oder bedeuten.

Besonders bevorzugte Verbindungen der Formeln 11a und 11b sind solche der folgenden Unterformeln

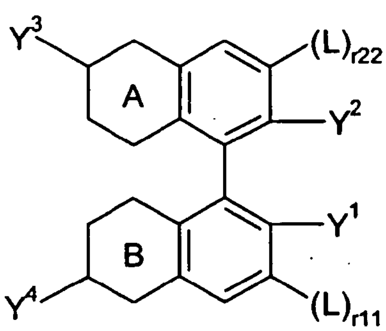

I1a1

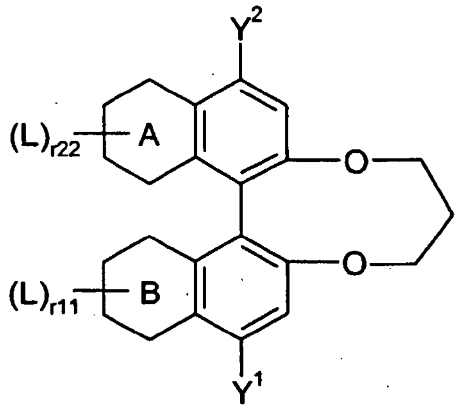

I1b1

worin $Y^{1,2}$, L, A und B die oben angegebene Bedeutung besitzen und r11 und r22 jeweils unabhängig voneinander 0 oder 1 bedeuten.

Besonders bevorzugt sind Verbindungen der Formel I1, I1a, I1a1, I1b, I1b1 und deren Unterformeln, worin einer oder mehrere der Reste $Y^{1-4}$-W-($Z^1$-$A^1$)$_{m1}$-Sp-P bedeuten, worin

W O, S, $CH_2$ oder eine Einfachbindung,

Sp eine Abstandsgruppe oder eine Einfachbindung,

P eine polymerisierbare Gruppe,

$A^1$ bei jedem Auftreten gleich oder verschieden Aryl, Heteroaryl oder ganz oder teilweise gesättigtes Cycloalkyl mit 4 bis 20 C-Atomen, welches optional substituiert ist und worin auch eine oder mehrere CH-Gruppen durch N und/oder eine oder mehrere $CH_2$-Gruppen durch O und/oder S so ersetzt sein können, dass O- und/oder S-Atome nicht direkt miteinander verknüpft sind,

$Z^1$ bei jedem Auftreten gleich oder verschieden -O-, -S-, -CO-,-COO-, -OCO-, -O-COO-, $-OCH_2-$, $-CH_2O-$, $-SCH_2-$, $-CH_2S-$,$-CF_2O-$, $-OCF_2-$, $-CF_2S-$, $-SCF_2-$, $-CH_2CH_2-$, $-CF_2CH_2-$,$-CH_2CF_2-$, $-CF_2CF_2-$, -CH=CH-, -CF=CF-, -C≡C-, -CH=CH-COO-, -OCO-CH=CH-, $CR^0R^{00}$ oder eine Einfachbindung,

$R^0$ und $R^{00}$ jeweils unabhängig voneinander H oder Alkyl mit 1 bis 4 C-Atomen, und

m1 0, 1, 2, 3 oder 4

bedeuten.

Ferner bevorzugt sind Verbindungen der Formel I1, I1a, I1a1, I1b, I1b1 und deren Unterformeln, worin einer oder mehrere der Reste $Y^{1-4}$ -W-($Z^1$-$A^1$)$_{m1}$-$R^y$ bedeuten, worin W, $Z^1$, $A^1$ und m1 die oben angegebene Bedeutung besitzen und

$R^y$ bei jedem Auftreten gleich oder verschieden H, L wie oben definiert, geradkettiges oder verzweigtes Alkyl mit 1 bis 25 C-Atomen, welches unsubstituiert oder durch F, Cl, Br, I, CN oder P-Sp- ein- oder mehrfach substituiert ist, und worin ein mehrere nicht benachbarte $CH_2$-Gruppen jeweils unabhängig voneinander durch -C($R^x$)=C($R^x$)-,-C≡C-, -N($R^x$)-, -O-, -S-, -CO-, -CO-O-, -O-CO-, -O-CO-O- so ersetzt sein können, dass O- und/oder S-Atome nicht direkt miteinander verknüpft sind, wobei $R^x$ die oben angegebene Bedeutung hat,

bedeutet, und, falls alle Reste $Y^{1-4}$ -W-($Z^1$-$A^1$)$_{m1}$-$R^y$ bedeuten, einer oder mehrere der Reste $R^y$ mindestens eine Gruppe P-Sp- aufweisen.

Die Gruppen -($Z^1$-$A^1$)$_{m1}$- sind vorzugsweise aus folgenden Formeln ausgewählt

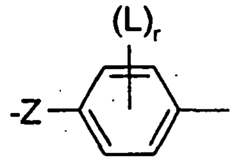

Ia

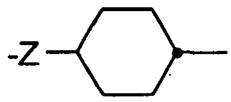

Ib

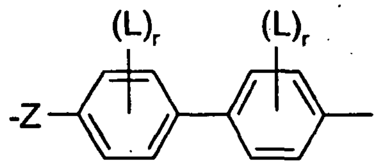

Id

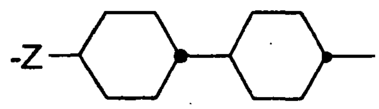

Ie

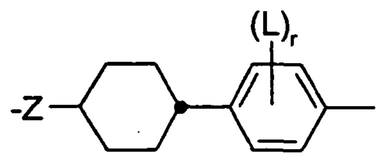

If

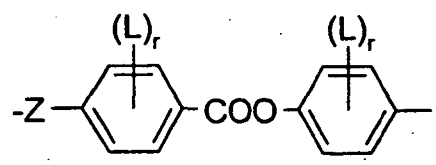

Ig

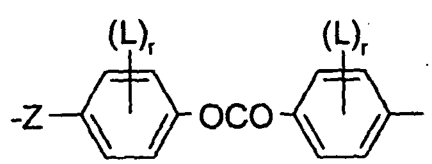

Ih

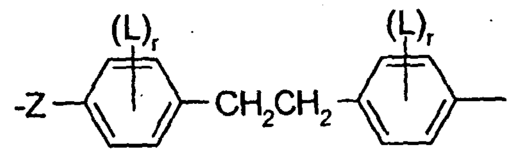

Ii

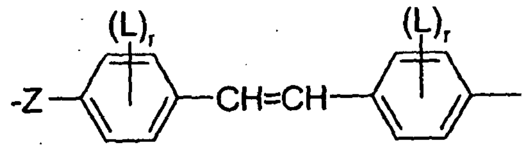

Ik

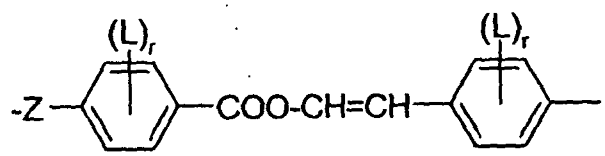

Im

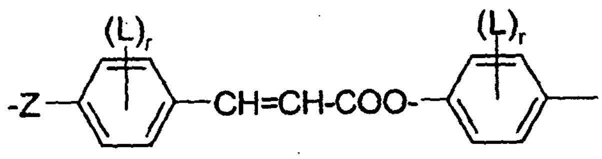

In

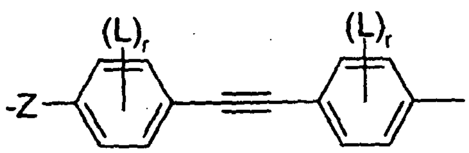

Io

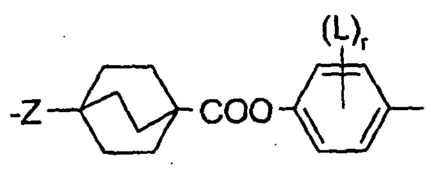

Ip

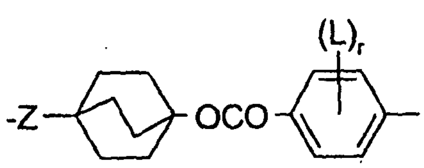

Iq

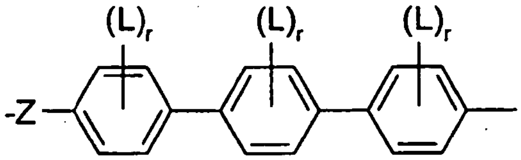

Ir

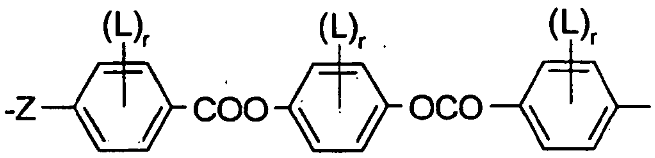

Is

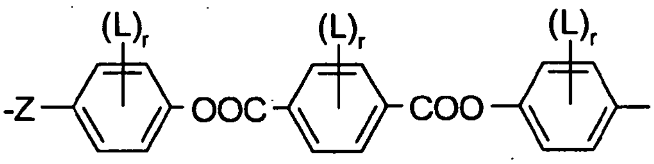

It

worin Z eine der für $Z^1$ angegebenen Bedeutungen und L die oben angegebene Bedeutung besitzen, und r 0, 1, 2, 3 oder 4, vorzugsweise 0, 1 oder 2 bedeutet. Besonders bevorzugt bedeutet Z OCO, $OCH_2$, $OCF_2$, $CH_2CH_2$, CH=CH, C≡C, oder eine Einfachbindung; L ist vorzugsweise F.

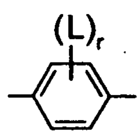

worin $r \neq 0$ ist, bedeutet vorzugsweise

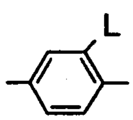 , 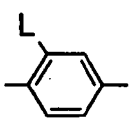 , 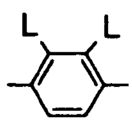 , 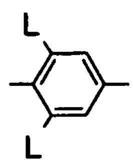

oder

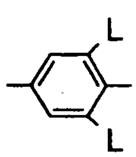 ,

worin L bei jedem Auftreten gleich oder verschieden eine der oben angegebenen Bedeutungen hat.

Ferner bevorzugt sind Verbindungen der Formel I1, I1a, I1a1, I1b, I1b1 und deren Unterformeln, worin

- $Y^1$ und $Y^2$ jeweils unabhängig voneinander eine der für $-W-(Z^1-A^1)_{m1}-Sp-P$ oder $-W-(Z^1-A^1)_{m1}-R^y$ angegebenen Bedeutungen besitzen,

- $Y^1$ und $Y^2$ identische Reste sind,
- $Y^1$ und $Y^2$ P-Sp- bedeuten,
- $Y^1$ und $Y^2$ P- bedeuten,
- $Y^1$ und/oder $Y^2$ -W-$(Z^1\text{-}A^1)_{m1}$-Sp-P bedeuten, worin m1 > 0 ist,
- $Y^1$ und/oder $Y^2$ -W-$(Z^1\text{-}A^1)_{m1}$-$R^y$ bedeuten, worin m1 > 0 ist,
- $Y^1$ und/oder $Y^2$ $R^y$ bedeuten,
- $Y^3$ und/oder $Y^4$ H bedeuten,
- $Y^3$ und $Y^4$ jeweils unabhängig voneinander eine der für -W-$(A^1\text{-}Z^1)_{m1}$-Sp-P oder -W-$(Z^1\text{-}A^1)_{m1}$-$R^y$ angegebenen Bedeutungen besitzen,
- $Y^3$ und $Y^4$ identische Reste sind,
- $Y^3$ und $Y^4$ P-Sp- bedeuten,
- $Y^3$ und $Y^4$ P- bedeuten,
- $Y^3$ und/oder $Y^4$ -W-$(Z^1\text{-}A^1)_{m1}$-Sp-P bedeuten, worin m1 > 0 ist,
- $Y^3$ und/oder $Y^4$ -W-$(Z^1\text{-}A^1)_{m1}$-$R^y$ bedeuten, worin m1 > 0 ist,
- $Y^3$ und/oder $Y^4$ $R^y$ bedeuten,
- einer oder mehrere Reste $Y^{1-4}$ eine der für -W-$(Z^1\text{-}A^1)_{m1}$-$R^y$ angegebenen Bedeutungen besitzen, worin einer oder mehrere der Reste $R^y$ zwei- oder mehrfach durch P-Sp- substituiert sind,
- $Y^1$ und $Y^2$ P, vorzugsweise Acrylat oder Methacrylat, bedeuten und $Y^3$ und $Y^4$ H oder Aryl, welches optional mit L ein- oder mehrfach substituiert ist, vorzugsweise H oder unsubstituiertes Phenyl, bedeuten,
- y3 und y4 jeweils 1 ist,
- m1 0 ist,
- m1 1 oder 2 ist,
- W eine Einfachbindung bedeutet,
- Sp eine Einfachbindung bedeutet,
- Sp -O-$(CH_2)_{p1}$-, -OCO-$(CH_2)_{p1}$- oder -OCOO-$(CH_2)_{p1}$- bedeutet und p1 eine ganze Zahl von 2 bis 12, vorzugsweise 3 oder 6 bedeutet,
- P Acrylat oder Methacrylat bedeutet,
- L F bedeutet,
- A und B einen Benzolring bedeuten,
- A und B einen Cyclohexanring bedeuten,
- y11 und y22 1 bedeuten,

- r1 und r2 0 bedeuten,
- r11 und r22 0 bedeuten,
- r11 und r22 1 bedeuten und L eine der oben angegebenen Bedeutungen hat und besonders bevorzugt Methyl oder Phenyl bedeutet.

Eine bevorzugte Ausführungsform der Erfindung richtet sich auf Verbindungen der Formel I1, I1a, I1a1, I1b, I1b1 und deren Unterformeln, worin einer oder mehrere der Reste $Y^{1-4}$, vorzugsweise die Reste $Y^1$ und $Y^2$, mit einem benachbarten Rest $Y^{1-4}$ oder mit dem Biarylgrundgerüst ein aliphatisches oder aromatisches, mono- oder polycyclisches und gegebenenfalls kondensiertes Ringsystem bilden. Solche Verbindungen sind beispielsweise in EP 1 249 483 A1, WO 02/034739 A1, WO 02/006195 A1 und WO 02/094805 A1 beschrieben.

Besonders bevorzugt sind jedoch Verbindungen der Formel I1, I1a, I1a1, I1b, I1b1 und deren Unterformeln, worin einer oder mehrere der Reste $Y^{1-4}$ , vorzugsweise mindestens die Reste $Y^1$ und $Y^2$, besonders bevorzugt alle Reste $Y^{1-4}$, endständige Gruppen bedeuten, d.h. miteinander oder mit dem Biarylgrundgerüst kein Ringsystem bilden.

In einer weiteren bevorzugten Ausführungsform der Erfindung enthalten die Verbindungen der Formel I1, I1a, I1a1, I1b, I1b1 und deren Unterformeln einen oder mehrere verzweigte Reste R mit zwei oder mehreren polymerisierbaren Gruppen P (multifunktionelle polymerisierbare Reste). Geeignete Reste dieses Typs, sowie diese enthaltende Binaphthylverbindungen, sind beispielsweise in US 7,060,200 B1 oder US 2006/0172090 A1 beschrieben. Besonders bevorzugt sind multifunktionelle polymerisierbare Reste R ausgewählt aus folgenden Formeln

$$\text{-X-alkyl-CHP}^1\text{-CH}_2\text{-CH}_2\text{P}^2 \qquad \text{I*a}$$

$$\text{-X-alkyl-C(CH}_2\text{P}^1\text{)(CH}_2\text{P}^2\text{)-CH}_2\text{P}^3 \qquad \text{I*b}$$

$$\text{-X-alkyl-CHP}^1\text{CHP}^2\text{-CH}_2\text{P}^3 \qquad \text{I*c}$$

$$\text{-X-alkyl-C(CH}_2\text{P}^1\text{)(CH}_2\text{P}^2\text{)-C}_{aa}\text{H}_{2aa+1} \qquad \text{I*d}$$

$$\text{-X-alkyl-CHP}^1\text{-CH}_2\text{P}^2 \qquad \text{I*e}$$

$$\text{-X-alkyl-CHP}^1\text{P}^2 \qquad \text{I*f}$$

$$\text{-X-alkyl-CP}^1\text{P}^2\text{-C}_{aa}\text{H}_{2aa+1} \qquad \text{I*g}$$

$$\text{-X-alkyl-C(CH}_2\text{P}^1\text{)(CH}_2\text{P}^2\text{)-CH}_2\text{OCH}_2\text{-C(CH}_2\text{P}^3\text{)(CH}_2\text{P}^4\text{)CH}_2\text{P}^5 \qquad \text{I*h}$$

$$\text{-X-alkyl-CH((CH}_2\text{)}_{aa}\text{P}^1\text{)((CH}_2\text{)}_{bb}\text{P}^2\text{)} \qquad \text{I*i}$$

$$\text{-X-alkyl-CHP}^1\text{CHP}^2\text{-C}_{aa}\text{H}_{2aa+1} \qquad \text{I*k}$$

worin

| | |
|---|---|
| alkyl | eine Einfachbindung oder geradkettiges oder verzweigtes Alkylen mit 1 bis 12 C-Atomen bedeutet, worin eine oder mehrere nicht benachbarte $CH_2$-Gruppen jeweils unabhängig voneinander durch $-C(R^x)=C(R^x)-$, -C≡C-, $-N(R^x)-$, -O-, -S-, -CO-, -CO-O-, -O-CO-, -O-CO-O- so ersetzt sein können, dass O-und/oder S-Atome nicht direkt miteinander verknüpft sind, und worin auch ein oder mehrere H-Atome durch F, Cl oder CN ersetzt sein können, wobei $R^x$ die oben angegebene Bedeutung hat und vorzugsweise $R^0$ wie oben definiert bedeutet, |
| aa und bb | jeweils unabhängig voneinander 0, 1, 2, 3, 4, 5 oder 6 bedeuten, |
| X | eine der für X' angegebenen Bedeutungen besitzt, und |
| $P^{1-5}$ | jeweils unabhängig voneinander eine der oben für P angegebenen Bedeutungen besitzen. |

Besonders bevorzugte Verbindungen der Formel I1a1 sind solche der folgenden Unterformeln

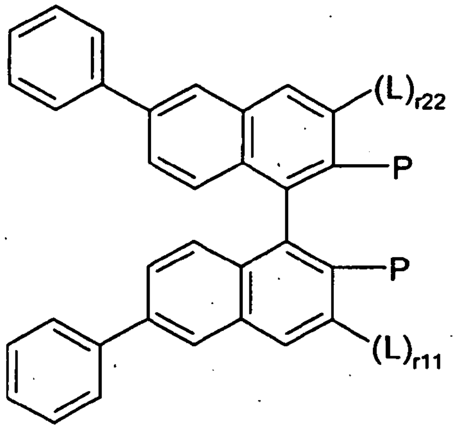

I1a1a

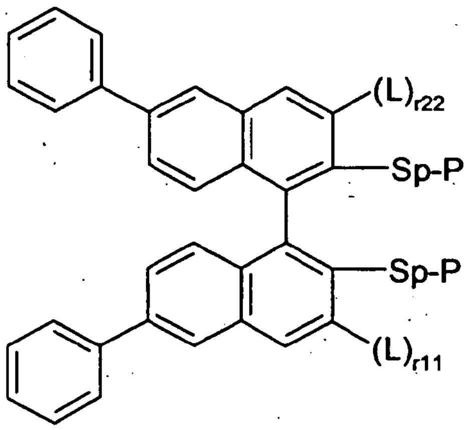

I1a1b

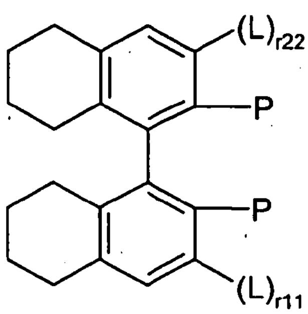

I1a1c

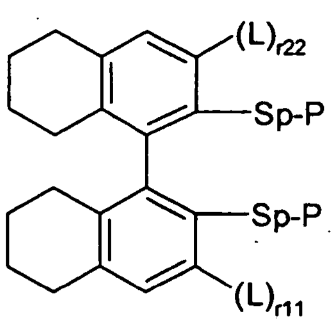

I1a1d

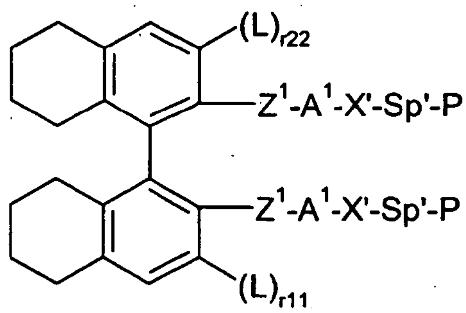

I1a1e

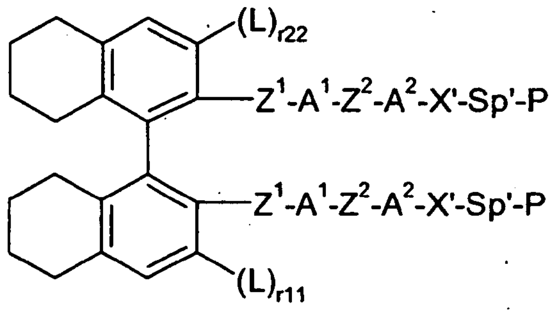

I1a1f

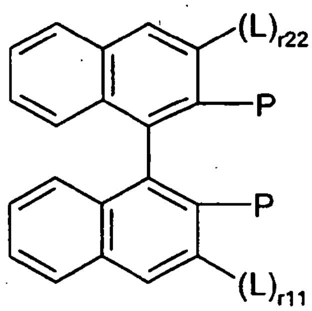

I1a1g

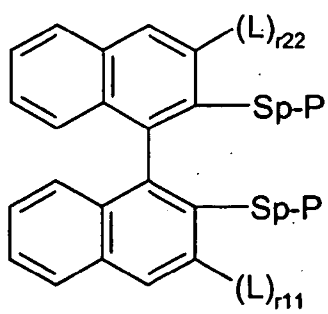

I1a1h

worin $Y^3$, $Y^4$, X', Sp', P, $Z^1$, $A^1$, L, r11 und r22 bei jedem Auftreten gleich oder verschieden die oben angegebenen Bedeutungen besitzen und $Z^2$ und $A^2$ eine der für $Z^1$ bzw. $A^1$ angegebenen Bedeutungen besitzen. Die Gruppen $-Z^1-A^1-$ und $-Z^1-A^1-Z^2-A^2-$ sind vorzugsweise aus den Formeln Ia-Iq ausgewählt.

Besonders bevorzugte Verbindungen der Formel I1b1 sind solche der folgenden Unterformeln

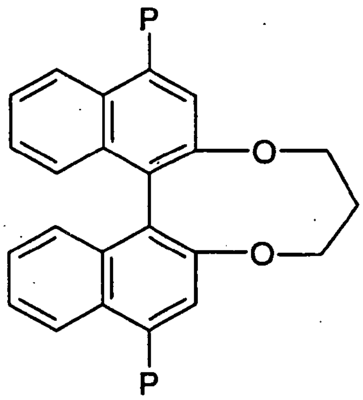

I1b1a

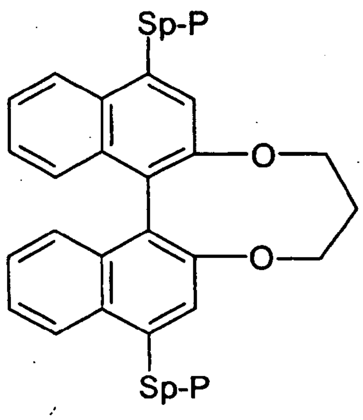

I1b1b

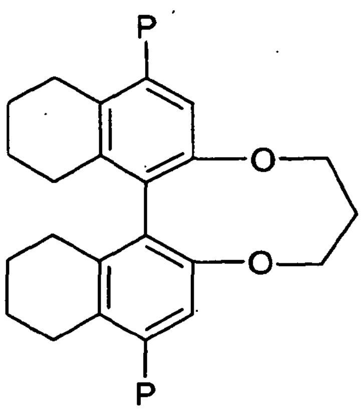

I1b1c

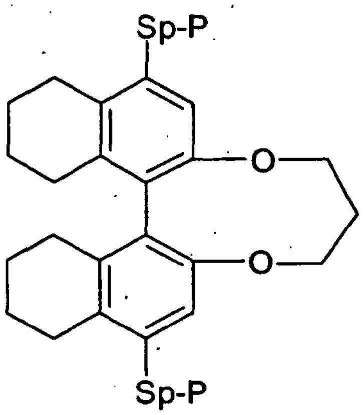

I1b1d

worin Sp und P bei jedem Auftreten gleich oder verschieden die oben angegebenen Bedeutungen besitzen.

Beispiele für besonders bevorzugte Verbindungen der Formel I1a1e und I1a1f sind in den folgenden Tabellen dargestellt (r11 = r22 = 0).

Formel I1a1e:

| Nr. | $Z^1$ | $A^1$ | X' | Sp' | P |
|---|---|---|---|---|---|
| 1 | 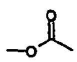 | 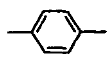 | O | $-C_3H_6-$ | 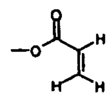 |
| 2 | 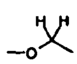 | 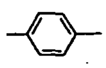 | O | $-C_3H_6-$ | 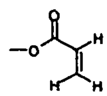 |
| 3 | 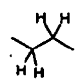 | 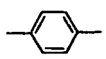 | O | $-C_3H_6-$ | 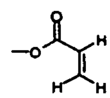 |
| 4 | 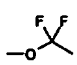 | 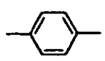 | O | $-C_3H_6-$ | 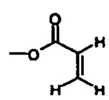 |
| 5 | 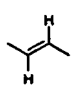 | 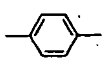 | O | $-C_3H_6-$ | 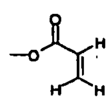 |
| 6 |  | 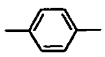 | O | $-C_3H_6-$ | 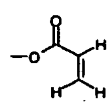 |
| 7 | 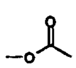 | 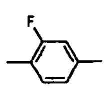 | O | $-C_3H_6-$ | 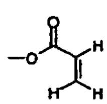 |

(fortgesetzt)

| Nr. | $Z^1$ | $A^1$ | X' | Sp' | P |
|---|---|---|---|---|---|
| 8 | 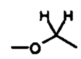 | 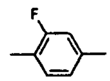 | O | $-C_3H_6-$ | 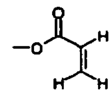 |
| 9 | 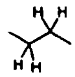 | 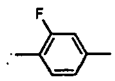 | O | $-C_3H_6-$ | 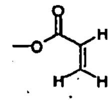 |
| 10 | 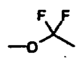 | 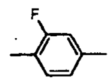 | O | $-C_3H_6-$ | 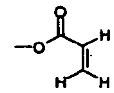 |
| 11 | 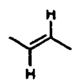 | 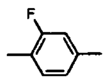 | O | $-C_3H_6-$ | 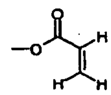 |
| 12 |  | 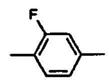 | O | $-C_3H_6-$ | 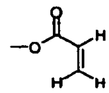 |
| 13 | 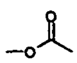 | 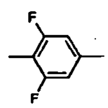 | O | $-C_3H_6-$ | 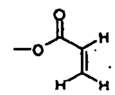 |
| 14 | 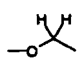 | 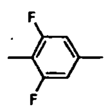 | O | $-C_3H_6-$ | 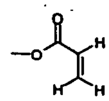 |
| 15 | 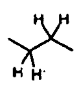 | 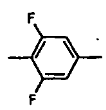 | O | $-C_3H_6-$ | 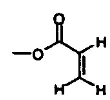 |
| 16 | 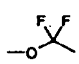 | 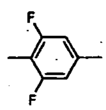 | O | $-C_3H_6-$ | 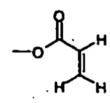 |
| 17 | 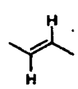 | 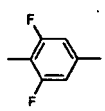 | O | $-C_3H_6-$ | 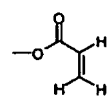 |
| 18 | 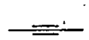 | 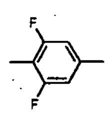 | O | $-C_3H_6-$ | 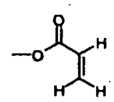 |
| 19 | 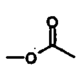 | 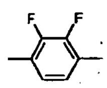 | O | $-C_3H_6-$ | 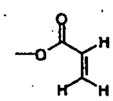 |
| 20 | 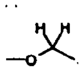 | 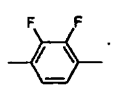 | O | $-C_3H_6-$ | 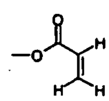 |

(fortgesetzt)

| Nr. | $Z^1$ | $A^1$ | X' | Sp' | P |
|---|---|---|---|---|---|
| 21 | 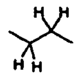 | 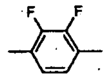 | O | $-C_3H_6-$ | 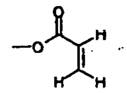 |
| 22 | 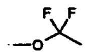 | 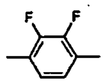 | O | $-C_3H_6-$ | 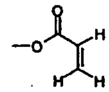 |
| 23 | 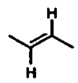 | 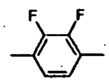 | O | $-C_3H_6-$ | 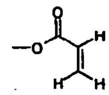 |
| 24 |  | 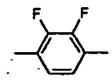 | O | $-C_3H_6-$ | 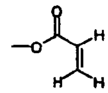 |
| 25 | 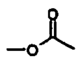 | 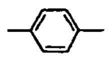 | O | $-C_6H_{12}-$ | 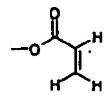 |
| 26 | 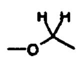 | 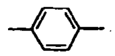 | O | $-C_6H_{12}-$ | 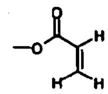 |
| 27 | 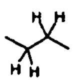 | 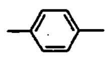 | O | $-C_6H_{12}-$ | 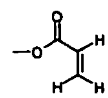 |
| 28 | 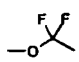 | 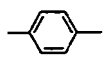 | O | $-C_6H_{12}-$ | 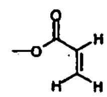 |
| 29 | 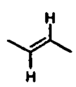 | 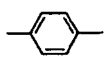 | O | $-C_6H_{12}-$ | 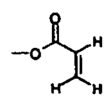 |
| 30 | 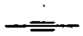 | 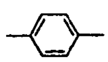 | O | $-C_6H_{12}-$ | 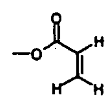 |
| 31 | 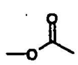 | 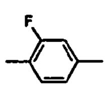 | O | $-C_6H_{12}-$ | 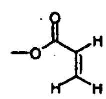 |
| 32 | 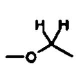 | 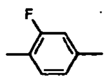 | O | $-C_6H_{12}-$ | 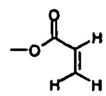 |
| 33 | 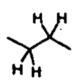 | 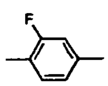 | O | $-C_6H_{12}-$ | 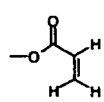 |

(fortgesetzt)

| Nr. | $Z^1$ | $A^1$ | X' | Sp' | P |
|---|---|---|---|---|---|
| 34 | 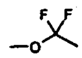 | 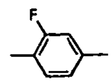 | O | $-C_6H_{12}-$ | 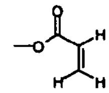 |
| 35 | 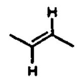 | 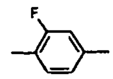 | O | $-C_6H_{12}-$ | 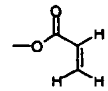 |
| 36 |  | 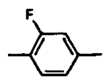 | O | $-C_6H_{12}-$ | 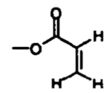 |
| 37 | 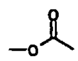 | 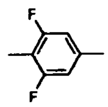 | O | $-C_6H_{12}-$ | 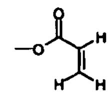 |
| 38 | 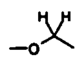 | 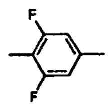 | O | $-C_6H_{12}-$ | 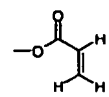 |
| 39 | 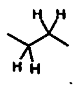 | 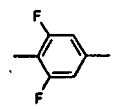 | O | $-C_6H_{12}-$ | 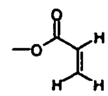 |
| 40 | 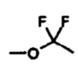 | 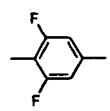 | O | $-C_6H_{12}-$ | 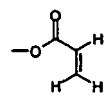 |
| 41 | 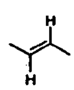 | 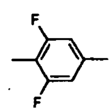 | O | $-C_6H_{12}-$ | 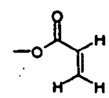 |
| 42 |  | 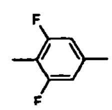 | O | $-C_6H_{12}-$ | 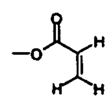 |
| 43 | 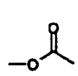 | 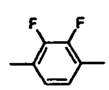 | O | $-C_6H_{12}-$ | 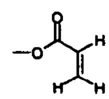 |
| 44 | 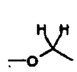 | 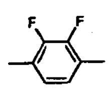 | O | $-C_6H_{12}-$ | 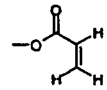 |
| 45 | 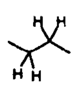 | 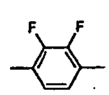 | O | $-C_6H_{12}-$ | 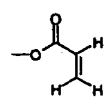 |

(fortgesetzt)

| Nr. | $Z^1$ | $A^1$ | X' | Sp' | P |
|---|---|---|---|---|---|
| 46 | 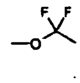 | 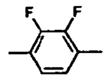 | O | $-C_6H_{12}-$ | 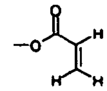 |
| 47 | 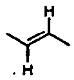 | 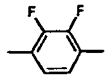 | O | $-C_6H_{12}-$ | 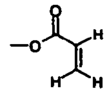 |
| 48 |  | 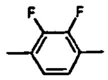 | O | $-C_6H_{12}-$ | 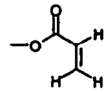 |
| 49 | 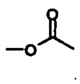 | 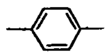 | O | $-C_3H_6-$ | 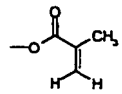 |
| 50 | 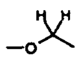 | 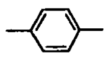 | O | $-C_3H_6-$ | 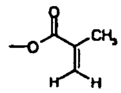 |
| 51 | 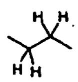 | 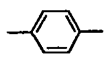 | O | $-C_3H_6-$ | 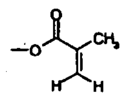 |
| 52 | 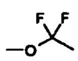 | 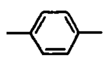 | O | $-C_3H_6-$ | 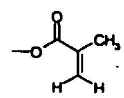 |
| 53 | 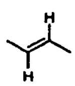 | 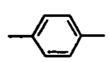 | O | $-C_3H_6-$ | 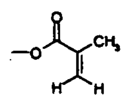 |
| 54 |  | 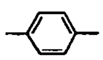 | O | $-C_3H_6-$ | 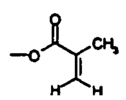 |
| 55 | 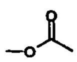 | 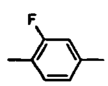 | O | $-C_3H_6-$ | 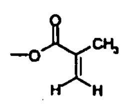 |
| 56 | 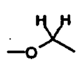 | 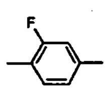 | O | $-C_3H_6-$ | 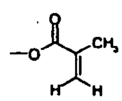 |
| 57 | 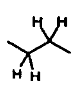 | 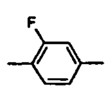 | O | $-C_3H_6-$ | 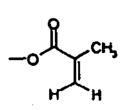 |
| 58 | 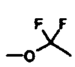 | 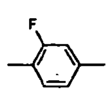 | O | $-C_3H_6-$ | 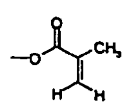 |

(fortgesetzt)

| Nr. | $Z^1$ | $A^1$ | X' | Sp' | P |
|---|---|---|---|---|---|
| 59 | 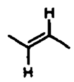 | 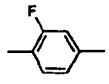 | ○ | $-C_3H_6-$ | 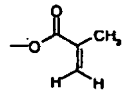 |
| 60 |  | 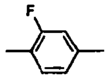 | ○ | $-C_3H_6-$ | 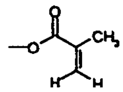 |
| 61 | 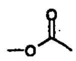 | 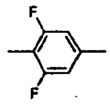 | ○ | $-C_3H_6-$ | 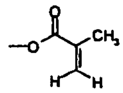 |
| 62 | 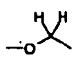 | 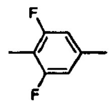 | ○ | $-C_3H_6-$ | 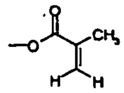 |
| 63 | 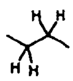 | 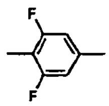 | ○ | $-C_3H_6-$ | 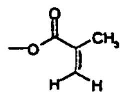 |
| 64 | 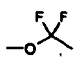 | 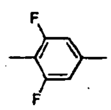 | ○ | $-C_3H_6-$ | 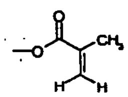 |
| 65 | 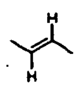 | 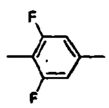 | ○ | $-C_3H_6-$ | 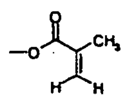 |
| 66 |  | 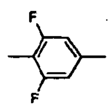 | ○ | $-C_3H_6-$ | 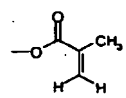 |
| 67 | 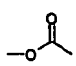 | 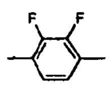 | ○ | $-C_3H_6-$ | 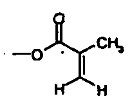 |
| 68 | 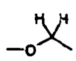 | 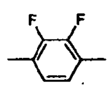 | ○ | $-C_3H_6-$ | 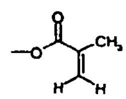 |
| 69 | 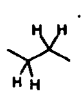 | 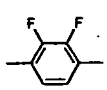 | O | $-C_3H_6-$ | 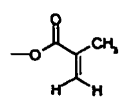 |
| 70 | 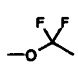 | 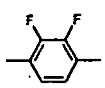 | O | $-C_3H_6-$ | 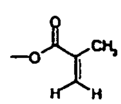 |
| 71 | 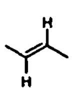 | 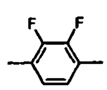 | O | $-C_3H_6-$ | 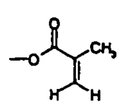 |

(fortgesetzt)

| Nr. | $Z^1$ | $A^1$ | X' | Sp' | P |
|---|---|---|---|---|---|
| 72 |  | 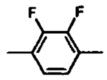 | O | $-C_3H_6-$ | 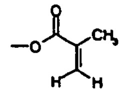 |
| 73 | 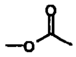 | 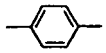 | O | $-C_6H_{12}-$ | 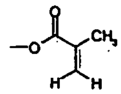 |
| 74 | 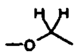 | 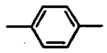 | O | $-C_6H_{12}-$ | 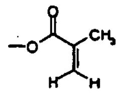 |
| 75 | 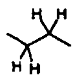 | 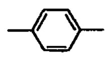 | O | $-C_6H_{12}-$ | 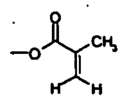 |
| 76 | 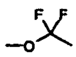 | 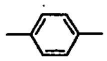 | O | $-C_6H_{12}-$ | 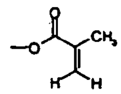 |
| 77 | 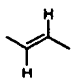 | 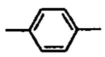 | O | $-C_6H_{12}-$ | 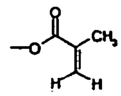 |
| 78 |  | 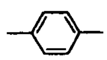 | O | $-C_6H_{12}-$ | 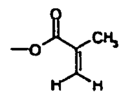 |
| 79 | 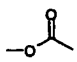 | 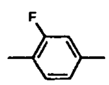 | O | $-C_6H_{12}-$ | 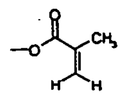 |
| 80 | 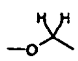 | 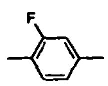 | O | $-C_6H_{12}-$ | 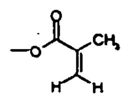 |
| 81 | 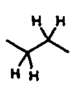 | 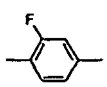 | O | $-C_6H_{12}-$ | 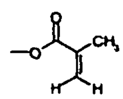 |
| 82 | 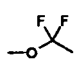 | 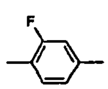 | O | $-C_6H_{12}-$ | 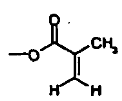 |
| 83 | 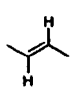 | 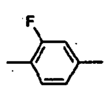 | O | $-C_6H_{12}-$ | 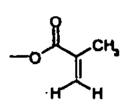 |
| 84 |  | 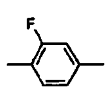 | O | $-C_6H_{12}-$ | 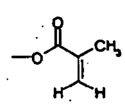 |

(fortgesetzt)

| Nr. | $Z^1$ | $A^1$ | X' | Sp' | P |
|---|---|---|---|---|---|
| 85 | 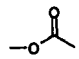 | 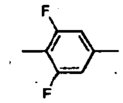 | O | $-C_6H_{12}-$ | 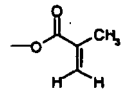 |
| 86 | 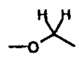 | 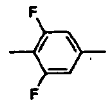 | O | $-C_6H_{12}-$ | 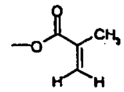 |
| 87 | 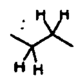 | 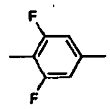 | O | $-C_6H_{12}-$ | 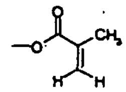 |
| 88 | 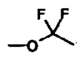 | 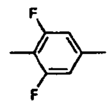 | O | $-C_6H_{12}-$ | 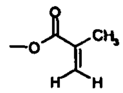 |
| 89 | 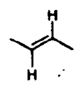 | 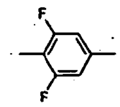 | O | $-C_6H_{12}-$ | 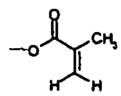 |
| 90 | 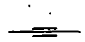 | 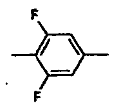 | O | $-C_6H_{12}-$ | 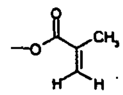 |
| 91 | 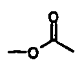 | 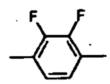 | O | $-C_6H_{12}-$ | 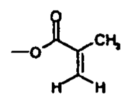 |
| 92 | 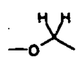 | 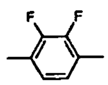 | O | $-C_6H_{12}-$ | 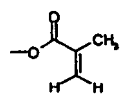 |
| 93 | 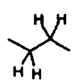 | 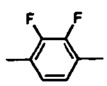 | O | $-C_6H_{12}-$ | 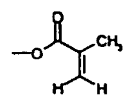 |
| 94 | 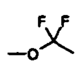 | 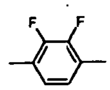 | O | $-C_6H_{12}-$ | 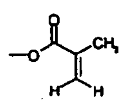 |
| 95 | 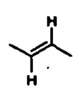 | 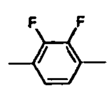 | O | $-C_6H_{12}-$ | 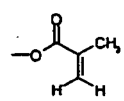 |
| 96 |  | 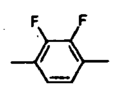 | O | $-C_6H_{12}-$ | 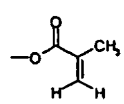 |

Formel I1a1f:

| Nr. | $Z^1$ | $A^1$ | $Z^2$ | $A^2$ | X' | Sp' | P |
|---|---|---|---|---|---|---|---|
| 97 | 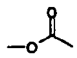 | 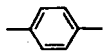 | - | 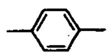 | O | $-C_3H_6-$ | 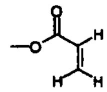 |
| 98 | 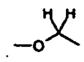 | 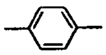 | - | 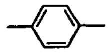 | O | $-C_3H_6-$ | 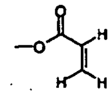 |
| 99 | 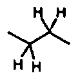 | 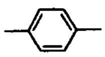 | - | 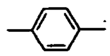 | O | $-C_3H_6-$ | 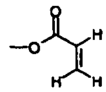 |
| 100 | 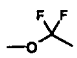 | 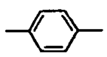 | - | 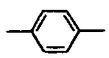 | O | $-C_3H_6-$ | 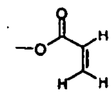 |
| 101 | 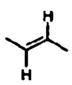 | 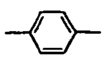 | - | 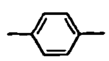 | O | $-C_3H_6-$ | 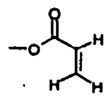 |
| 102 |  | 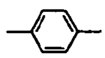 | - | 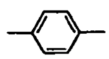 | O | $-C_3H_6-$ | 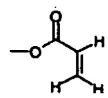 |
| 103 | 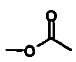 | 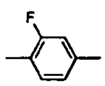 | - | 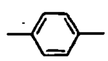 | O | $-C_3H_6-$ | 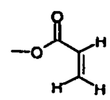 |
| 104 | 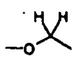 | 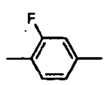 | - | 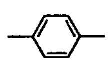 | O | $-C_3H_6-$ | 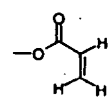 |
| 105 | 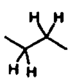 | 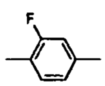 | - | 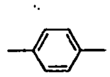 | O | $-C_3H_6-$ | 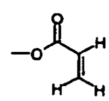 |
| 106 | 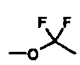 | 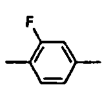 | - | 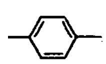 | O | $-C_3H_6-$ | 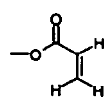 |
| 107 | | | - | | O | $-C_3H_6-$ | |
| 108 | | | - | | O | $-C_3H_6-$ | |

(fortgesetzt)

| Nr. | $Z^1$ | $A^1$ | $Z^2$ | $A^2$ | X' | Sp' | P |
|---|---|---|---|---|---|---|---|
| 109 | 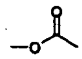 | 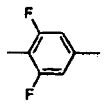 | - | 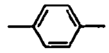 | O | $-C_3H_6-$ | 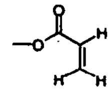 |
| 110 | 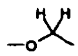 | 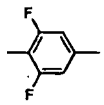 | - | 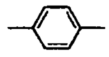 | O | $-C_3H_6-$ | 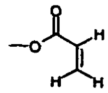 |
| 111 | 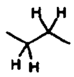 | 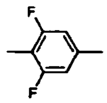 | - | 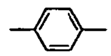 | O | $-C_3H_6-$ | 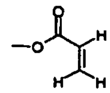 |
| 112 | 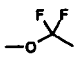 | 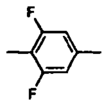 | - | 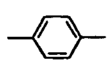 | O | $-C_3H_6-$ | 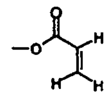 |
| 113 | 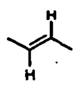 | 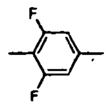 | - | 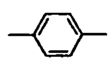 | O | $-C_3H_6-$ | 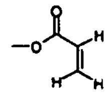 |
| 114 | 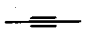 | 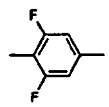 | - | 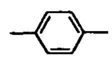 | O | $-C_3H_6-$ | 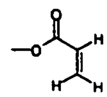 |
| 115 | 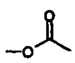 | 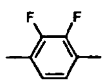 | - | 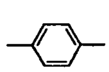 | O | $-C_3H_6-$ | 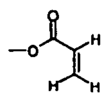 |
| 116 | 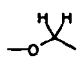 | 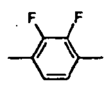 | - | 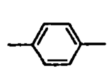 | O | $-C_3H_6-$ | 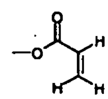 |
| 117 | 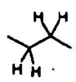 | 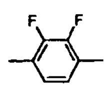 | - | 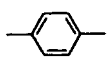 | O | $-C_3H_6-$ | 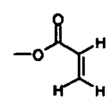 |
| 118 | 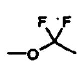 | 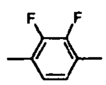 | - | 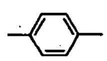 | O | $-C_3H_6-$ | 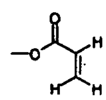 |
| 119 |  |  | - |  | O | $-C_3H_6-$ |  |
| 120 |  |  | - |  | O | $-C_3H_6-$ |  |
| 121 |  |  | - |  | O | $-C_6H_{12}-$ |  |

(fortgesetzt)

| Nr. | $Z^1$ | $A^1$ | $Z^2$ | $A^2$ | X' | Sp' | P |
|---|---|---|---|---|---|---|---|
| 122 | 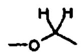 | 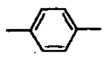 | - | | O | $-C_6H_{12}-$ | 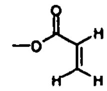 |
| 123 | 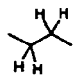 | 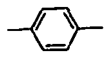 | - | | O | $-C_6H_{12}-$ | 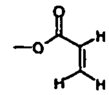 |
| 124 | 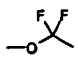 | 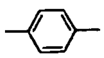 | - | | O | $-C_6H_{12}-$ | 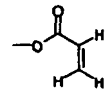 |
| 125 | 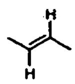 | 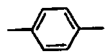 | - | | O | $-C_6H_{12}-$ | 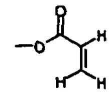 |
| 126 |  | 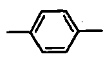 | - | | O | $-C_6H_{12}-$ | 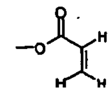 |
| 127 | 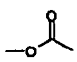 | 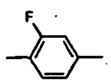 | - | | O | $-C_6H_{12}-$ | 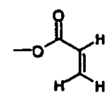 |
| 128 | 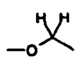 | 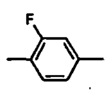 | - | | O | $-C_6H_{12}-$ | 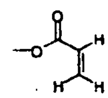 |
| 129 | 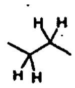 | 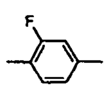 | - | | O | $-C_6H_{12}-$ | 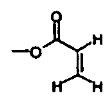 |
| 130 | 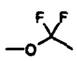 | 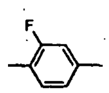 | - | | O | $-C_6H_{12}-$ | 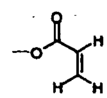 |
| 131 | 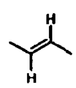 | 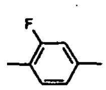 | - | | O | $-C_6H_{12}-$ | 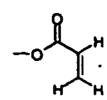 |
| 132 |  | 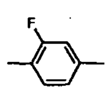 | - | | O | $-C_6H_{12}-$ | 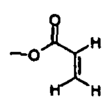 |
| 133 | 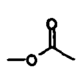 | 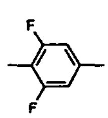 | - | | O | $-C_6H_{12}-$ | 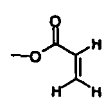 |
| 134 | 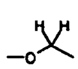 | 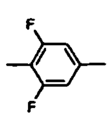 | - | | O | $-C_6H_{12}-$ | 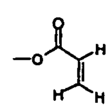 |

(fortgesetzt)

| Nr. | $Z^1$ | $A^1$ | $Z^2$ | $A^2$ | X' | Sp' | P |
|---|---|---|---|---|---|---|---|
| 135 | 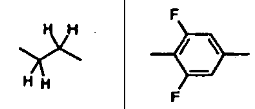 | | - | 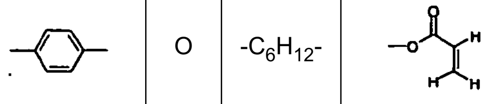 | O | $-C_6H_{12}-$ | |
| 136 | 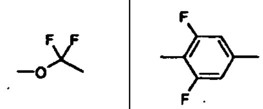 | | - | 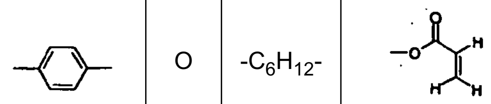 | O | $-C_6H_{12}-$ | |
| 137 | 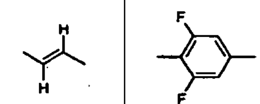 | | - | 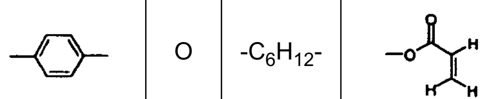 | O | $-C_6H_{12}-$ | |
| 138 | 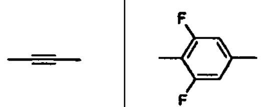 | | - | 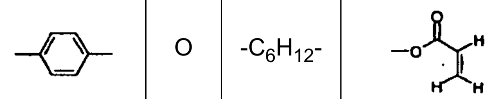 | O | $-C_6H_{12}-$ | |
| 139 | 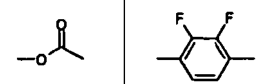 | | - | 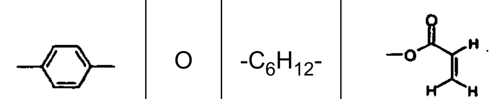 | O | $-C_6H_{12}-$ | |
| 140 | 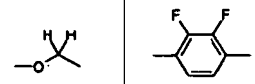 | | - | 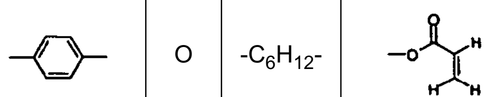 | O | $-C_6H_{12}-$ | |
| 141 | 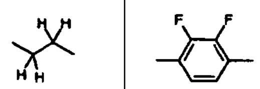 | | - | 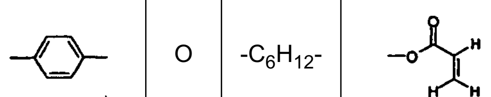 | O | $-C_6H_{12}-$ | |
| 142 | 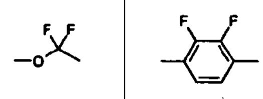 | | - | 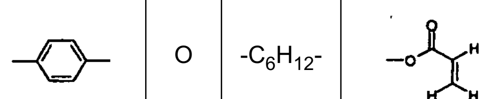 | O | $-C_6H_{12}-$ | |
| 143 | 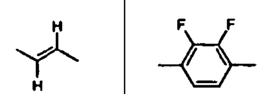 | | - | 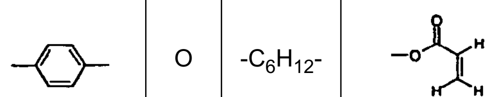 | O | $-C_6H_{12}-$ | |
| 144 | 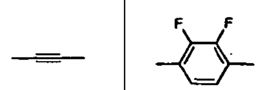 | | - | 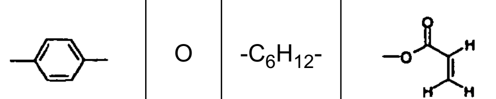 | O | $-C_6H_{12}-$ | |
| 145 | 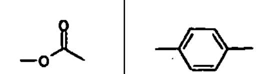 | | - | 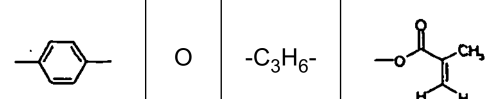 | O | $-C_3H_6-$ | |
| 146 | 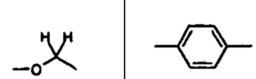 | | - | 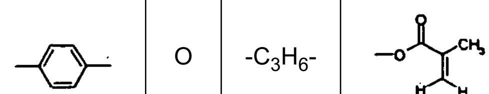 | O | $-C_3H_6-$ | |
| 147 | 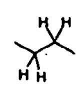 | | - | 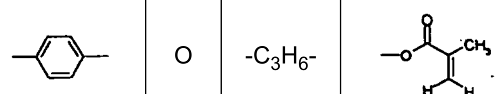 | O | $-C_3H_6-$ | |
| 148 | 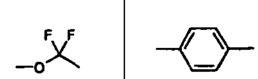 | | - | 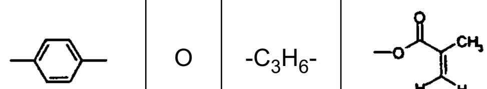 | O | $-C_3H_6-$ | |

(fortgesetzt)

| Nr. | $Z^1$ | $A^1$ | $Z^2$ | $A^2$ | X' | Sp' | P |
|---|---|---|---|---|---|---|---|
| 149 | 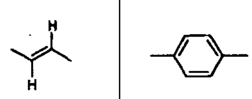 | | - | 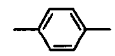 | O | $-C_3H_6-$ | 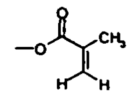 |
| 150 | 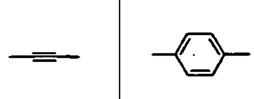 | | - | 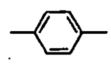 | O | $-C_3H_6-$ | 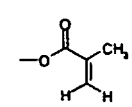 |
| 151 | 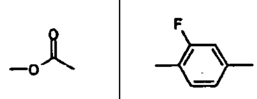 | | - | 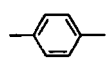 | O | $-C_3H_6-$ | 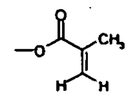 |
| 152 | 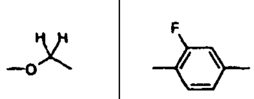 | | - | 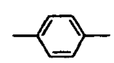 | O | $-C_3H_6-$ | 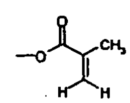 |
| 153 | 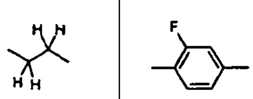 | | - | 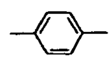 | O | $-C_3H_6-$ | 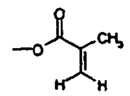 |
| 154 | 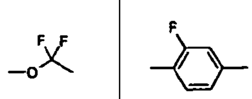 | | - | 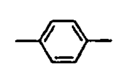 | O | $-C_3H_6-$ | 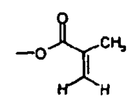 |
| 155 | 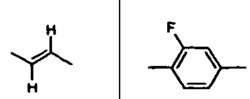 | | - | 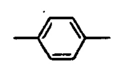 | O | $-C_3H_6-$ | 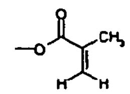 |
| 156 | 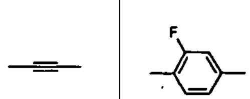 | | - | 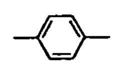 | O | $-C_3H_6-$ | 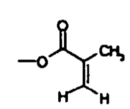 |
| 157 | 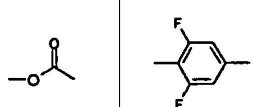 | | - | 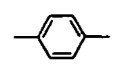 | O | $-C_3H_6-$ | 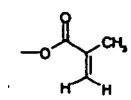 |
| 158 | 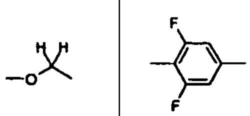 | | - | 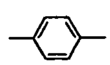 | O | $-C_3H_6-$ | 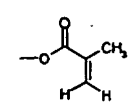 |
| 159 | 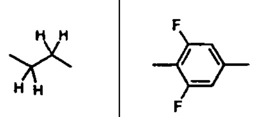 | | - | 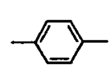 | O | $-C_3H_6-$ | 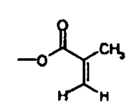 |
| 160 | 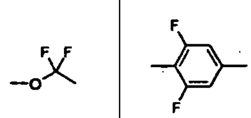 | | - | 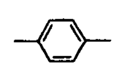 | O | $-C_3H_6-$ | 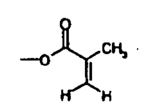 |
| 161 | 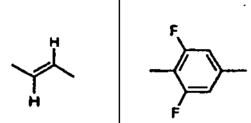 | | - | 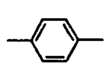 | O | $-C_3H_6-$ | |
| 162 | 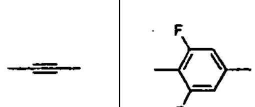 | | - | 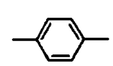 | O | $-C_3H_6-$ | |

(fortgesetzt)

| Nr. | $Z^1$ | $A^1$ | $Z^2$ | $A^2$ | X' | Sp' | P |
|---|---|---|---|---|---|---|---|
| 163 | 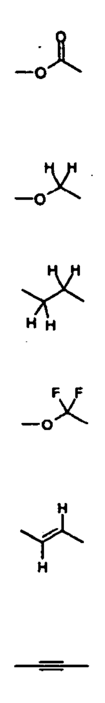 | 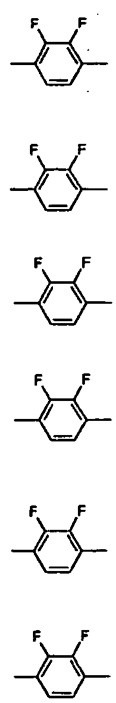 | - | 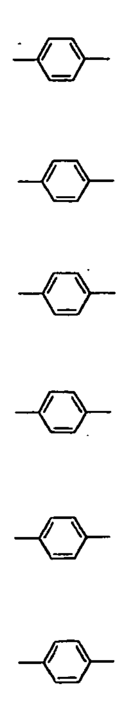 | O | $-C_3H_6-$ | 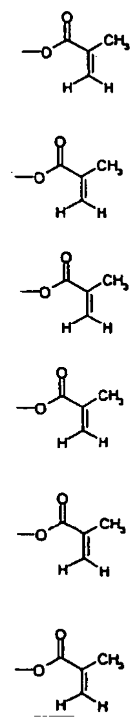 |
| 164 | | | - | | O | $-C_3H_6-$ | |
| 165 | | | - | | O | $-C_3H_6-$ | |
| 166 | | | - | | O | $-C_3H_6-$ | |
| 167 | | | - | | O | $-C_3H_6-$ | |
| 168 | | | - | | O | $-C_3H_6-$ | |
| 169 | 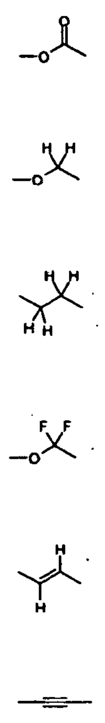 | 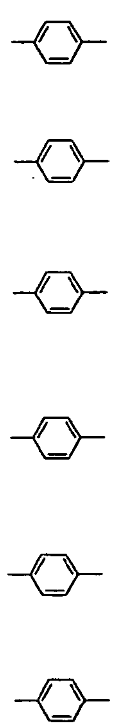 | - | 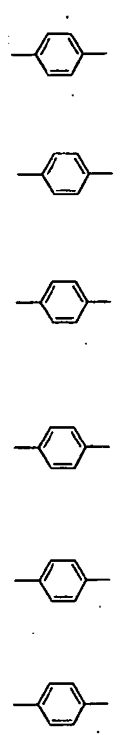 | O | $-C_6H_{12}-$ | 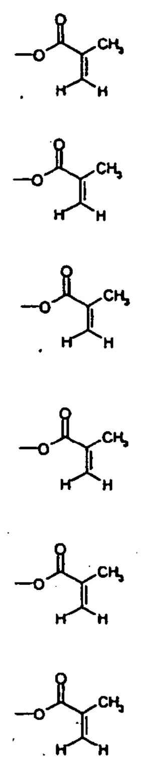 |
| 170 | | | - | | O | $-C_6H_{12}-$ | |
| 171 | | | - | | O | $-C_6H_{12}-$ | |
| 172 | | | - | | O | $-C_6H_{12}-$ | |
| 173 | | | - | | O | $-C_6H_{12}-$ | |
| 174 | | | - | | O | $-C_6H_{12}-$ | |
| 175 | 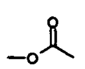 | 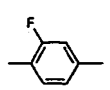 | - | 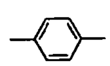 | O | $-C_6H_{12}-$ | 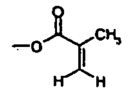 |

(fortgesetzt)

| Nr. | $Z^1$ | $A^1$ | $Z^2$ | $A^2$ | X' | Sp' | P |
|---|---|---|---|---|---|---|---|
| 176 | 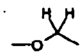 | 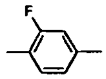 | - | | O | $-C_6H_{12}-$ | 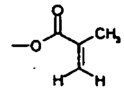 |
| 177 | 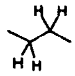 | 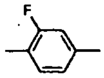 | - | | O | $-C_6H_{12}-$ | 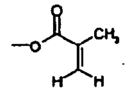 |
| 178 | 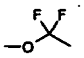 | 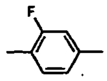 | - | | O | $-C_6H_{12}-$ | 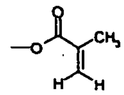 |
| 179 | 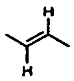 | 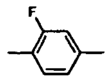 | - | | O | $-C_6H_{12}-$ | 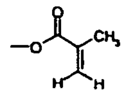 |
| 180 |  | 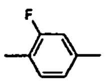 | - | | O | $-C_6H_{12}-$ | 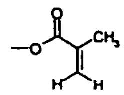 |
| 181 | 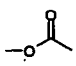 | 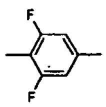 | - | | O | $-C_6H_{12}-$ | 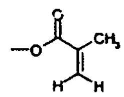 |
| 182 | 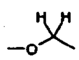 | 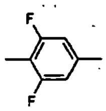 | - | | O | $-C_6H_{12}-$ | 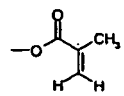 |
| 183 | 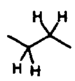 | 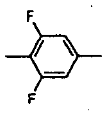 | - | | O | $-C_6H_{12}-$ | 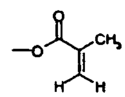 |
| 184 | 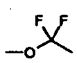 | 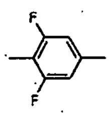 | - | | O | $-C_6H_{12}-$ | 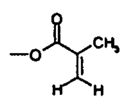 |
| 185 | 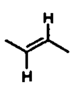 | 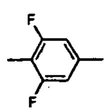 | - | | O | $-C_6H_{12}-$ | 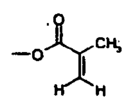 |
| 186 |  | 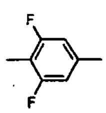 | - | | O | $-C_6H_{12}-$ | 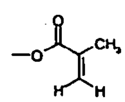 |
| 187 | 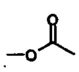 | 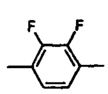 | - | | O | $-C_6H_{12}-$ | 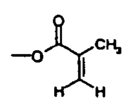 |
| 188 | 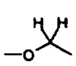 | 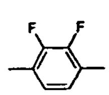 | - | | O | $-C_6H_{12}-$ | 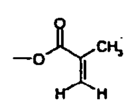 |

(fortgesetzt)

| Nr. | $Z^1$ | $A^1$ | $Z^2$ | $A^2$ | X' | Sp' | P |
|---|---|---|---|---|---|---|---|
| 189 | 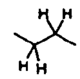 | 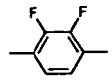 | - | 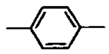 | O | $-C_6H_{12}-$ | 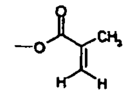 |
| 190 | 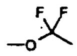 | 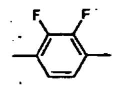 | - | 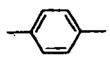 | O | $-C_6H_{12}-$ | 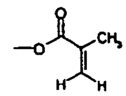 |
| 191 | 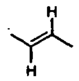 | 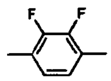 | - | 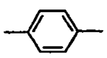 | O | $-C_6H_{12}-$ | 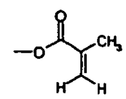 |
| 192 |  | 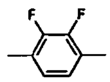 | - | 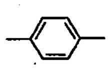 | O | $-C_6H_{12}-$ | 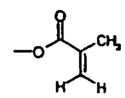 |

Die Herstellung der polymerisierbaren Verbindungen erfolgt in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart. Polymerisierbare Binaphthylverbindungen und ihre Synthese sind beispielsweise in DE 43 42 280 A1, DE 195 20 704 A1, GB 2 328 436 A, GB 2 398 569 A, GB 2 298 202 A, EP 0 964 035 A1, EP 1 249 483 A1, WO 02/034739 A1, WO 02/006195 A1, WO 02/094805 A1, US 2005/179005 A1, JP 2001-066431 A, JP 2005-170934 A oder JP 2005-171235 A beschrieben.

Die polymerisierbaren Verbindungen werden im FK-Medium zwischen den Substraten der FK-Anzeige unter Anlegen einer Spannung durch in-situ-Polymerisation polymerisiert bzw. vernetzt (falls eine Verbindung zwei oder mehr polymerisierbare Gruppen enthält). Geeignete und bevorzugte Polymerisationsmethoden sind beispielsweise die thermische oder Photopolymerisation, vorzugsweise Photopolymerisation, insbesondere UV- Photopolymerisation. Dabei können ggf. auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich z.B. die kommerziell erhältlichen Photoinitiatoren Irgacure651®, Irgacure184®, oder Darocure1173® (Ciba AG). Falls ein Initiator eingesetzt wird, beträgt dessen Anteil im Gesamtgemisch vorzugsweise 0,001 bis 5 Gew.-%, besonders bevorzugt 0,005 bis 1 Gew.-%. Die Polymerisation kann aber auch ohne Zusatz eines Initiators erfolgen.

Die erfindungsgemäßen Biarylverbindungen eignen sich besonders für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte.

Das FK-Medium kann auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind z.B. die kommerziell erhältlichen Stabilisatoren der Serie Irganox ® (Ciba AG). Falls Stabilisatoren eingesetzt werden, beträgt deren Anteil, bezogen auf die Gesamtmenge an polymerisierbaren Verbindungen vorzugsweise 10 bis 5000 ppm, besonders bevorzugt 50 bis 500 ppm.

Die erfindungsgemäßen FK-Medien enthalten vorzugsweise < 5%, besonders bevorzugt < 1 %, ganz besonders bevorzugt < 0.5 % an polymerisierbaren Verbindungen, insbesondere Biarylverbindungen der oben genannten Formeln.

Die erfindungsgemäßen polymerisierbaren Verbindungen können einzeln den FK-Medien zugesetzt werden, es können aber auch Mischungen enthaltend zwei oder mehr erfindungsgemäße polymerisierbare Biarylverbindungen, oder Mischungen enthaltend eine oder mehrere erfindungsgemäße polymerisierbare Biarylverbindungen und eine oder mehrere zusätzliche polymerisierbare Verbindungen (Comonomere) verwendet werden. Die Comonomere können mesogen oder nichtmesogen sein. Bei Polymerisation solcher Mischungen entstehen Copolymere. Die vor- und nachstehend genannten polymerisierbaren Mischungen sind ein weiterer Gegenstand der Erfindung.

Geeignete und bevorzugte mesogene Comonomere sind beispielsweise solche ausgewählt aus den folgenden Formeln:

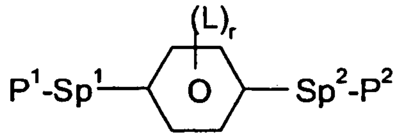

I*1

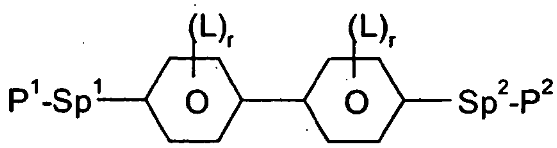

I*2

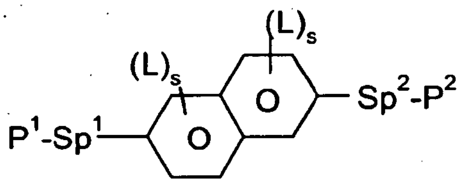

I*3

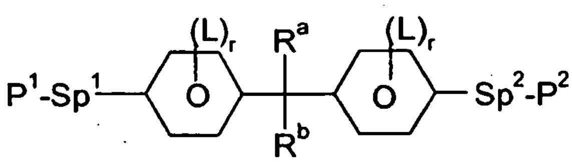

I*4

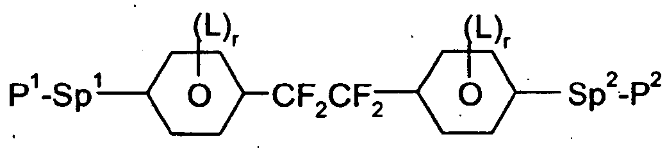

I*5

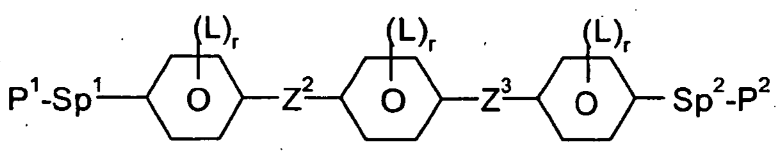

I*6

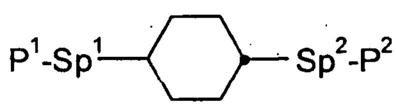

I*7

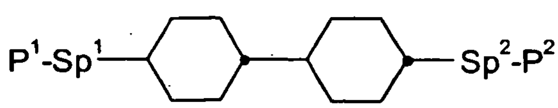

I*8

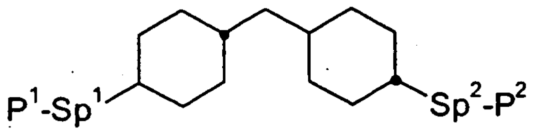

I*9

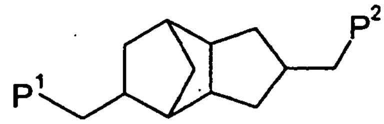

I*10

worin $P^1$ und $P^2$ eine der für P angegebenen Bedeutungen besitzen und vorzugsweise Acrylat oder Methacrylat bedeuten, $Sp^1$ und $Sp^2$ eine der für Sp angegebenen Bedeutungen besitzen oder eine Einfachbindung bedeuten, $Z^2$ und $Z^3$ jeweils unabhängig voneinander eine der für $Z^1$ angegebenen Bedeutungen besitzen und vorzugsweise -COO- oder-OCO bedeuten, L und r bei jedem Auftreten gleich oder verschieden eine der oben angegebenen Bedeutungen besitzen und L vorzugsweise F oder $CH_3$ bedeutet, s 0, 1, 2 oder 3 bedeutet, und $R^a$ und $R^b$ jeweils unabhängig voneinander H oder $CH_3$ bedeuten.

Die FK-Medien zur Verwendung in den erfindungsgemäßen FK-Anzeigen enthalten neben den oben beschriebenen polymerisierbaren Biarylverbindungen eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der Biarylverbindungen verwendeten Bedingungen. Prinzipiell eignet sich als Host-Mischung jede zur Verwendung in herkömmlichen VA- und OCB-Anzeigen geeignete FK-Mischung. Geeignete FK-Mischungen sind dem Fachmann bekannt und in der Literatur beschrieben, beispielsweise Mischungen in VA-Anzeigen in EP 1 378 557 A1 und Mischungen für OCB-Anzeigen in EP 1 306 418 A1 und DE 102 24 046 A1.

Besonders bevorzugte FK-Medien werden im Folgenden genannt:

1) FK-Medium, welches eine oder mehrere Verbindungen der Formel II enthält:

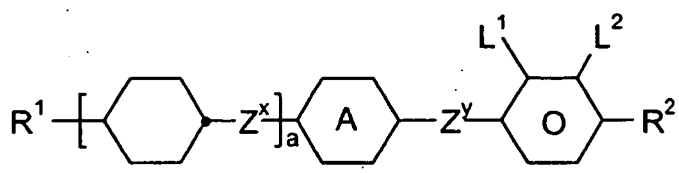

II

worin

| | |
|---|---|
| $R^1$ und $R^2$ | jeweils unabhängig voneinander Alkyl mit 1 bis 12 C-Atomen, wobei auch ein oder zwei nicht benachbarte $CH_2$-Gruppen durch -O-, -CH=CH-, -CO-, -OCO- oder -COO-so ersetzt sein können, daß O-Atome nicht direkt miteinander verknüpft sind, |
| $Z^x$ und $Z^y$ | jeweils unabhängig voneinander $-CH_2CH_2-$, -CH=CH-, $-CF_2O-$, $-OCF_2-$, $-CH_2O-$, $-OCH_2-$, -COO-, -OCO-, $-C_2F_4-$, -CF=CF-, $-CH=CHCH_2O-$, oder eine Einfachbindung, vorzugsweise eine Einfachbindung, |
| $L^1$ und $L^2$ | jeweils unabhängig voneinander F, Cl, $OCF_3$, $CF_3$, $CH_3$, $CH_2F$ oder $CHF_2$, |
| a | 0 oder 1 und |

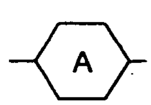

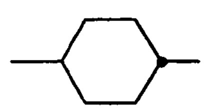

oder

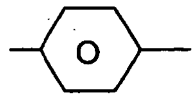

bedeuten. Vorzugsweise bedeuten $L^1$ und $L^2$ F, oder einer der Reste $L^1$ und $L^2$ Cl und der andere F.

Die Verbindungen der Formel II sind vorzugsweise ausgewählt aus den folgenden Formeln

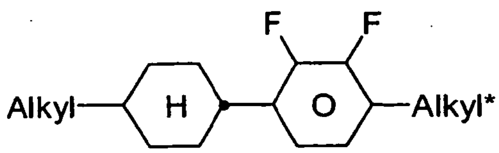

II1

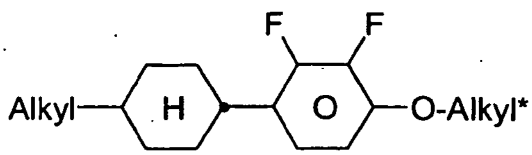

II2

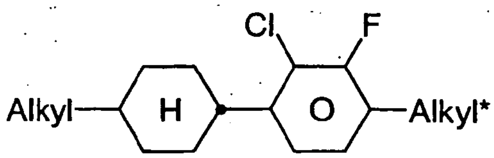

II3

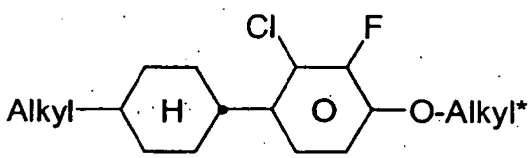

II4

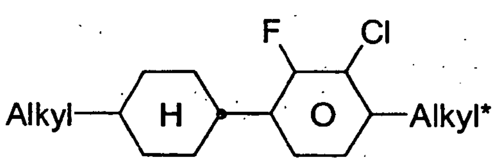

II5

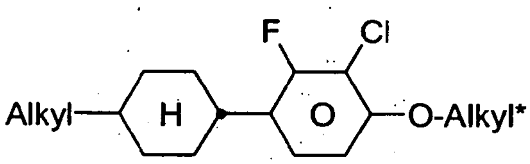

II6

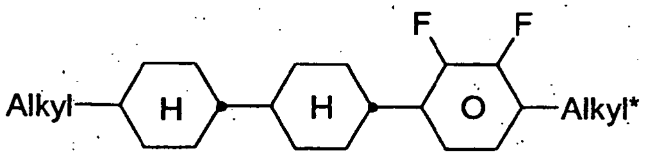

II7

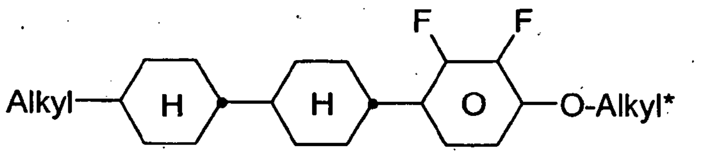

II8

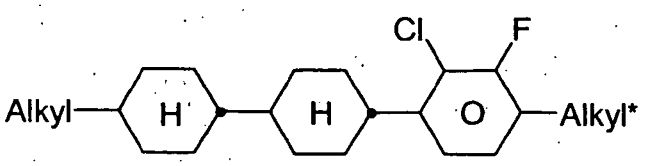

II9

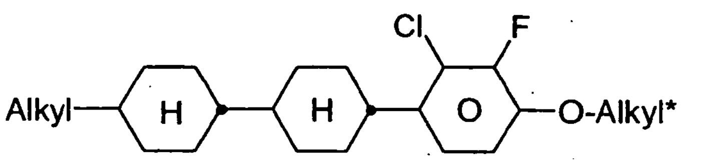

II10

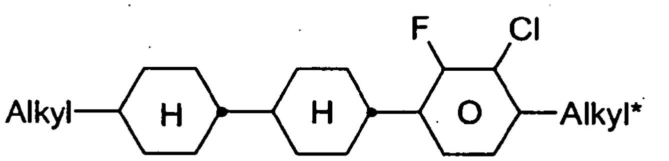

II11

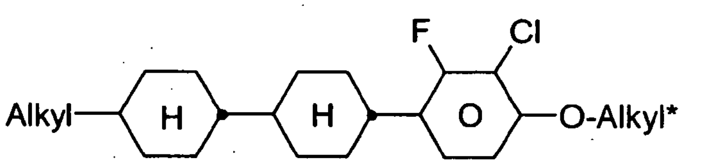

II12

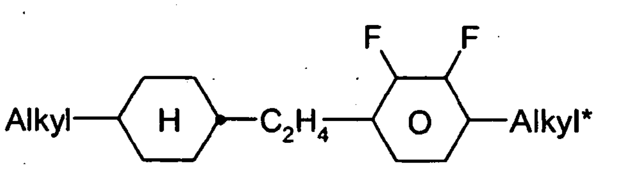

II13

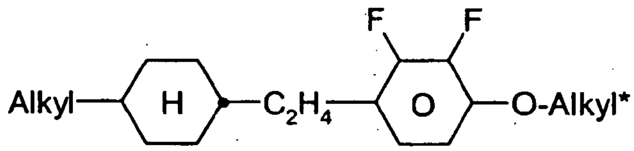

II14

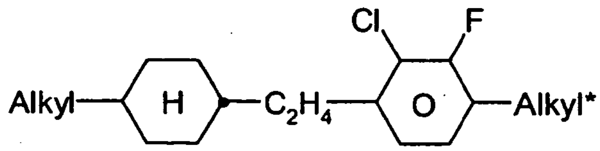

II15

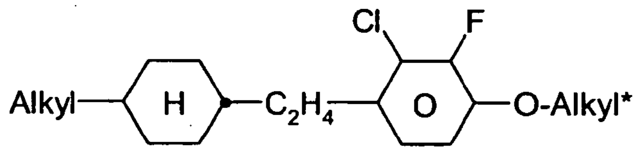

II16

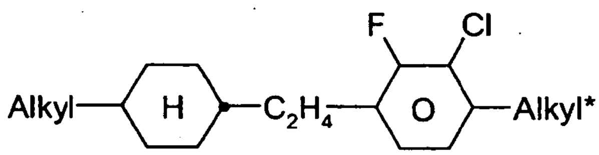

II17

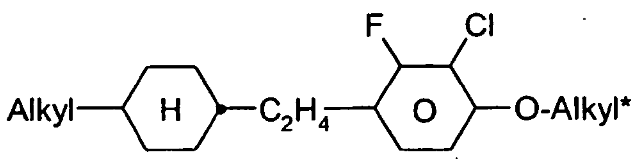

II18

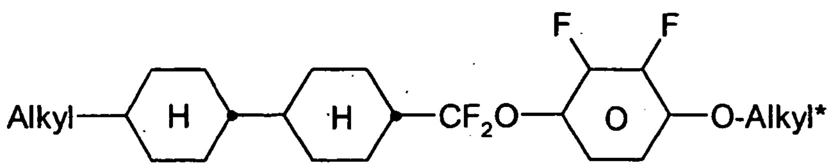

II19

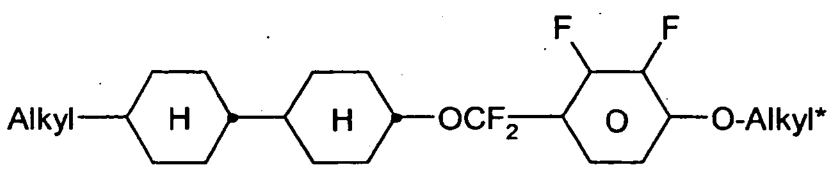

II20

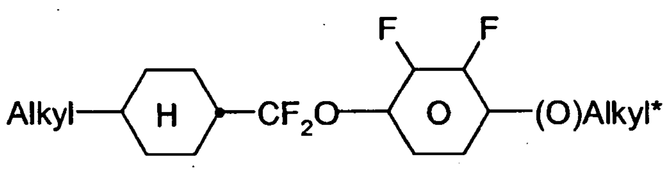

II21

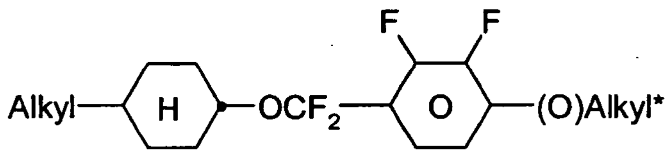

II22

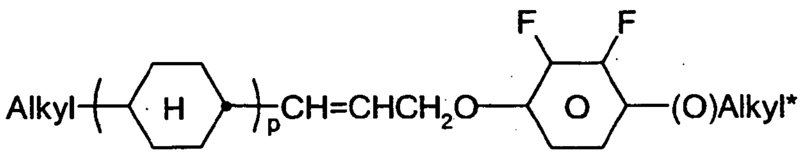

II23

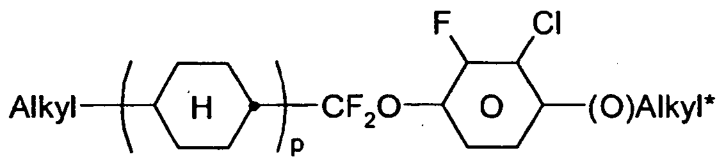

II24

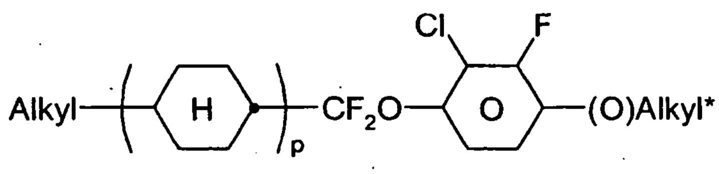

II25

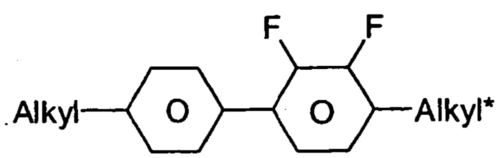

II26

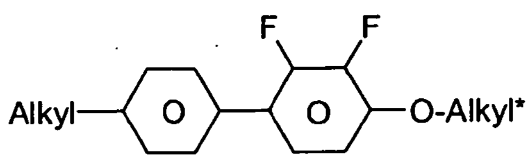

II27

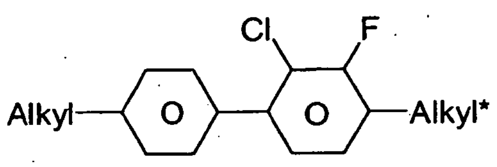

II28

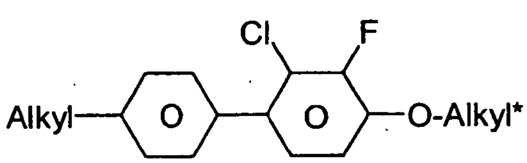

II29

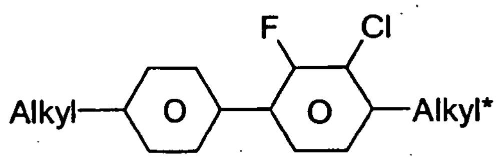

II30

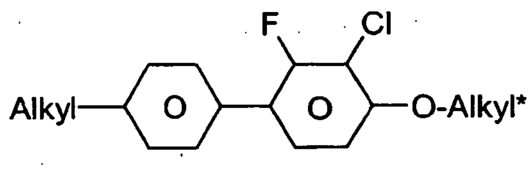

II31

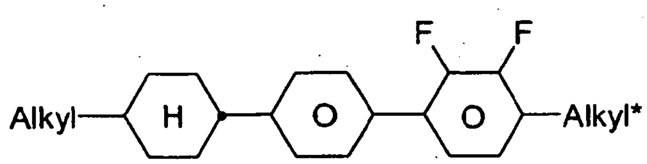

II32

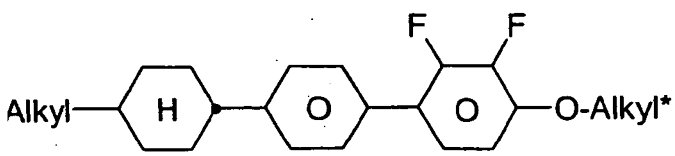

II33

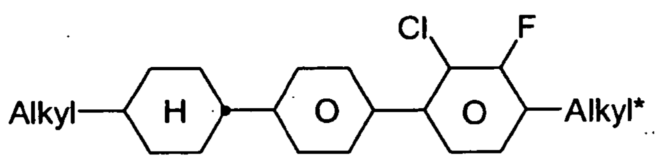

II34

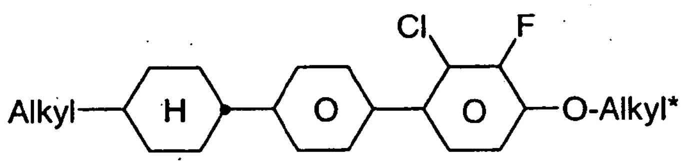

II35

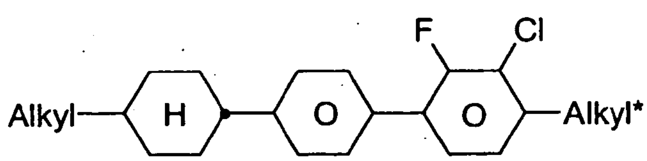

II36

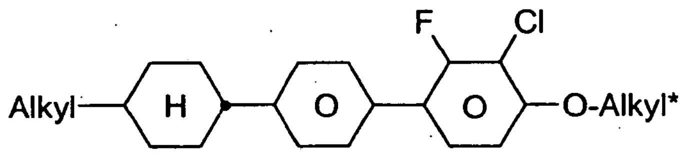

II37

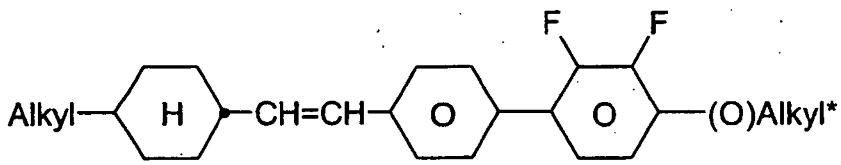

II38

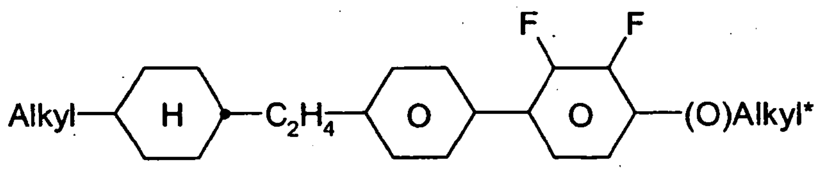

II39

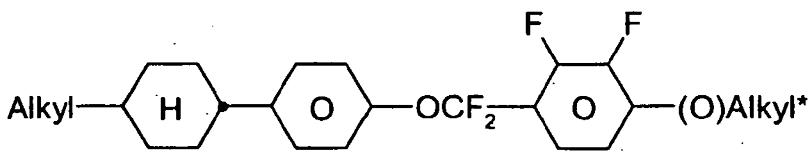

II40

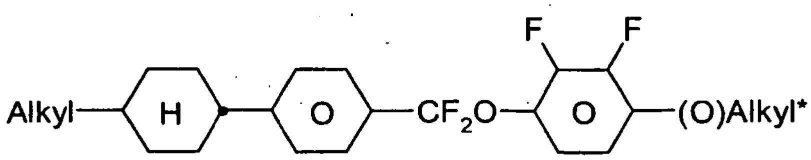

II41

worin p 1 oder 2 und Alkyl und Alkyl* jeweils unabhängig voneinander einen geradkettigen Alkylrest mit 1-6 C-Atomen und Alkenyl einen geradkettigen Alkenylrest mit 2-6 C-Atomen bedeuten.

2) Medium, welches zusätzlich eine oder mehrere Verbindungen der Formel III enthält:

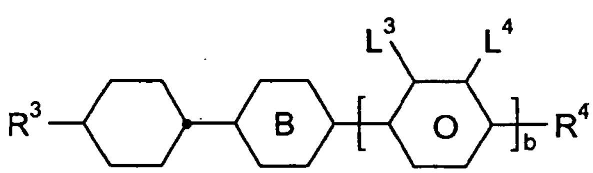

III

worin die einzelnen Reste folgende Bedeutung besitzen

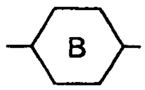 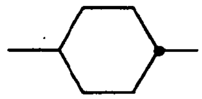, 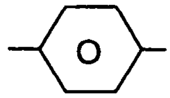 oder 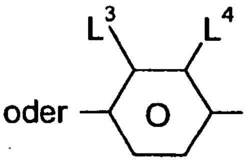

oder

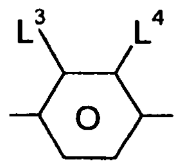

b 0 oder 1,

$L^3$ und $L^4$ jeweils unabhängig voneinander H, F oder Cl,

$R^3$ Alkenyl mit 2 bis 9 C-Atomen,

$R^4$ Alkyl mit 1 bis 12 C-Atomen, worin auch ein oder zwei nicht benachbarte $CH_2$-Gruppen durch -O-, -CH=CH-,-CO-, -OCO- oder -COO- so ersetzt sein können, daß O-Atome nicht direkt miteinander verknüpft sind, oder, falls b=0 ist und der Ring B Cyclohexylen bedeutet, auch $R^1$.

$R^4$ ist vorzugsweise geradkettiges Alkyl oder Alkoxy mit 1 bis 8 C-Atomen, besonders bevorzugt Methoxy, Ethoxy, n-Propoxy oder n-Butoxy. Vorzugsweise bedeuten $L^3$ und $L^4$ F, oder $L^3$ Cl und $L^4$ F.
Die Verbindungen der Formel III sind vorzugsweise ausgewählt aus den folgenden Formeln:

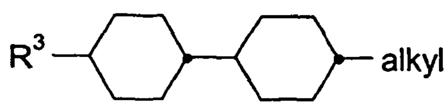

IIIa

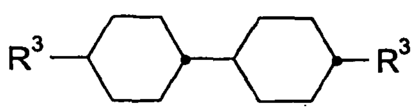

IIIb

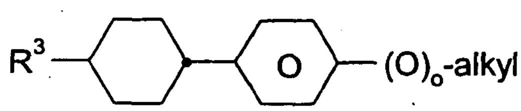

IIIc

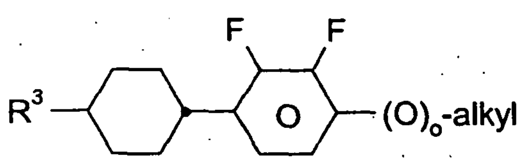

IIId

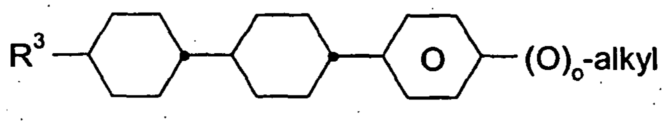

IIIe

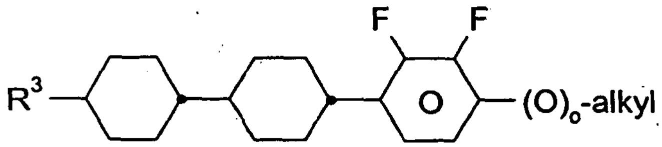

IIIf

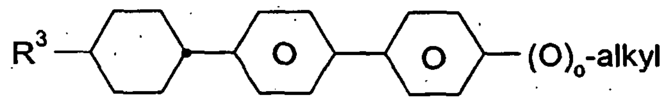

IIIg

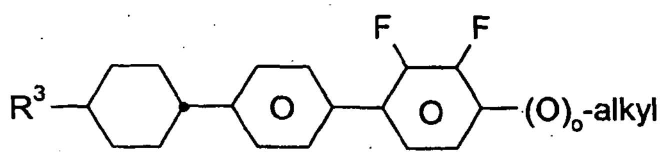

IIIh

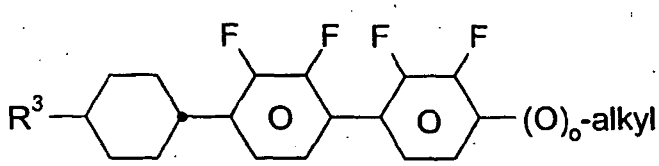

IIIi

worin $R^3$ bei jedem Auftreten gleich oder verschieden die oben angegebene Bedeutung besitzt, o 0 oder 1 ist, und "alkyl" $C_{1-6}$-alkyl, welches vorzugsweise geradkettig ist, bedeutet. Besonders bevorzugt sind Verbindungen der Formeln IIIa, IIIb, IIId und IIIf.

3) Medium, welches zusätzlich eine oder mehrere Verbindungen der Formel IV enthält:

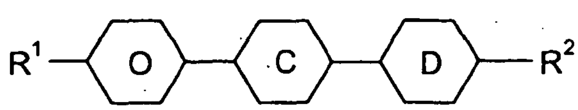

IV

worin $R^1$ und $R^2$ jeweils unabhängig voneinander eine der in Formel II angegebenen Bedeutungen besitzen und

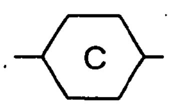

und

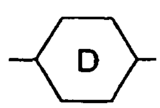

jeweils unabhängig voneinander

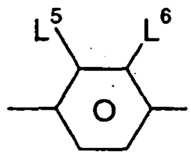 , 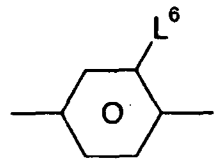 ,

oder

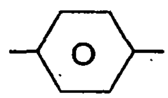

bedeuten, worin $L^5$ F oder Cl, vorzugsweise F, und $L^6$ F, Cl, $OCF_3$, $CF_3$, $CH_3$, $CH_2F$, oder $CHF_2$, vorzugsweise F, bedeuten.

Die Verbindungen der Formel IV sind vorzugsweise ausgewählt aus den folgenden Formeln

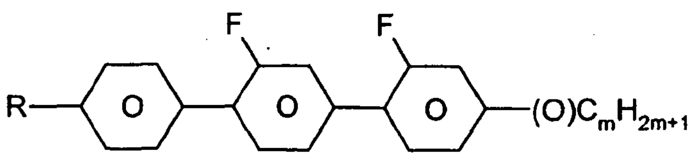 IV1

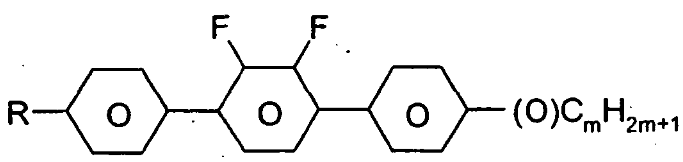 IV2

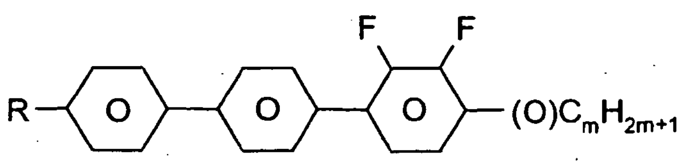 IV3

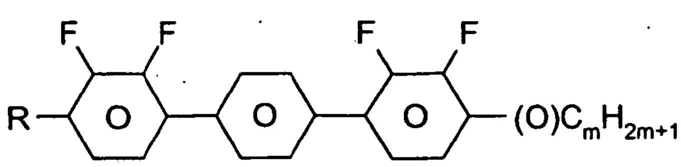 IV4

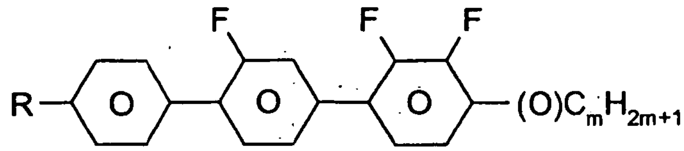
IV5

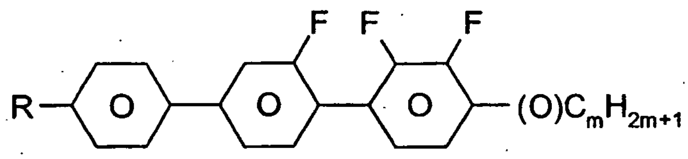
IV6

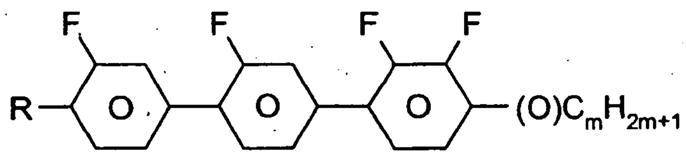
IV7

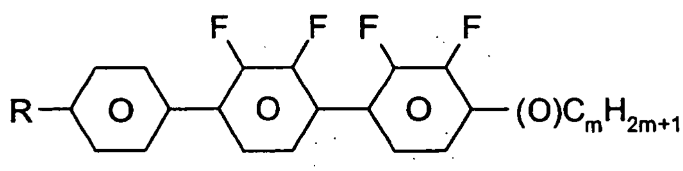
IV8

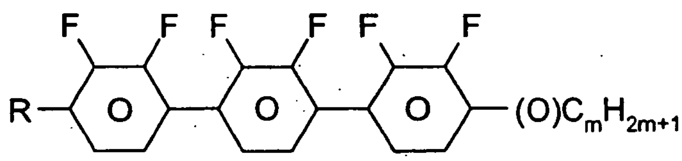
IV9

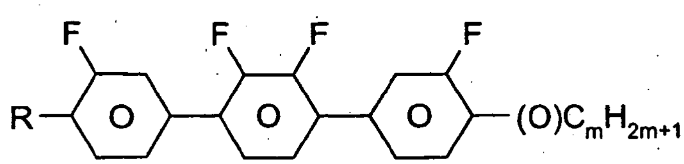
IV10

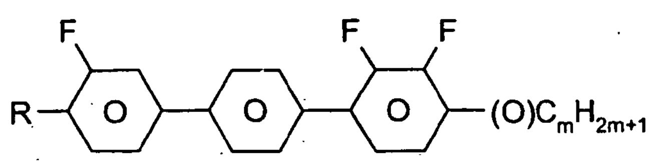
IV11

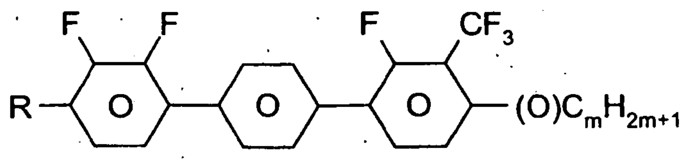

IV12

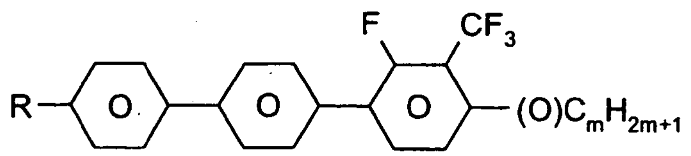

IV13

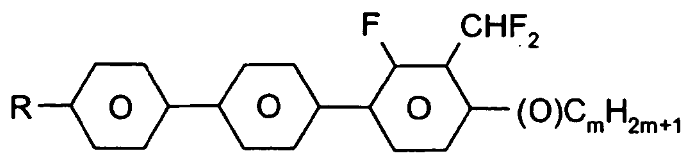

IV14

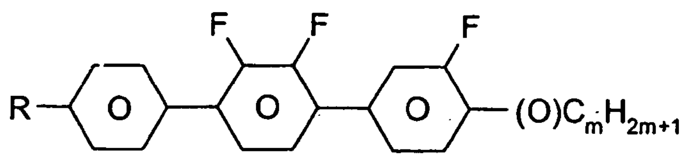

IV15

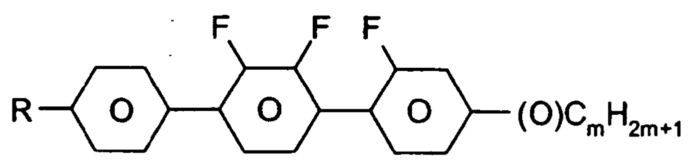

IV16

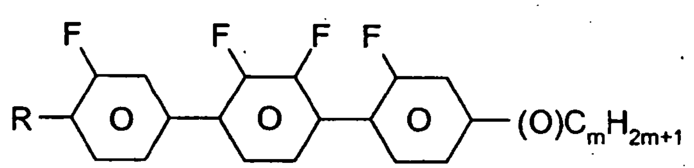

IV17

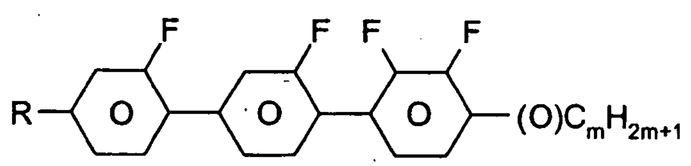

IV18

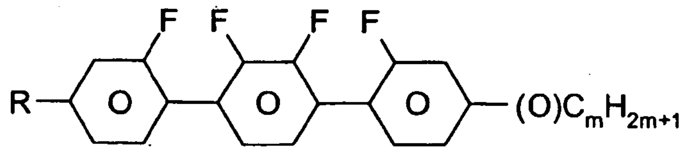

IV19

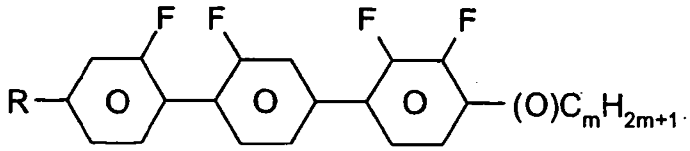

IV20

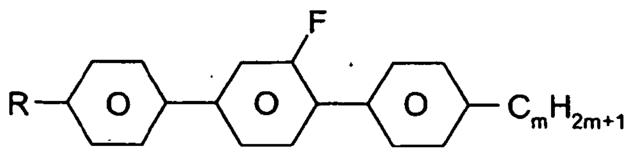

IV21

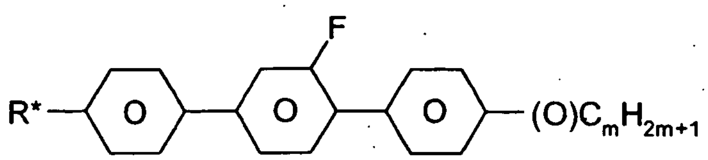

, IV22

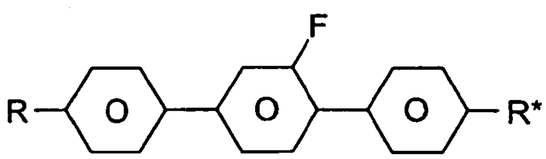

IV23

worin R einen geradkettigen Alkyl- oder Alkoxyrest mit 1-7 C-Atomen, R* einen geradkettigen Alkenylrest mit 2-7 C-Atomen, und m eine ganze Zahl von 1 bis 6 bedeutet.

4) Medium, welches zusätzlich eine oder mehrere Verbindungen ausgewählt aus den Formeln Va und Vb enthält:

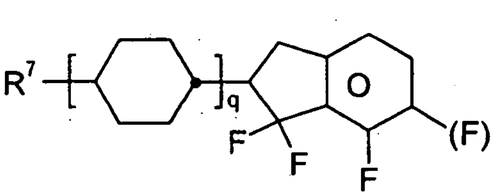

Va

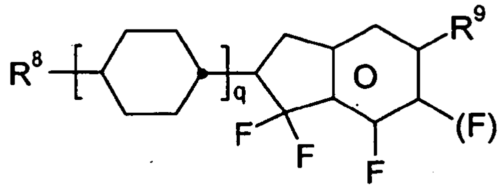

Vb

worin $R^7$ und $R^8$ jeweils unabhängig voneinander eine der für $R^1$ angegebenen Bedeutungen haben, $R^9$ $CH_3$, $C_2H_5$ oder n-$C_3H_7$ und q 1 oder 2 bedeutet.

Besonders bevorzugte Verbindungen der Formel Va und Vb sind ausgewählt aus den folgenden Unterformeln:

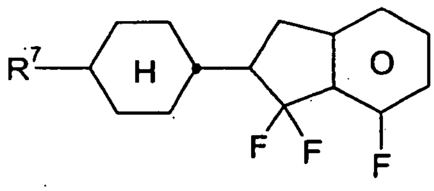

Va1

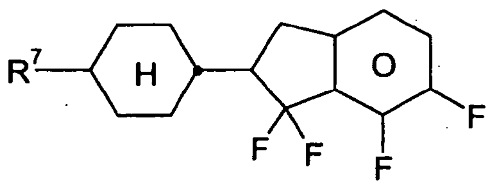

Va2

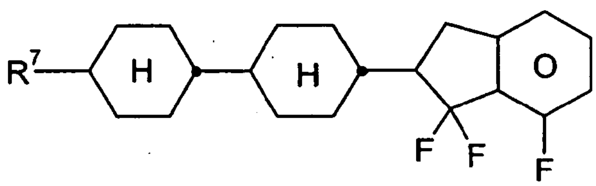

Va3

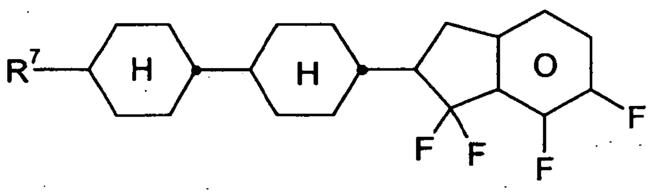

Va4

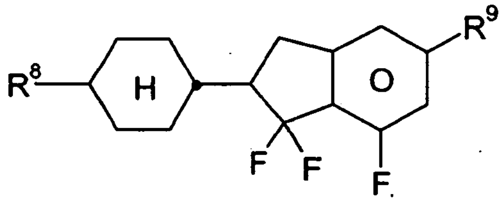

Vb1

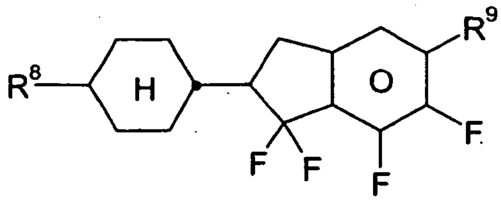

Vb2

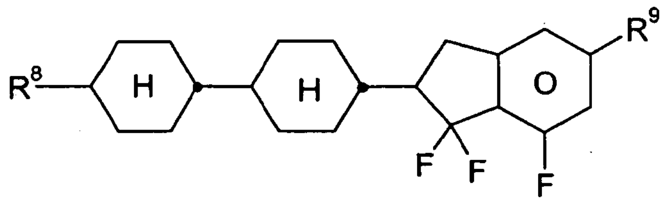

Vb3

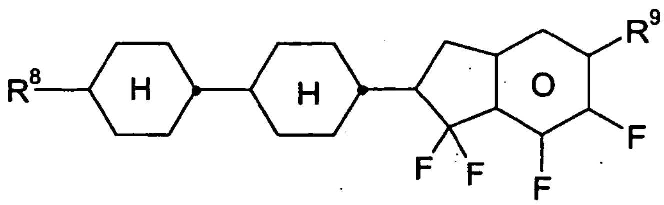

Vb4

worin $R^7$ und $R^8$ vorzugsweise geradkettiges Alkyl bedeuten und $R^9$ $CH_3$, $C_2H_5$ oder n-$C_3H_7$ bedeutet.

5) Medium, welches zusätzlich eine oder mehrere Verbindungen enthält, die eine Tetrahydronaphthyl- oder Naphthyl-Einheit aufweisen, wie z.B. die Verbindungen ausgewählt aus den folgenden Formeln:

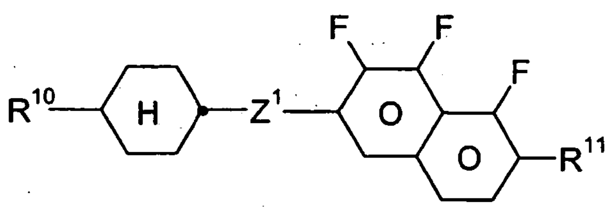

VIa

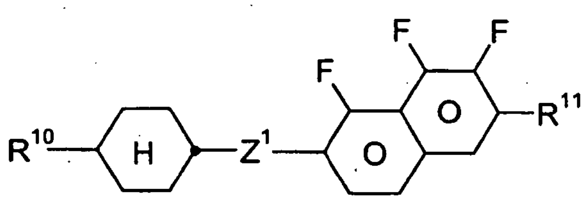

VIb

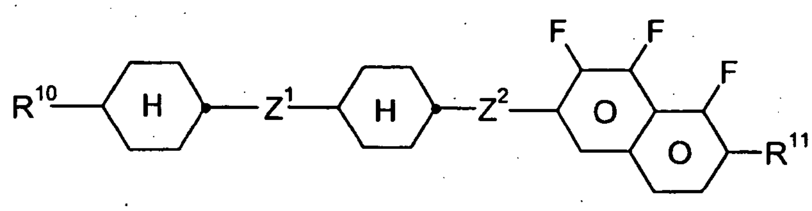

VIc

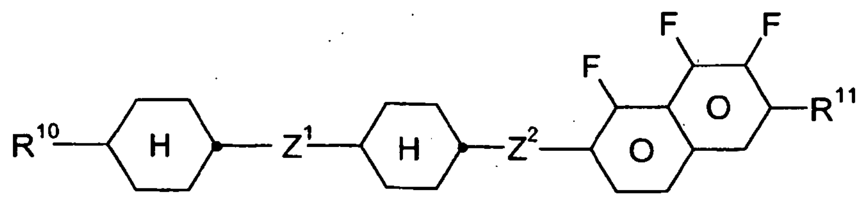

VId

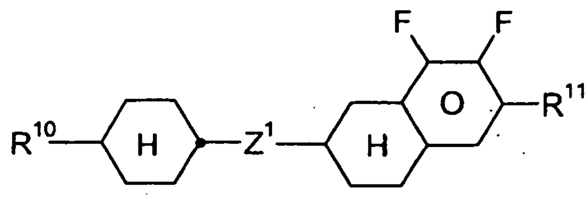

VIe

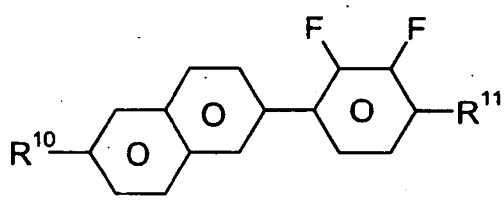

VIf

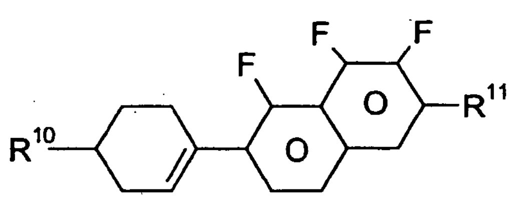

VIg

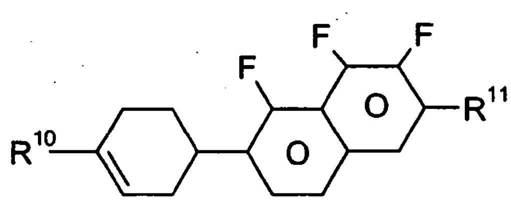

VIh

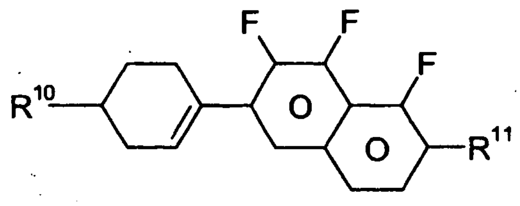

VIi

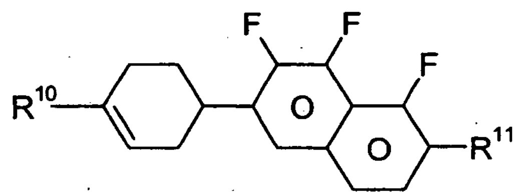

VIk

worin $R^{10}$ und $R^{11}$ jeweils unabhängig voneinander eine der für $R^1$ angegebenen Bedeutungen haben, vorzugsweise geradkettiges Alkyl, geradkettiges Alkoxy oder geradkettiges Alkenyl bedeuten, und $Z^1$ und $Z^2$ jeweils unabhängig voneinander $-C_2H_4-$, -CH=CH-, $-(CH_2)_4-$, $-(CH_2)_3O-$, $-O(CH_2)_3-$, $-CH=CHCH_2CH_2-$, $-CH_2CH_2CH=CH-$, $-CH_2O-$,$-OCH_2-$, -COO-, -OCO-, $-C_2F_4-$, -CF=CF-, -CF=CH-, -CH=CF-, $-CH_2-$oder eine Einfachbindung bedeuten.

6) Medium, welches zusätzlich eine oder mehrere Verbindungen der Formel VII enthält:

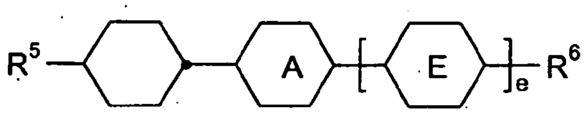

VII

worin

$R^5$ und $R^6$. unabhängig voneinander eine der für $R^1$ in Formel II angegebenen Bedeutungen haben,

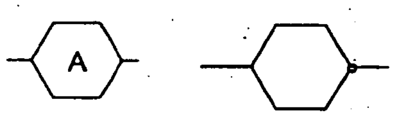

oder

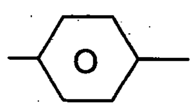

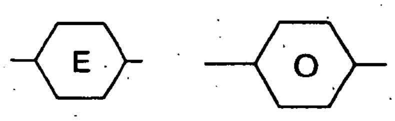

oder

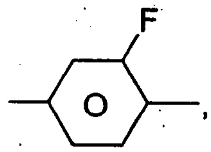,

und

e 0 oder 1 bedeuten.

Die Verbindungen der Formel VII sind vorzugsweise ausgewählt aus den folgenden Formeln:

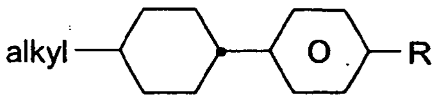

VIIa

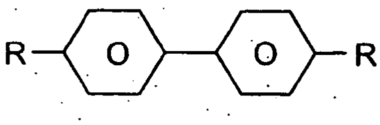

VIIb

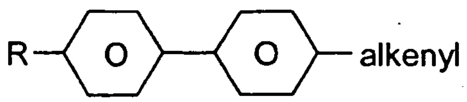

VIIc

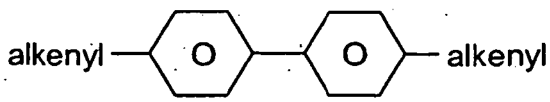

VIId

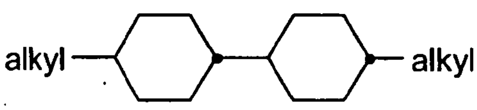

VIIe

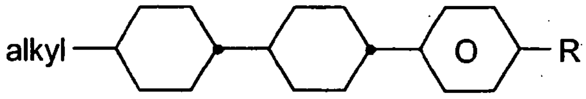

VIIf

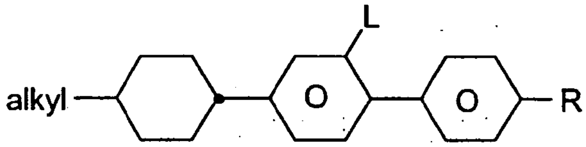

VIIg

worin "alkyl" $C_{1-6}$-alkyl, R $C_{1-6}$-alkyl oder -alkoxy, "alkenyl" $C_{2-7}$-alkenyl und L H oder F bedeuten.

7) Medium, welches zusätzlich eine oder mehrere Verbindungen ausgewählt aus den Formeln VIIIa bis VIIId enthält:

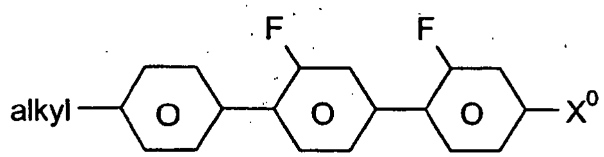
VIIIa

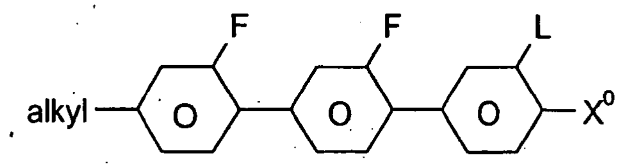
VIIIb

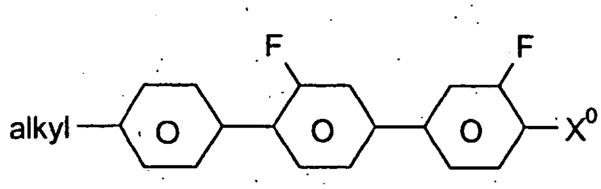
VIIIc

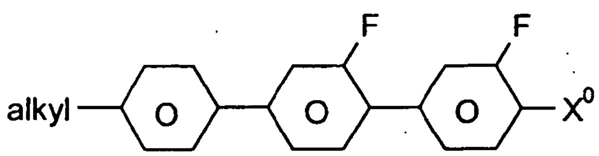
VIIId

worin "alkyl" $C_{1-6}$-alkyl, L H oder F und $X^0$ F oder Cl bedeuten. Besonders bevorzugt sind Verbindungen der Formel VIIIa, worin $X^0$ F bedeutet.

8) Medium, welches zusätzlich eine oder mehrere Verbindungen ausgewählt aus den folgenden Formeln enthält:

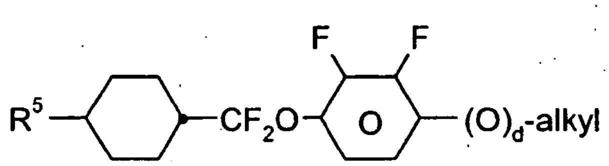
IXa

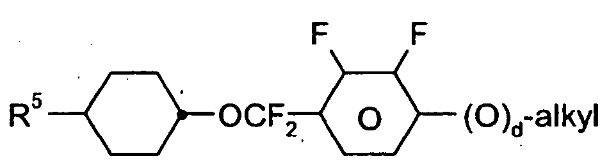
IXb

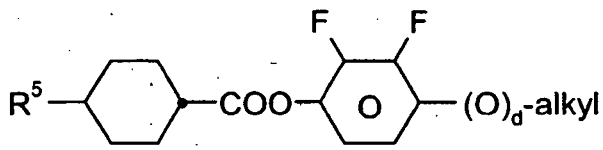

IXc

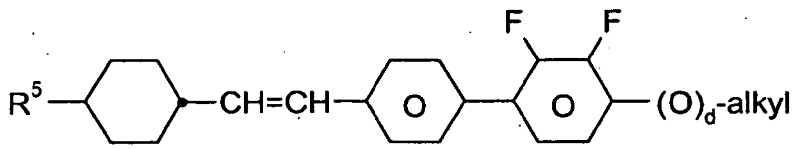

Xa

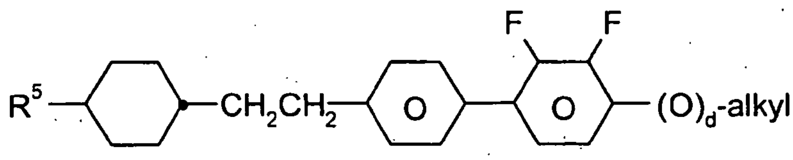

Xb

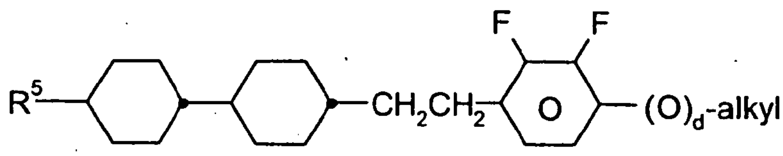

XIa

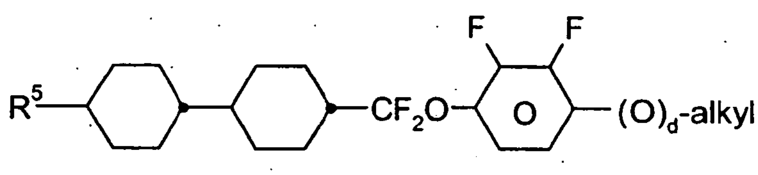

XIb

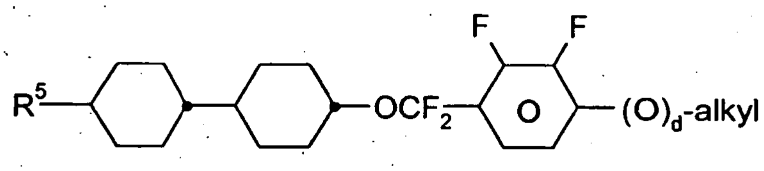

XIc

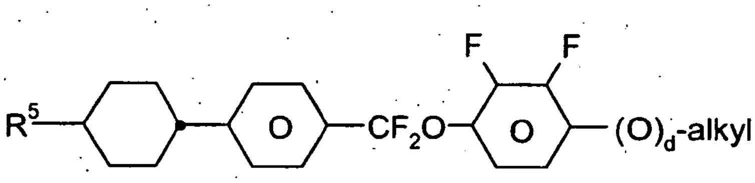

XIIa

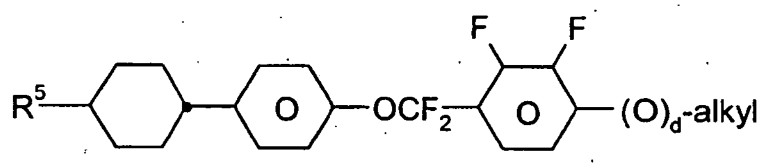

XIIb

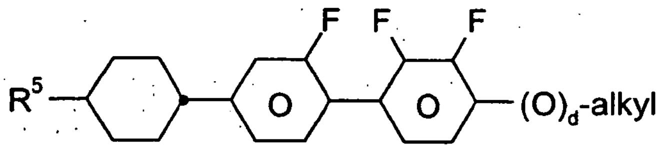

XIIIa

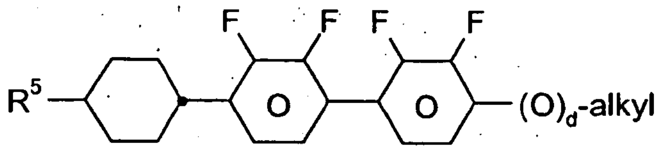

XIIIb

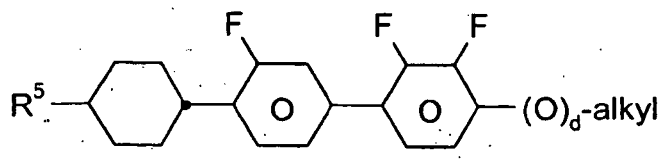

XIIIc

worin $R^5$ und "alkyl" die oben angegebenen Bedeutungen besitzen und d 0 oder 1 bedeutet. $R^5$ ist in diesen Verbindungen besonders bevorzugt $C_{1-6}$-alkyl oder -alkoxy, d ist vorzugsweise 1. Besonders bevorzugt sind Verbindungen der Formel Xb und XIb.

9) Medium, welches zusätzlich eine oder mehrere Verbindungen ausgewählt aus den folgenden Formeln enthält:

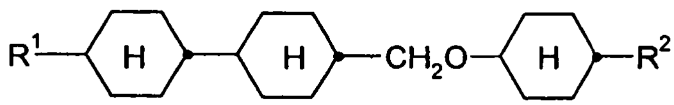

XIV1

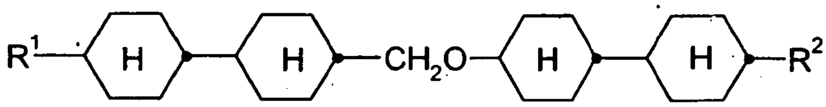

XIV2

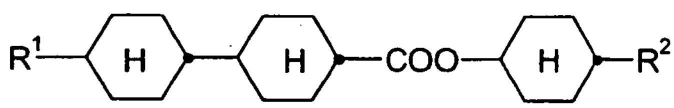

XIV3

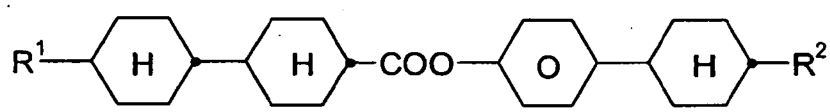

XIV4

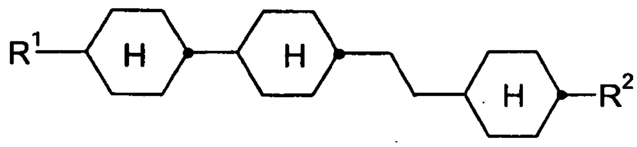

XIV5

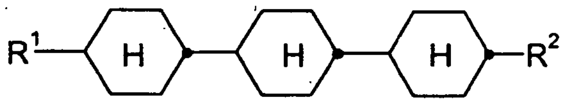

XIV6

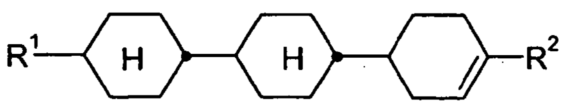

XIV7

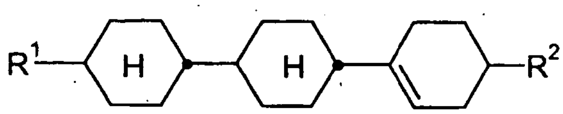

XIV8

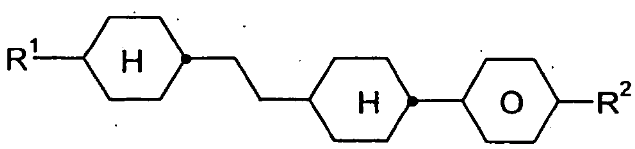

XIV9

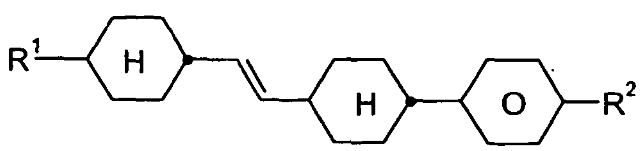

XIV10

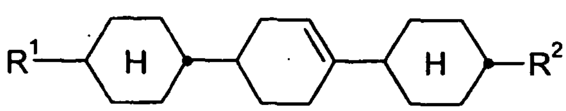

XIV11

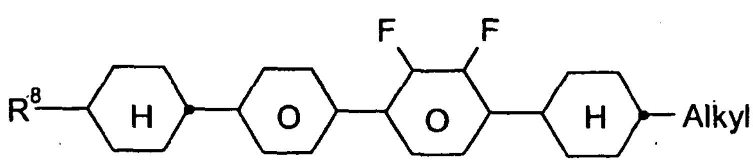

XV1

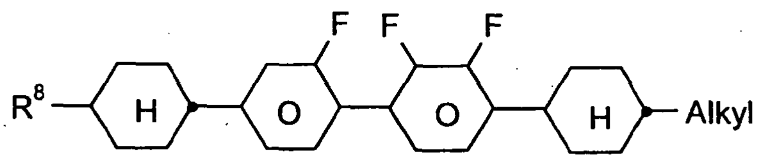

XV2

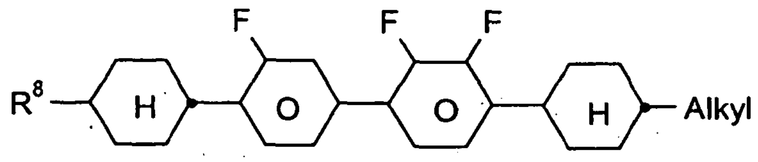

XV3

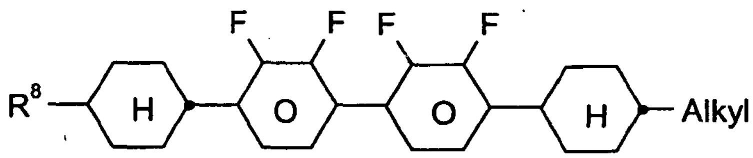

XV4

worin $R^5$ und $R^6$ die oben angegebenen Bedeutungen besitzen und vorzugsweise Alkyl mit 1 bis 8 C-Atomen bedeuten, und $R^8$ die für $R^1$ angegebene Bedeutung hat und Alkyl einen geradkettigen Alkylrest mit 1-6 C-Atomen bedeutet.

10) Medium, welches außer den polymerisierbaren Verbindungen der Formeln I und 11 und deren Unterformeln sowie den Comonomeren keine Verbindungen enthält, die eine Alkenylseitenkette mit einer endständigen Vinyl- oder Vinyloxygruppe ($-CH=CH_2$ oder $-O-CH=CH_2$) aufweisen.

11) Medium, welches 1 bis 8, vorzugsweise 1 bis 5 Verbindungen der Formel II2 und /oder II27 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.

12) Medium, welches 1 bis 8, vorzugsweise 1 bis 5 Verbindungen der Formel II8 und/oder II33 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.

13) Medium, welches 1 bis 8, vorzugsweise 1 bis 5 Verbindungen der Formel III enthält, insbesondere Verbindungen der Formel IIIa, IIIb, IIId, IIIe oder IIIf. Der Anteil an Verbindungen der Formel III im Gesamtgemisch beträgt vorzugsweise 2 bis 70 %, besonders bevorzugt 5 bis 60 %. Der Gehalt der einzelnen Verbindungen der Formel III beträgt vorzugsweise jeweils 1 bis 60 %.

14) Medium, welches 1 bis 10, vorzugsweise 1 bis 8 Verbindungen der Formel VIIc enthält.

15) Medium, welches 1 bis 10, vorzugsweise 1 bis 8 Verbindungen der Formel VII enthält, insbesondere Verbindungen der Formel VIIa und/oder VIId.

16) Medium, worin der Anteil an Verbindungen der Formel II33, IV2 und IV3 im Gesamtgemisch vorzugsweise 5 bis 50 %, besonders bevorzugt 10 bis 35 % beträgt. Der Gehalt der einzelnen Verbindungen der Formel II33 beträgt vorzugsweise jeweils 2 bis 15 %. Der Gehalt der einzelnen Verbindungen der Formel IV2 beträgt vorzugsweise jeweils 2 bis 10 %. Der Gehalt der einzelnen Verbindungen der Formel IV3 beträgt vorzugsweise jeweils 2 bis 20 %.

17) Medium, worin der Anteil an Verbindungen der Formel Va und Vb im Gesamtgemisch vorzugsweise bis zu 30 %, besonders bevorzugt bis zu 20 % beträgt. Der Gehalt der einzelnen Verbindungen der Formel Va und Vb beträgt vorzugsweise jeweils 2 bis 12 %.

18) Medium, worin der Anteil an Verbindungen mit einer Tetrahydronaphthyl- oder Naphthyleinheit (z.B. der Formel VIa-VIk) im Gesamtgemisch vorzugsweise bis zu 30 %, besonders bevorzugt bis zu 20 % beträgt. Der Gehalt der einzelnen Verbindungen dieses Typs beträgt vorzugsweise jeweils 2 bis 20%.

19) Medium, worin der Anteil an Verbindungen der Formel VII bis XV und XIX bis XXIV im Gesamtgemisch 10 bis 70 %, vorzugsweise 10 bis 60 % beträgt.

20) Medium, welches 1 bis 5, vorzugsweise 1, 2 oder 3 polymerisierbare Verbindungen enthält.

21) Medium, worin der Anteil an polymerisierbaren Verbindungen im Gesamtgemisch 0,05 bis 5 %, vorzugsweise 0,1 bis 1 % beträgt.

22) Medium, welches zusätzlich eine oder mehrere Verbindungen der folgenden Formeln enthält, insbesondere zur Anwendung in OCB-Anzeigen:

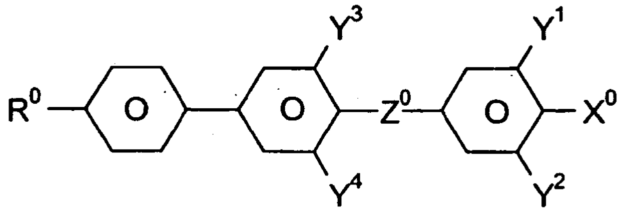

XVI

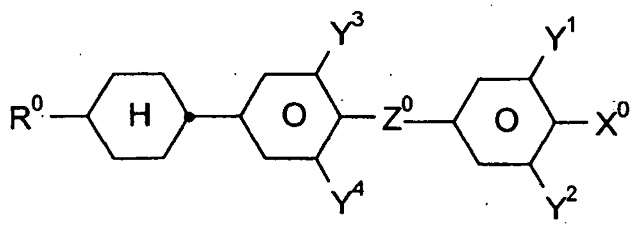

XVII

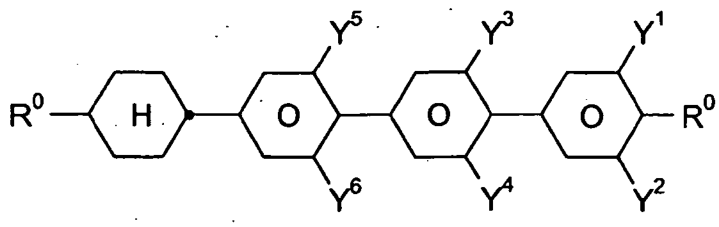

XVIII

worin

$R^0$ bei jedem Auftreten gleich oder verschieden n-Alkyl, Alkoxy, Oxaalkyl, Fluoralkyl oder Alkenyl mit jeweils bis zu 9 C-Atomen,
$X^0$ F, Cl oder jeweils halogeniertes Alkyl, Alkenyl, Alkenyloxy oder Alkoxy mit jeweils bis zu 6 C-Atomen,
$Z^0$ $-CF_2O-$ oder eine Einfachbindung,
$Y^{1-6}$ jeweils unabhängig voneinander H oder F,

bedeuten.

$X^0$ ist vorzugsweise F, Cl, $CF_3$, $CHF_2$, $OCF_3$, $OCHF_2$, $OCFHCF_3$, $OCFHCHF_2$, OCFHCHF2, $OCF_2CH_3$, $OCF_2CHF_2$, $OCF_2CHF_2$, $OCF_2CF_2CHF_2$, $OCF_2CF_2CHF_2$, $OCFHCF_2CF_3$, $OCFHCF_2CHF_2$, $OCF_2CF_2CF_3$, $OCF_2CF_2CClF_2$, $OCClFCF_2CF_3$ oder $CH=CF_2$, besonders bevorzugt F oder $OCF_3$.

Die Verbindungen der Formel XVI sind vorzugsweise ausgewählt aus den folgenden Formeln:

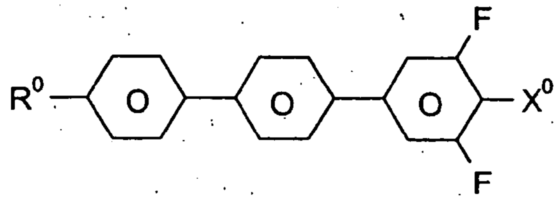

XVIa

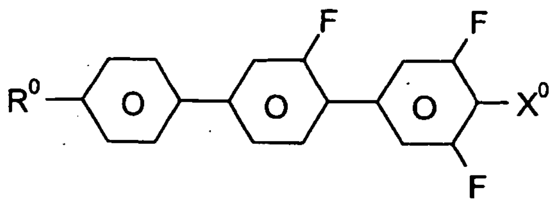

XVIb

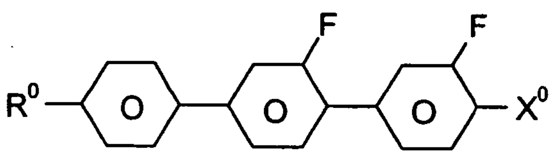

XVIc

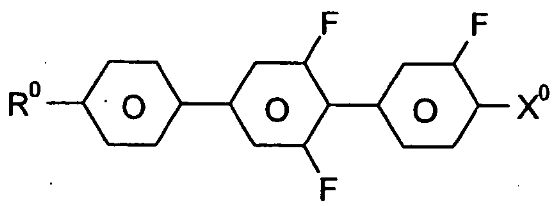

XVId

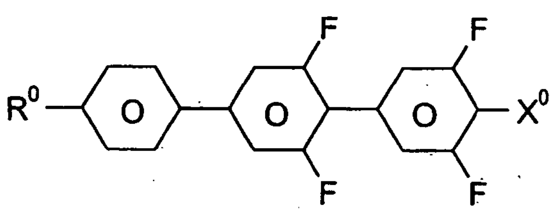

XVIe

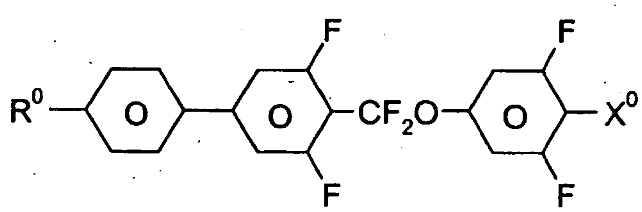

XVIf

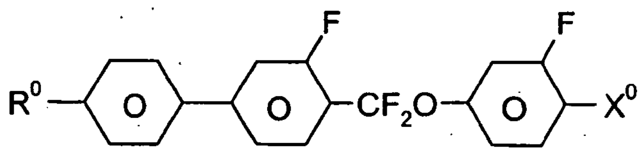

XVIg

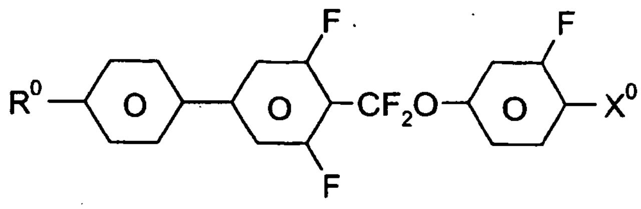

XVIh

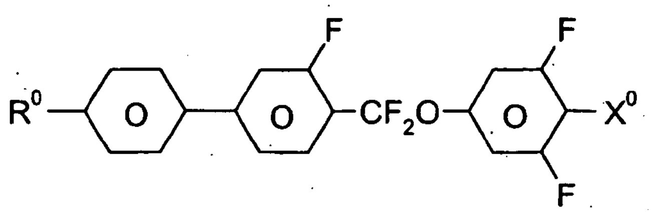

XVIi

worin $R^0$ und $X^0$ die oben angegebene Bedeutung besitzen, und $X^0$ vorzugsweise F bedeutet. Besonders bevorzugt sind Verbindungen der Formeln XVIb und XVIf.

Die Verbindungen der Formel XVII sind vorzugsweise ausgewählt aus den folgenden Formeln-.

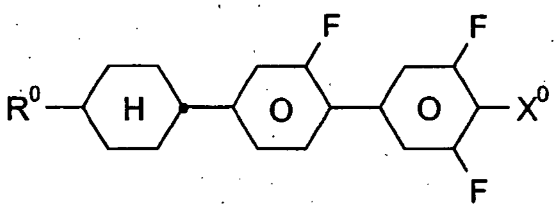

XVIIa

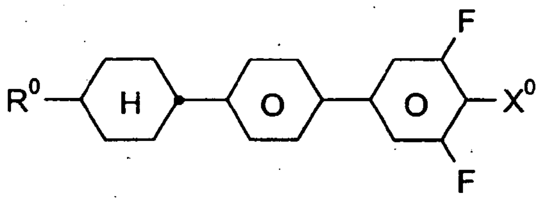

XVIIb

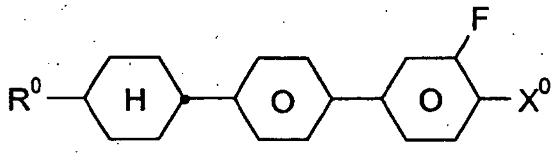

XVIIc

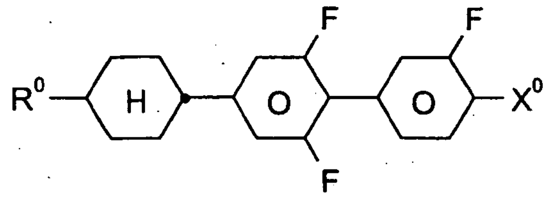

XVIId

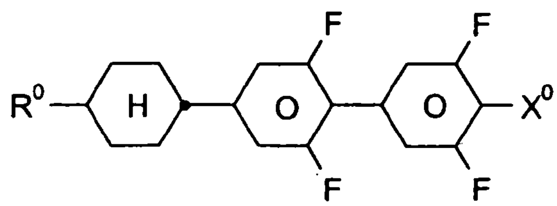

XVIIe

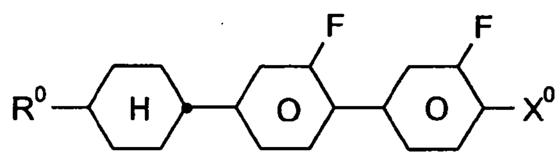

XVIIf

worin $R^0$ und $X^0$ die oben angegebene Bedeutung besitzen, und $X^0$ vorzugsweise F bedeutet. Besonders bevorzugt sind Verbindungen der Formeln XVIIa, XVIIb und XVIIe.
Die Verbindungen der Formel XVIII sind vorzugsweise ausgewählt aus den folgenden Formeln:

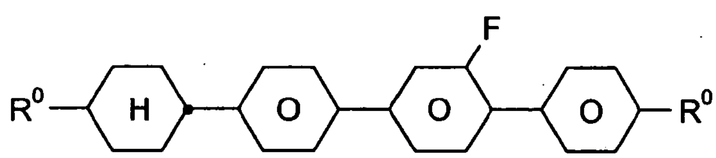

XVIIIa

worin $R^0$ bei jedem Auftreten gleich oder verschieden die oben angegebene Bedeutung besitzt, und vorzugsweise Alkyl mit 1 bis 6 A-Atomen bedeutet.

23) Medium, welches zusätzlich eine oder mehrere Verbindungen der folgenden Formel enthält:

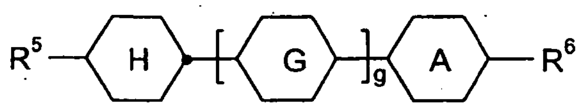

XIX

worin die einzelnen Reste folgende Bedeutung haben:

$R^5$ und $R^6$ jeweils unabhängig voneinander eine der oben für $R^1$ angegebenen Bedeutungen,

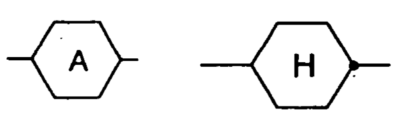

oder

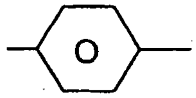 ,

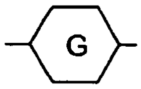 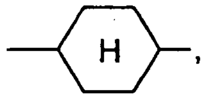, 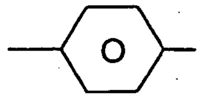

oder

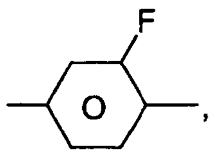,

und

g 1 oder 2.

Die Verbindungen der Formel XIX sind vorzugsweise ausgewählt aus den folgenden Unterformeln:

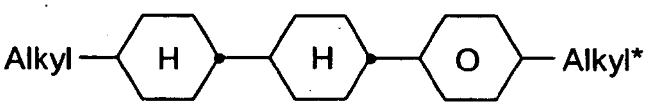

XIX1

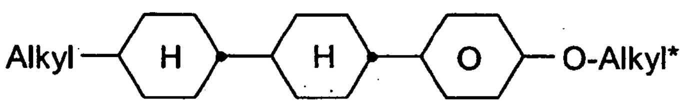

XIX2

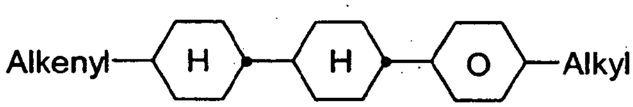

XIX3

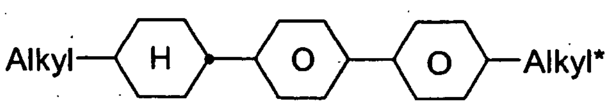

XIX4

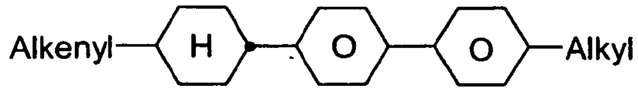

XIX5

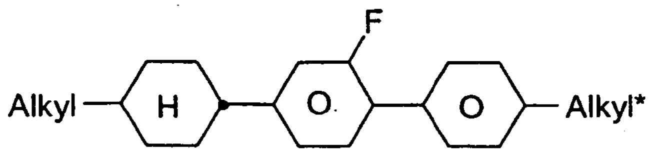

XIX6

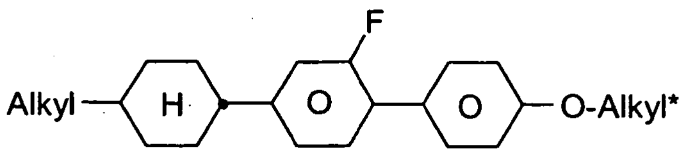

XIX7

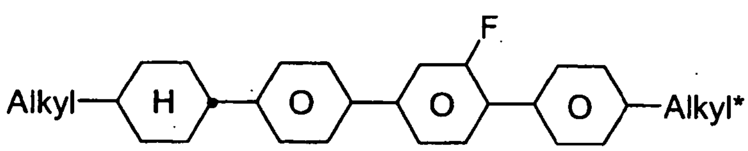

XIX8

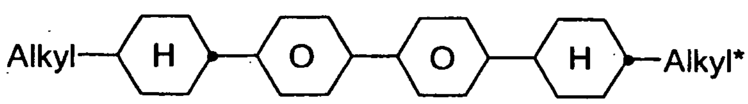

XIX9

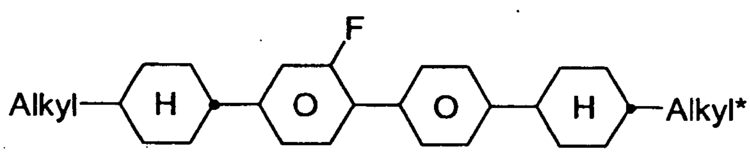

XIX10

worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten.

24) Medium, welches zusätzlich eine oder mehrere Verbindungen der folgenden Formel enthält:

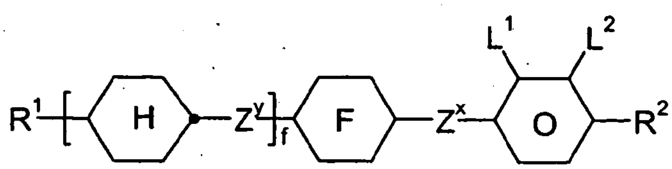

XX

worin die einzelnen Reste folgende Bedeutung besitzen

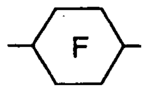 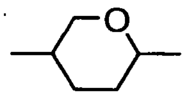, 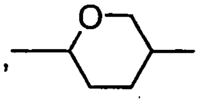, 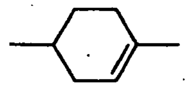

oder

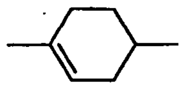

f 0 oder 1,

$R^1$ und $R^2$ jeweils unabhängig voneinander Alkyl mit 1 bis 12 C-Atomen, wobei auch eine oder zwei nicht benachbarte $CH_2$-Gruppen durch -O-, -CH=CH-, -CO-, -OCO- oder-COO- so ersetzt sein können, dass O-Atome nicht direkt miteinander verknüpft sind,

$Z^x$ und $Z^y$ jeweils unabhängig voneinander $-CH_2CH_2-$, -CH=CH-, $-CF_2O-$, $-OCF_2-$, $-CH_2O-$, $-OCH_2-$, -COO-, -OCO-, $-C_2F_4-$, -CF=CF-, $-CH=CHCH_2O-$, oder eine Einfachbindung, vorzugsweise eine Einfachbindung,

$L^1$ und $L^2$ jeweils unabhängig voneinander F, Cl, $OCF_3$; $CF_3$, $CH_3$, $CH_2F$, $CHF_2$.

Vorzugsweise bedeuten beide Reste $L^1$ und $L^2$ F oder einer der Reste $L^1$ und $L^2$ F und der andere Cl.
Die Verbindungen der Formel XX sind vorzugsweise ausgewählt aus den folgenden Unterformeln

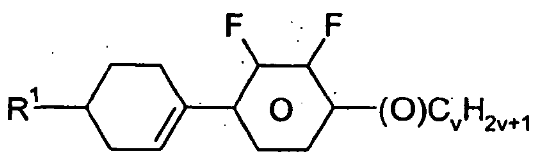

XX1

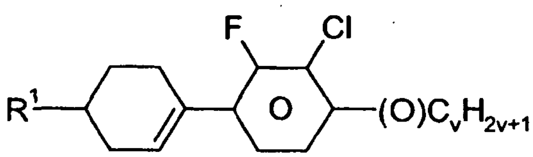

XX2

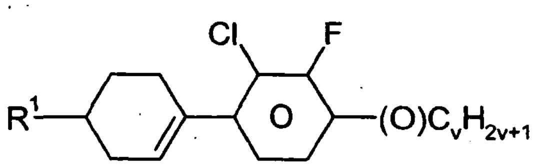

XX3

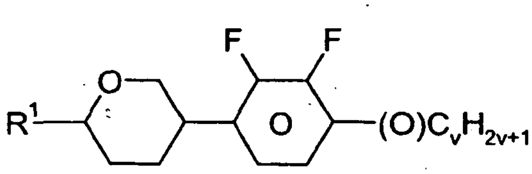

XX4

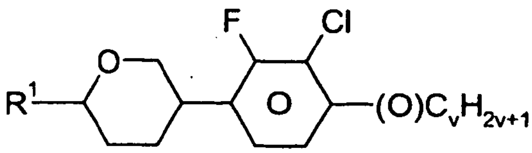

XX5

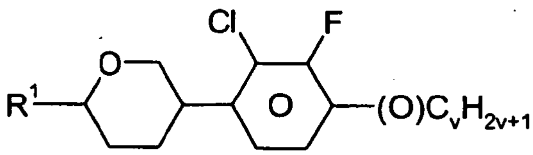

XX6

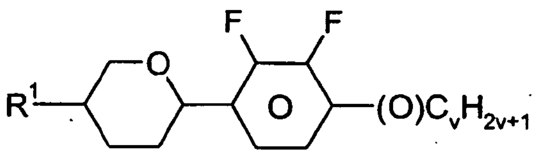

XX7

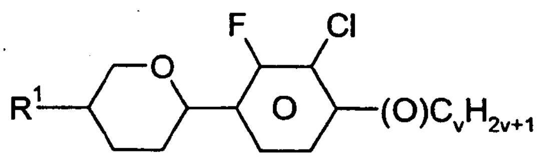

XX8

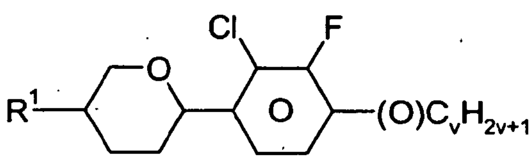

XX9

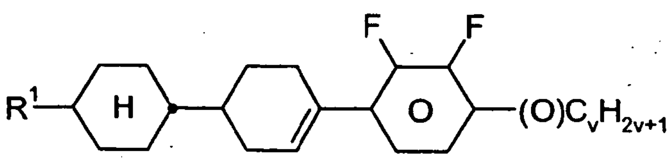

XX10

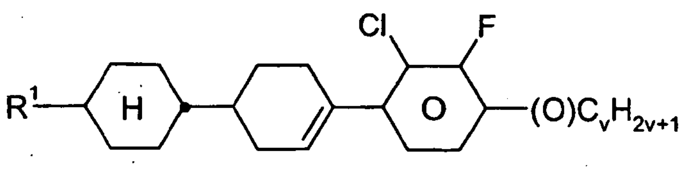

XX11

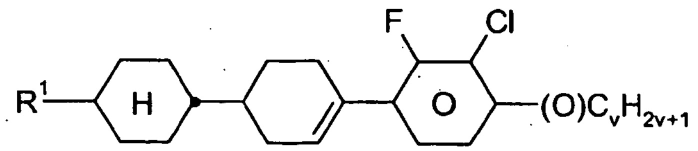

XX12

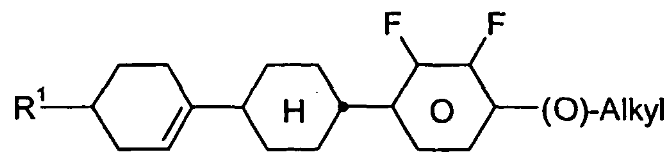

XX13

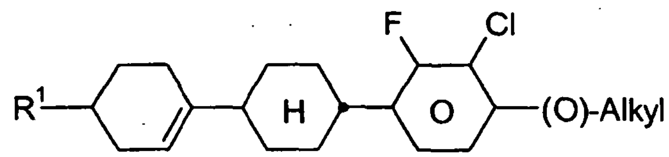

XX14

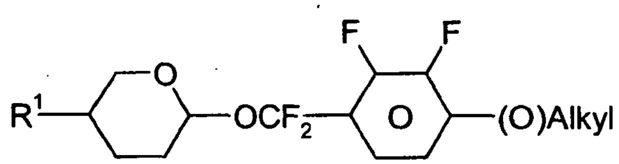

XX15

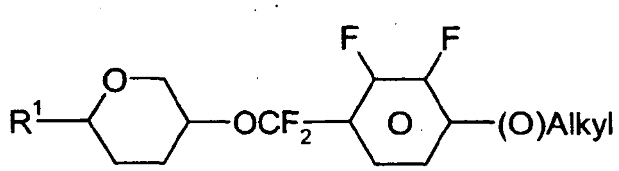

XX16

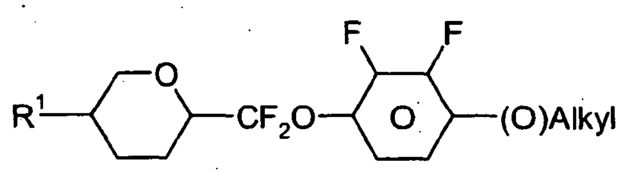

XX17

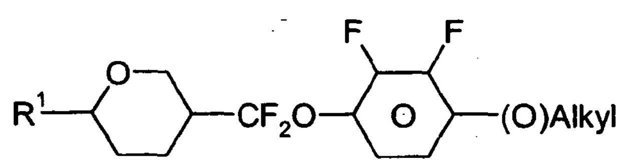

XX18

worin $R^1$ die oben angegebene Bedeutung hat und v eine ganze Zahl von 1 bis 6 bedeutet. $R^1$ bedeutet vorzugsweise

geradkettiges Alkyl oder Alkenyl.

25) Medium, welches zusätzlich eine oder mehrere eine oder mehrere Difluordibenzochromane und/oder Chromane der folgenden Formeln enthält:

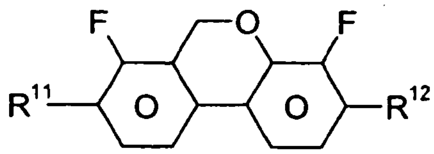

XXI

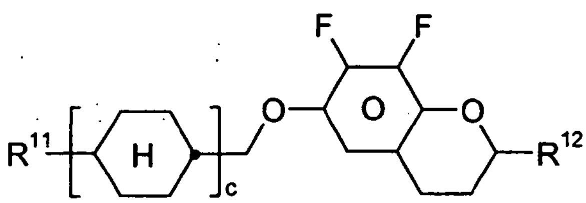

XXII

worin $R^{11}$ und $R^{12}$ jeweils unabhängig voneinander die oben angegebene Bedeutung aufweisen, und c 0 oder 1 bedeutet, vorzugsweise in Mengen von 3 bis 20 Gew.%, insbesondere in Mengen von 3 bis 15 Gew.%. Besonders bevorzugte Verbindungen der Formeln XXI und XXII sind ausgewählt aus den folgenden Unterformeln:

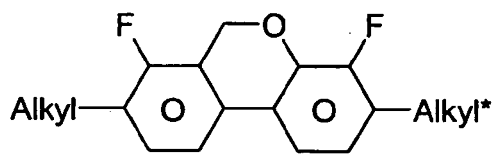

XXI1

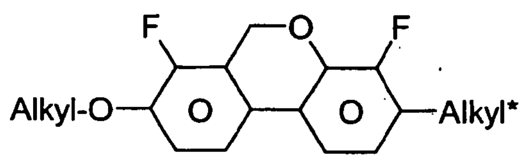

XXI2

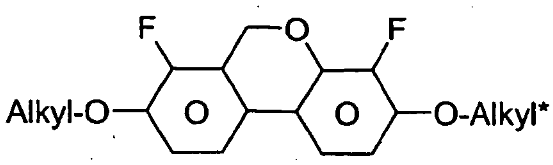

XXI3

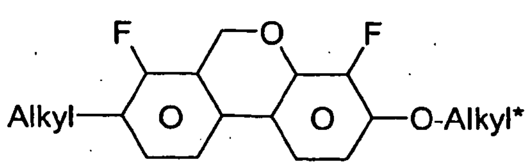

XXI4

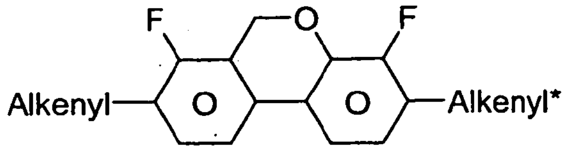

XXI5

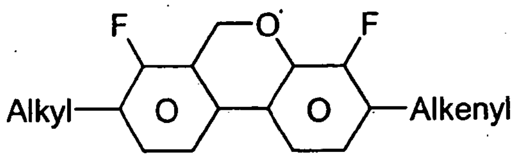

XXI6

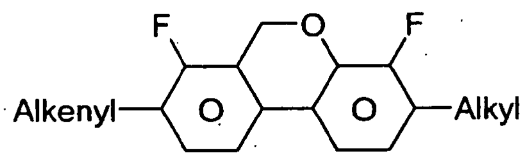

XXI7

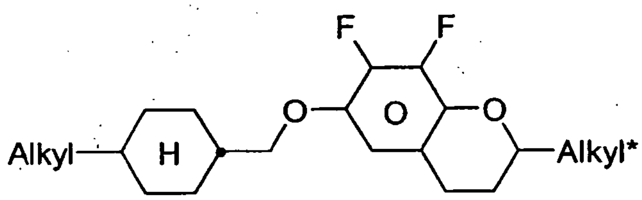

XXII1

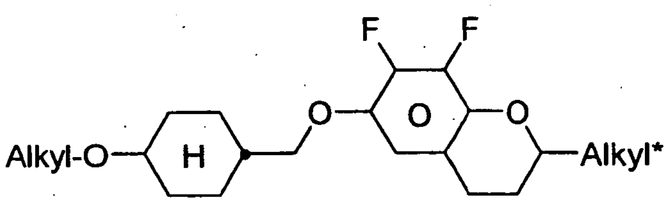

XXII2

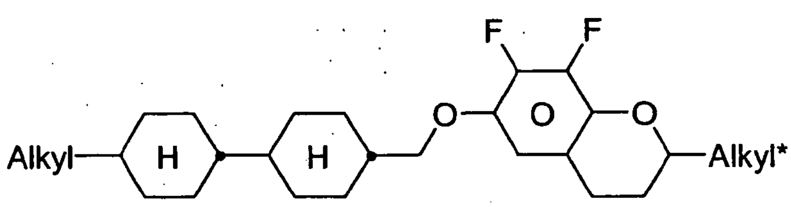

XXII3

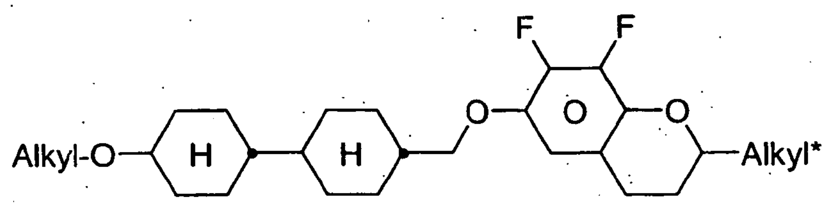

XXII4

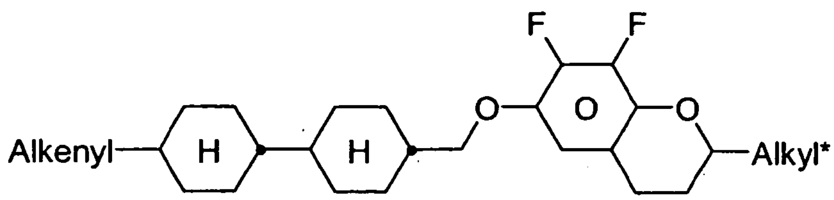

XXII5

worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten.

26) Medium, welches zusätzlich eine oder mehrere fluorierte Phenanthrene oder Dibenzofurane der folgenden Formeln enthält:

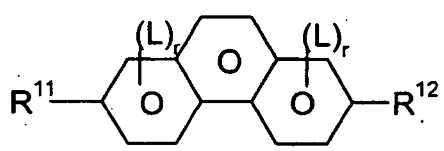

XXIII

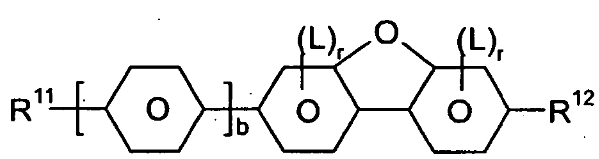

XXIV

worin $R^{11}$ und $R^{12}$ jeweils unabhängig voneinander die oben angegebenen Bedeutungen besitzen, b 0 oder 1, L F und r 1, 2 oder 3 bedeutet.

Besonders bevorzugte Verbindungen der Formeln XXIII und XXIV sind ausgewählt aus den folgenden Unterformeln:

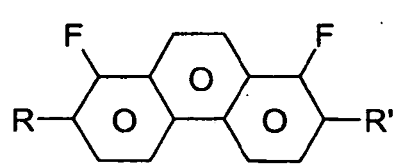

XXIII1

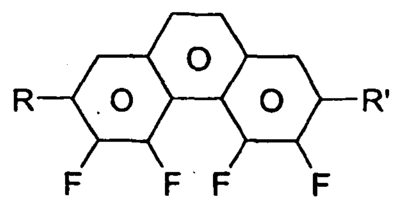

XXIII2

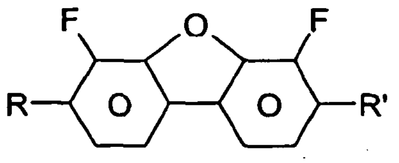

XXIV1

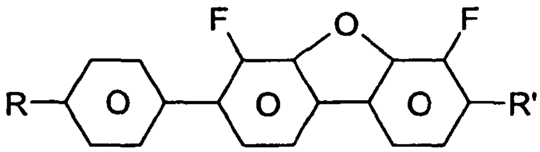

XXIV2

worin R und R' jeweils unabhängig voneinander einen geradkettigen Alkyl- oder Alkoxyrest mit 1-7 C-Atomen bedeuten.

Die Kombination von Verbindungen der Formel II-XXIV mit den oben beschriebenen polymerisierten Biarylverbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die Einstellung eines Pretilt-Winkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPa·s, bei 20°C auf.

Erfindungsgemäße FK-Medien zur Verwendung in Anzeigen des VA-Typs weisen eine negative dielektrische Anisotropie $\Delta\varepsilon$ auf, vorzugsweise von etwa -0,5 bis -7,5, insbesondere von etwa -2,8 bis -5,5 bei 20°C und 1 kHz.

Erfindungsgemäße FK-Medien zur Verwendung in Anzeigen des OCB-Typs weisen eine positive dielektrische Anisotropie $\Delta\varepsilon$ auf, vorzugsweise von etwa +7 bis +17 bei 20°C und 1 kHz.

Die Doppelbrechung $\Delta n$ in erfindungsgemäßen FK-Medien zur Verwendung in Anzeigen des VA-Typs liegt vorzugsweise unter 0,16, besonders bevorzugt zwischen 0,06 und 0,14, insbesondere zwischen 0,07 und 0,12.

Die Doppelbrechung $\Delta n$ in erfindungsgemäßen FK-Medien zur Verwendung in Anzeigen des OCB-Typs liegt vorzugsweise zwischen 0,14 und 0,22, insbesondere zwischen 0,16 und 0,22.

Die Dielektrika können auch weitere, dem Fachmann bekannte und in der Literatur beschriebene Zusätze enthalten. Beispielsweise können 0 bis 15 Gew.-% pleochroitische Farbstoffe zugesetzt werden, ferner Nanopartikel, Leitsalze, vorzugsweise Ethyl-dimethyldodecylammonium-4-hexoxy-benzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der Formel II bis XXIV der erfindungsgemäßen FK-Mischungen sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel II werden beispielsweise in EP-A-0 364 538 beschrieben. Entsprechende Verbindungen der Formel III werden beispielsweise in EP-A-0 122 389 beschrieben. Entsprechende Verbindungen der Formel VII werden beispielsweise DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere Verbindungen der Formel II-XXIVI mit einer oder mehreren polymerisierbaren

Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen FK-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele sollen die Erfindung erläutern, ohne sie zu begrenzen. Vor- und nachstehend bedeuten Prozentangaben Gewichtsprozent, sofern nicht anders angegeben; alle Temperaturen sind in Grad Celsius angegeben.

Folgende Abkürzungen und Acronyme werden verwendet:

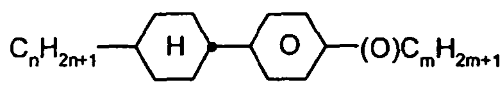

**PCH-n(O)m**

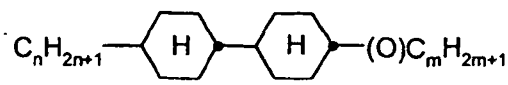

**CCH-n(O)m**

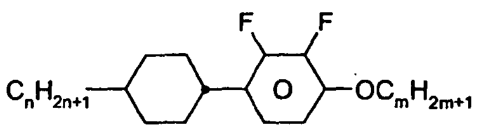

**CY-n-Om**

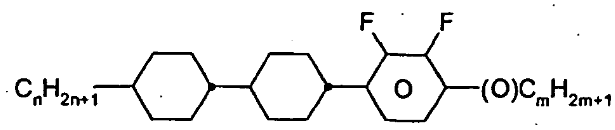

**CCY-n-(O)m**

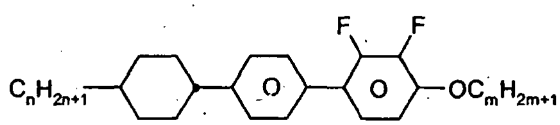

**CPY-n-Om**

Weiterhin bedeuten:

| | |
|---|---|
| Tg | Glasübergang |
| N | nematische Phase |
| I | isotrope Phase |
| Kp. | Klärpunkt [°C] |
| $\Delta n$ | optische Anisotropie (Doppelbrechung) bei 20°C und 589 nm |
| $\Delta\varepsilon$ | dielektrische Anisotropie bei 20°C und 1 kHz |
| $\varepsilon_{\parallel}$ | Dielektrizitätskonstante parallel zum Direktor bei 20°C und 1 kHz |
| $K_3/K_1$ | Verhältnis der elastischen Konstanten $K_3$ und $K_1$ |
| $\gamma_1$ | Rotationsviskosität [mPa·s] (bei 20°C, sofern nicht anders angegeben) |
| $V_0$ | kapazitive Schwellenspannung [V] |
| $\tau$ | Schaltzeit in msec |

Soweit nicht explizit anders vermerkt, sind in der vorliegenden Anmeldung alle Konzentrationen in Gewichtsprozent angegeben, und beziehen sich auf die entsprechende Mischung oder Mischungskomponente soweit nicht

explizit anders angegeben.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S, N) und der Klärpunkt T(N,I), in Grad Celsius (°C) angegeben.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und $\Delta n$ wird bei 589 nm und $\Delta\varepsilon$ bei 1 kHz bestimmt, sofern nicht jeweils explizit anders angegeben.

Die zur Messung der kapazitiven Schwellenspannung verwendete Anzeige weist zwei planparallele Trägerplatten im Abstand von 4 $\mu$m und Elektrodenschichten mit darüberliegenden Orientierungsschichten aus geriebenem Polyimid auf den Innenseiten der Trägerplatten auf, welche eine homöotrope Randorientierung der Flüssigkristallmoleküle bewirken.

Die polymerisierbaren Verbindungen werden in der Anzeige durch UV-Bestrahlung, z.B. mit einer Stärke von 28 mW/cm$^2$, und vorgegebener Zeit polymerisiert, wobei gleichzeitig eine Spannung an die Anzeige angelegt wird (üblicherweise 10V bis 30V Wechselstrom).

Der Tiltwinkel wird per Drehkristall-Experiment (Autronic-Melchers TBA-105) bestimmt. Ein kleiner Wert (d.h. eine große Abweichung vom 90°-Winkel) entspricht dabei einem großen Tilt.

Beispiel 1

2-Methylacrylsäure-2'-(2-methyl-acryloyloxy)[1,1'] binaphthalinyl-2-ylester (Verbindung 1)

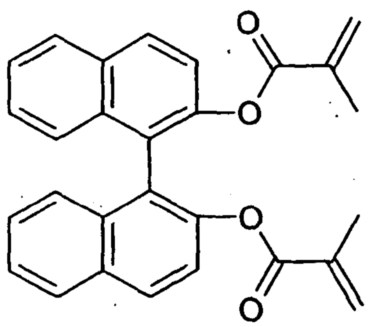

(1)

Die Verbindung ist als Racemat beschrieben von S. Zheng und D. Y. Sogah, Polymer Preprints 2001, 42(1), 452-453, und wird in optisch aktiver Form aus (+)-R-1,1'-Bi-2-naphthol in analoger Weise erhalten.

Phasenverhalten: Tg 12°C I

In analoger Weise wird Verbindung (15) hergestellt:

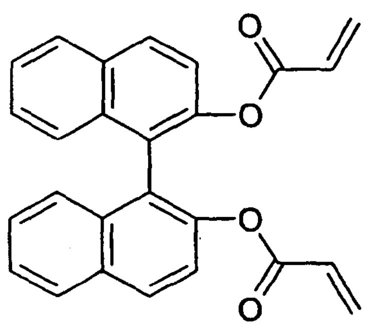

(15)

Phasenverhalten: Tg 16 K 128 I

Beispiel 2

(±)-2-Methyl-acrylsäure-2'-(2-methyl-acryloyloxy)-6,6'-diphenyl-[1,1'] binaphthalinyl-2-ylester (Verbindung 2) wird wie folgt hergestellt:

Stufe 1: (±)-6,6'-Diphenyl-[1,1']binaphthalinyl-2,2'-diol

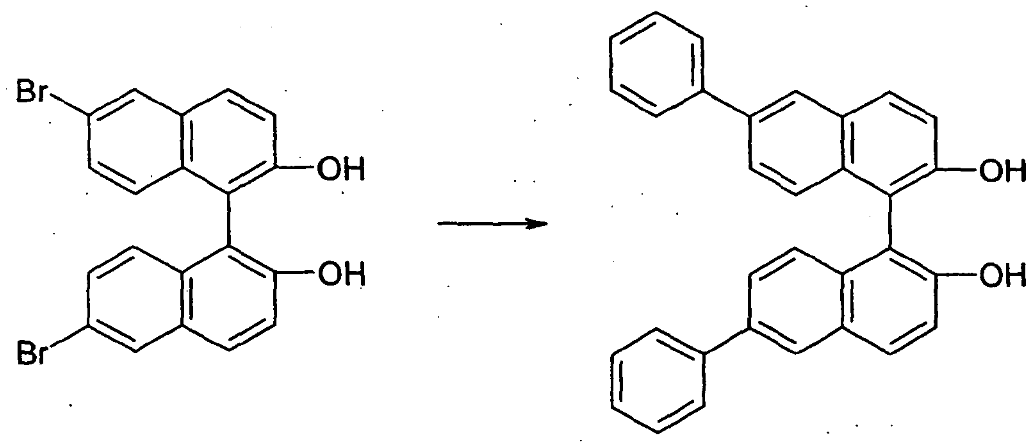

12,0 g (25,7 mmol) (±)-6,6'-Dibrom-[1,1']binaphthalinyl-2,2'-diol, 11,5 g (41 mmol) Natriummetaborat-Oktahydrat und 800 mg Bistriphenylphosphinpalladium(II)chlorid werden in 100 ml Wasser und 20 ml THF vorgelegt, 0,13 ml Hydrazinhydrat hinzugefügt und eine Lösung von 7,0 g (57 mmol) Benzolboronsäure in 80 ml THF zutropfen gelassen. Anschließend wird der Ansatz über Nacht unter Rückfluß erhitzt, auf Wasser gegeben und dreimal mit MTB-Ether extrahiert. Die vereinigten org. Phasen werden mit Wasser gewaschen und über Natriumsulfat getrocknet. Das Lösungsmittel wird i. Vak. entfernt und der Rückstand mit Toluol/Essigester (19:1) über Kieselgel filtriert, eingeengt und das Produkt je einmal aus Toluol/Cyclohexan (1:1) und Isopropanol umkristallisiert. Man erhält (±)-6,6'-Diphenyl-[1,1']binaphthalinyl-2,2'-diol als farblose Kristalle.
**MS(EI)**: m/z (%) = 438 [$M^+$] (100).

Stufe 2: (±)-2-Methyl-acrylsäure-2'-(2-methyl-acryloyloxy)-6,6'-diphenyl-[1,1']bi-naphthaiinyi-2-ylester

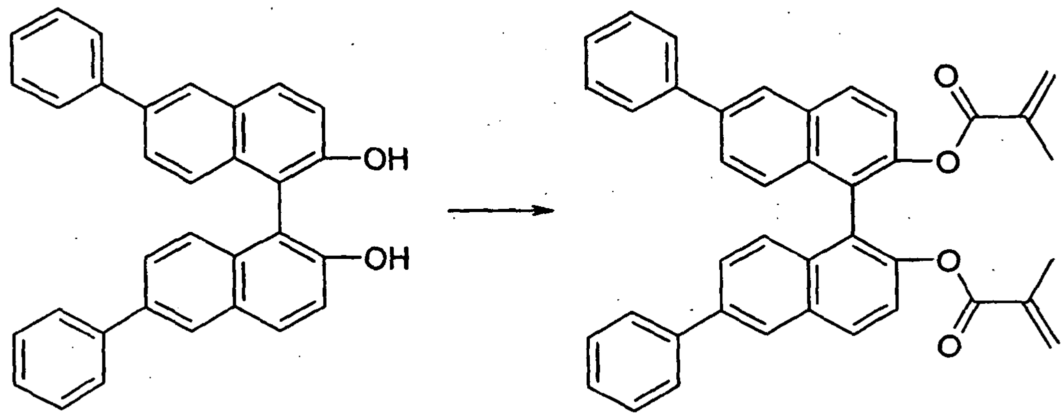

(2)

6,6 g (14,5 mmol) (±)-6,6'-Diphenyl-[1,1']binaphthalinyl-2,2'-diol und 11 ml (80 mmol) Triethylamin werden in 350 ml Dichlormethan gelöst und unter Eiskühlung mit 3,7 ml (37,8 mmol) Methacrylsäurechlorid versetzt. Der Ansatz wird über Nacht bei Raumtemp. gerührt, eingeengt und der Rückstand mit Toluol/Essigester (19:1) an Kieselgel chromatographiert.

Kristallisation des Rohproduktes aus Diethylether liefert farblose Kristalle vom Schmp. 180 °C (Racemat).

Die reinen, optisch aktiven Enantiomere von (2) können analog der oben beschriebenen Synthese aus kommerziell erhältlichem (R)- (CAS-Nr. 65283-60-5) oder (S)- 6,6'-Dibrom-[1,1']binaphthalinyl-2,2'-diol (CAS-Nr. 80655-81-8) erhalten werden.

Beispiel 3

Verbindung (3) wird wie folgt hergestellt:

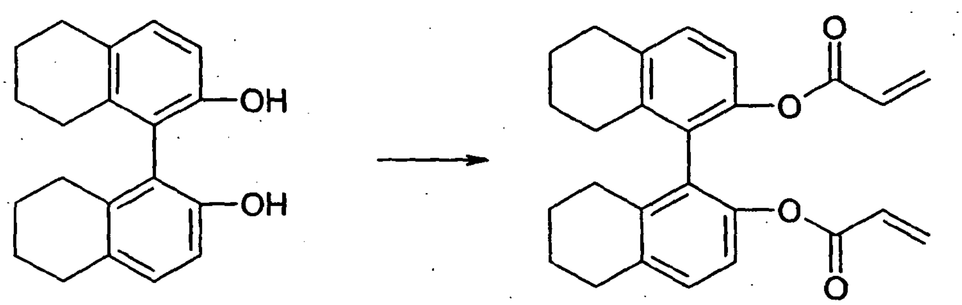

(3)

Ausgehend von 5,6,7,8,5',6',7',8'-Octahydro-[1,1']binaphthalenyl-2,2'-diol, das nach D.J. Cram et al., J. Org. Chem. 1981, 46, 393-406 durch katalytisch Hydrierung von [1,1']Binaphthalenyl-2,2'-diol zugänglich ist, erhält man in Analogie zu Beispiel 1 und 2 durch Umsetzung mit Acrylsäurechorid den Acrylsäure-2'-acryloyloxy-5,6,7,8,5',6',7',8'-octahydro-[1,1']binaphthalenyl-2-ylester als farblosen Feststoff.

In analoger Weise werden folgende Verbindungen hergestellt (Me = Methyl, Ph = Phenyl):

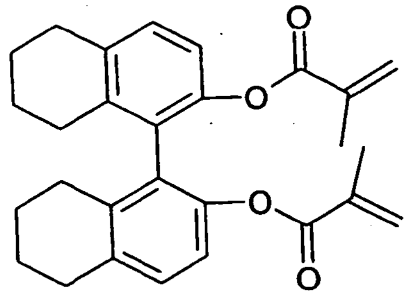

(4)

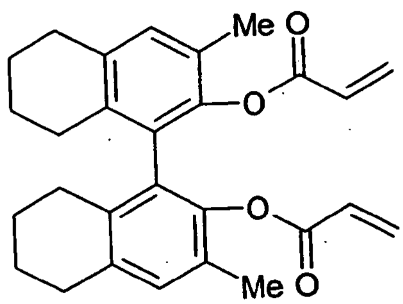

(5)

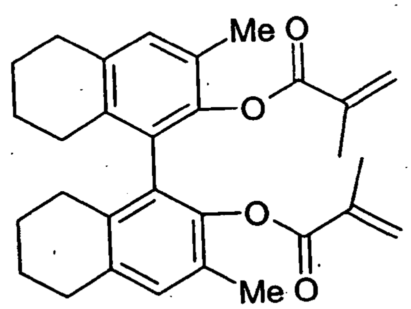

(6)

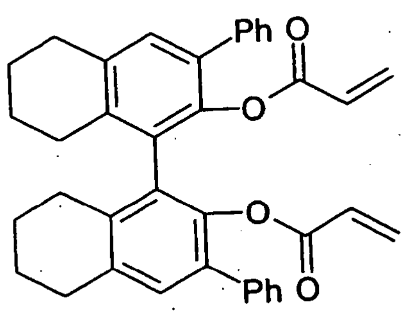

(7)

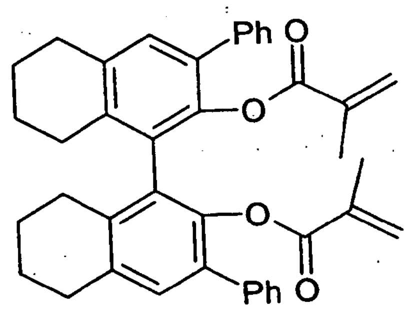

(8)

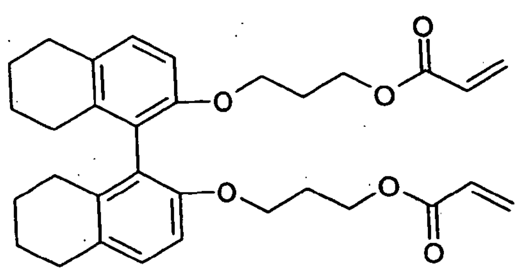

(9)

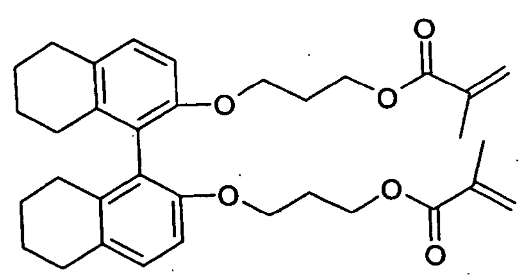

(10)

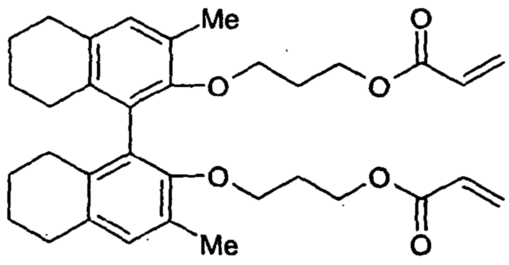

(11)

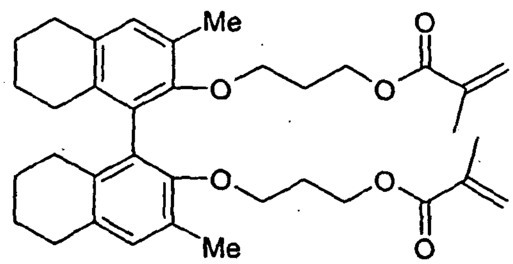

(12)

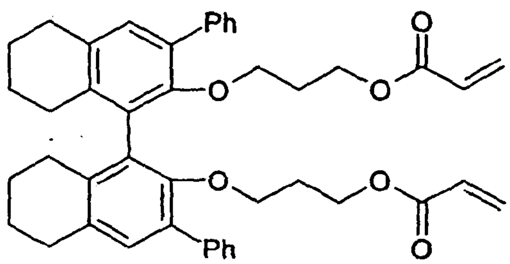

(13)

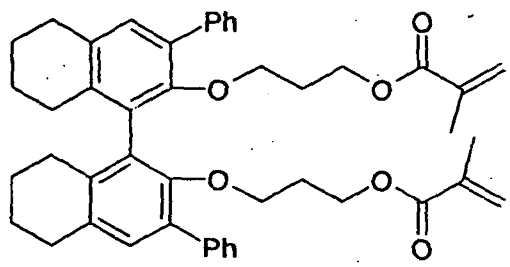

(14)

Beispiel 4

Verbindung (16):

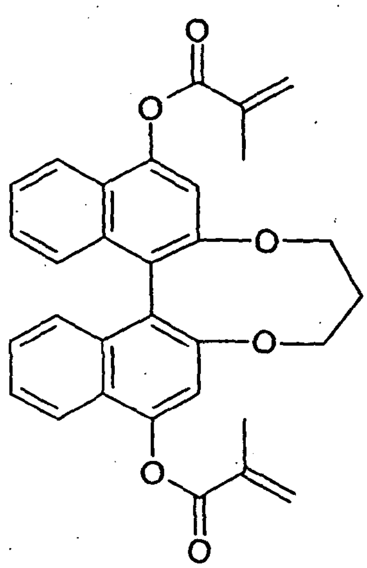

(16)

Die Herstellung des Ausgangsmaterials 4,4'-Dibrom-[1,1']binaphthaünyl-2,2'-diol erfolgt nach M. Noji, M. Nakajima, K. Koga, Tetrahedron Lett. 1994, 35, 7983-7984, aus 4-Brom-2-naphthol, hergestellt nach M. S. Newman, V. Sankaran, D. R. Olson, J. Am. Chem. Soc. 1976, 98, 3237-3241. Die Trennung der Enantiomeren erfolgt durch HPLC

(Säule: Chiralpak AD-H 20 um, Eluens: Ethanol).

Stufe 1: 9,17-Dibrom-13,14-dihydro-12H-naphtho[2,1,10,13-fgh][1,5]dioxonin

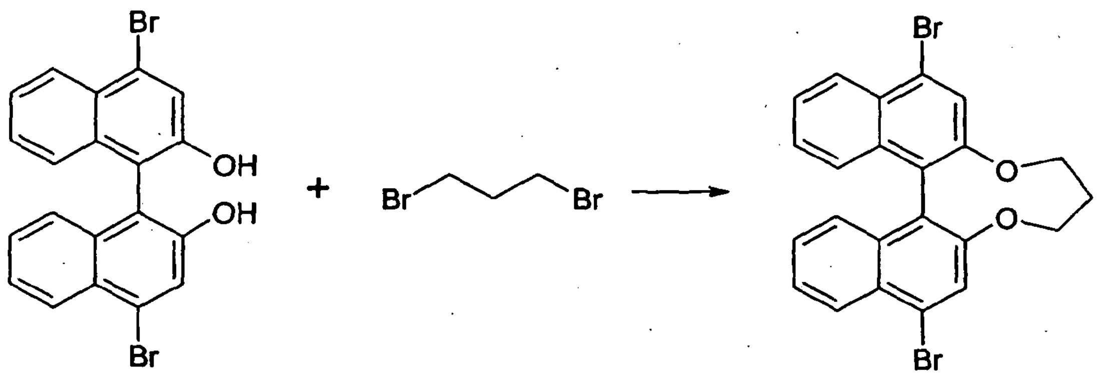

5,66 g (12,7 mmol) 4,4'-Dibrom-[1,1']binaphthalinyl-2,2'-diol, 9,5 g (29,2 mmol) Cäsiumcarbonat werden in 75 ml DMF vorgelegt und bei 80 °C mit 1,4 ml (13,4 mmol) 1,3-Dibrompropan versetzt. Der Ansatz wird über Nacht rühren gelassen, auf Wasser gegeben und mit Toluol extrahiert. Die vereinigten org. Phasen werden eingeengt und der Rückstand mit Toluol über Kieselgel filtriert. Man erhält 9,17-Dibrom-13,14-dihydro-12H-naphtho[2,1,10,13-fgh][1,5]dioxonin als farblose Kristalle.

$^{1}$H-NMR (400 MHz, $CDCl_3$):

$\delta$ = 1,99 ($m_c$, 2 H, $-OCH_2CH_2CH_2O-$), 4,35 ($m_c$, 4 H, $-OCH_2CH_2CH_2O-$), 7,26 ($m_c$, 4 H, Ar-H), 7,48 (ddd, J = 1,4 Hz, J = 6,64 Hz, J = 8,56 Hz, 2 H, Ar-H), 7,78 (s, 2 H, Ar-H), 8,27 (d, J = 8,6 Hz, 2 H, Ar-H).

Stufe 2: 13,14-Dihydro-12H-naphtho[2,1,10,13-fgh][1,5]dioxonin-9,17-diyl-9,17-diboronsäure

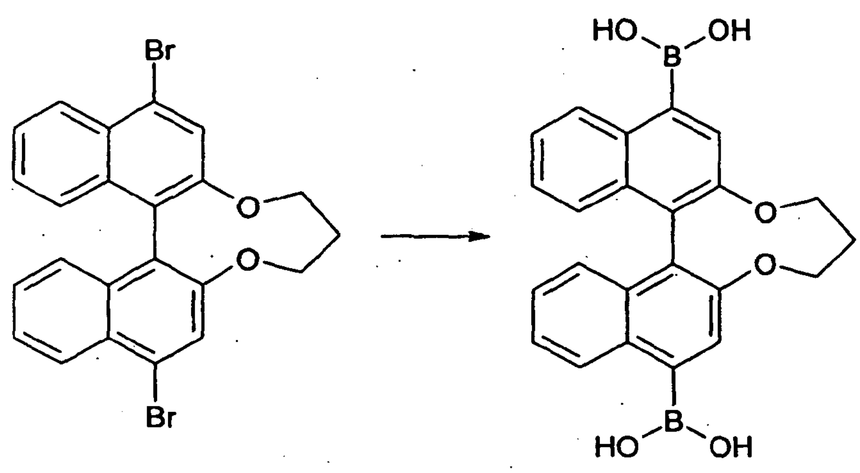

4,00 g (8,26 mmol) 9,17-Dibrom-13,14-dihydro-12H-naphtho[2,1,10,13-fgh][1,5]-dioxonin werden in 100 ml Dioxan gelöst, 5,0 g (50,9 mmol) Kaliumacetat, 6,5 g (25,1 mmol) Bis(pinacolato)dibor und 400 mg (0,545 mmol) 1,1'-Bis(diphenylphosphino)ferrocenpalladium(II)chlorid hinzugefügt und 4 h unter Rückfluß erhitzt. Anschließend wird der Ansatz auf Wasser gegeben, mit MTB-Ether extrahiert und über Natriumsulfat getrocknet. Das Lösungsmittel wird i. Vak. entfernt, der Rückstand mit Heptan/Essigester (2:1) über Kieselgel filtriert, eingeengt, in 200 ml Aceton aufgenommen, mit 9 g (45 mmol) Natriumperiodat und 3,2 g (45 mmol) Ammoniumacetat versetzt, über Nacht bei Raumtemp. und bis zum vollständigen Umsatz (DC) bei 45 °C rühren gelassen. Anschließend wird das Lösungsmittel i. Vak. entfernt, der Rückstand mit Wasser versetzt und mit MTB-Ether extrahiert. Die vereinigten org. Phasen werden über Natriumsulfat getrocknet. Nach entfernen des Lösungsmittels erhält man 13,14-Dihydro-12H-naphtho[2,1,10,13-fgh][1,5]dioxonin-9,17-diyl-9,17-diboronsäure, die ohne Aufreinigung in der nächsten Stufe eingesetzt wird.

Stufe 3: 9,17-Dihydroxy-13,14-dihydro-12H-naphtho[2,1,10,13-fgh][1,5]dioxonin

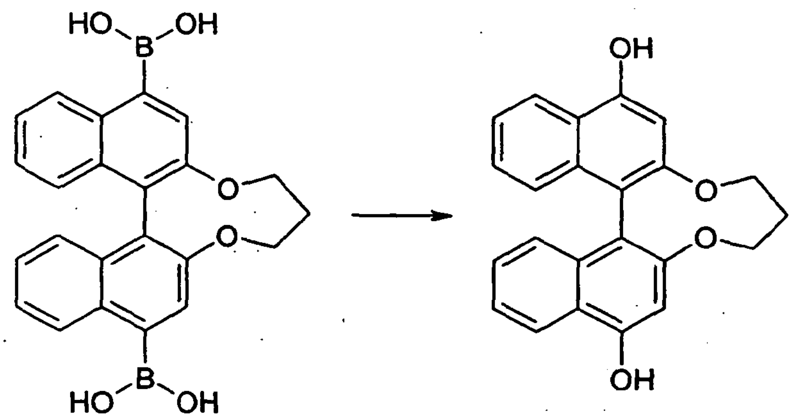

2,6 g (6,4 mmol) 13,14-Dihydro-12H-naphtho[2,1,10,13-fgh][1,5]dioxonin-9,17-diyl-9,17-diboronsäure werden in 50 ml THF vorgelegt, 2 ml Wasser und 2,2 ml Eisessig hinzugegeben und anschließend unter Eiskühlung 3,5 ml (36 mmol) 35proz. Wasserstoffperoxid langsam hinzugefügt. Der Ansatz wird 3 h bei Raumtemp. rühren gelassen, mit Wasser verdünnt und mit MTB-Ether extrahiert. Die vereinigten org. Phasen werden mit Ammoniumeisen(II)sulfatlösung und mit Wasser gewaschen, über Natriumsulfat getrocknet und eingeengt. Nach Chromatographie des Rohproduktes mit Heptan/MTB-Ether (1:4) an Kieselgel erhält man 9,17-Dihydroxy-13,14-dihydro-12H-naphtho[2,1,10,13-fgh][1,5] dioxonin als farblosen Feststoff.

$^1$H-NMR (400 MHz, $CDCl_3$):

δ = 1,95 ($m_c$, 2 H, $-OCH_2CH_2CH_2O-$), 4,31 ($m_c$, 4 H, $-OCH_2CH_2CH_2O-$), 5,40 (s, 2 H, OH), 6,84 (s, 2 H, Ar-H), 7,26 ($m_c$, 4 H, Ar-H), 7,36 (ddd, J = 4,0 Hz, J = 4,0 Hz, J = 8,1 Hz, 2 H, Ar-H), 8,16 (d, J = 8,4 Hz, 2 H, Ar-H).

Stufe 4: 13,14-Dihydro-12H-naphtho[2,1,10,13-fgh][1,5]dioxonin-9,17-diyldimethacrylat

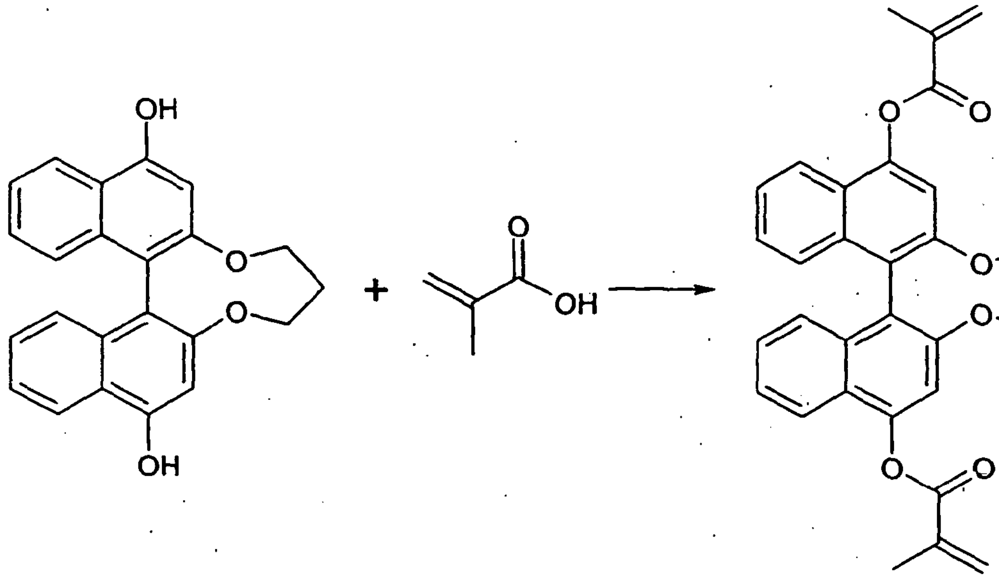

(16)

1,20 g (3,35 mmol) 9,17-Dihydroxy-13,14-dihydro-12H-naphtho[2,1,10,13-fgh][1,5]dioxonin werden in 30 ml Toluol gelöst und Dicyclohexylcarbodiimid in 20 ml Toluol sowie 150 mg N,N-Dimethylaminopyridin hinzugegeben. Anschließend werden 0,85 ml (10 mmol) Acrylsäure hizugefügt und der Ansatz über Nacht rühren gelassen. Nach Zugabe von 0,5 g Oxalsäuredihydrat wird weitere 2 h rühren gelassen, der ausgefallene Feststoff abfiltriert, das Filtrat eingeengt, der Rückstand mit Heptan/Essigester (6:1) an Kieselgel chromatographiert und das Rohprodukt aus Ether/ Essigester umkristallisiert. Man erhält 13,14-Dihydro-12H-naphtho[2,1,10,13-fgh][1,5]dioxonin-9,17-diyldimethacrylat als farblosen Feststoff.

Phasenverhalten: Tg 157 K 240 I

Spezifische Drehung (c = 0,5, $CH_2Cl_2$): $[a]_{20}^{D} = -210,5$

Mischungsbeispiel A

Die nematische FK-Host-Mischung N1 wird wie folgt formuliert

| | | | |
|---|---|---|---|
| CCH-501 | 9,00 % | Kp. | + 70,0 |
| CCH-35 | 14,00 % | $\Delta n$ | 0,0825 |
| PCH-53 | 8,00 % | $\Delta\varepsilon$ | - 3,5 |
| CY-3-O4 | 14,00 % | $\varepsilon_{\parallel}$ | 3,5 |
| CY-5-O4 | 13,00 % | $K_3/K_1$ | 1,00 |
| CCY-3-O2 | 8,00 % | $\gamma_1$ | 141 |
| CCY-5-O2 | 8,00 % | $V_0$ | 2,06 |
| CCY-2-1 | 9,00 % | | |
| CCY-3-1 | 9,00 % | | |
| CPY-2-O2 | 8,00 % | | |

Zur FK-Mischung N1 werden 0.3% einer polymerisierbaren monomeren Verbindung aus den Beispielen 1 bis 4 zugesetzt, und die dadurch entstandenenen Mischungen in VA-e/o-Testzellen gefüllt (90° gerieben, Orientierungsschicht VA-Polyimid, Schichtdicke d≈4μm). Unter Anlegen einer Spannung von 10V (Wechselstrom) wird jede Zelle 20 Minuten lang mit UV-Licht der Intensität 28mW/cm$^2$ bestrahlt, dadurch erfolgt Polymerisation der monomeren Verbindung. In einer zweiten Versuchsreihe wird der FK/Monomer-Mischung noch zusätzlich 0.006% des Photoinitiators Irg-

acure-651 zugesetzt und die Belichtungszeit auf 2 Minuten verkürzt. Vor und nach der UV-Bestrahlung wird per Drehkristall-Experiment (Autronic-Melchers TBA-105) der Tiltwinkel bestimmt. Die Ergebnisse sind in Tabelle 1 zusammengefasst.

Tabelle 1

| Monomer | Initiator | Tilt vor UV | Tilt nach UV |
|---|---|---|---|
| *R*-(1) | nein | 89.9° | 87.6° |
| *R*- (1) | ja | 89.9° | 88.6° |
| *rac*-(1) | nein | 89.9° | 87.8° |
| *rac*- (1) | ja | 89.9° | 89.4° |
| *R**-(2) | nein | 89.8° | 89.0° |
| *R**-(2) | ja | 89.7° | 89.5° |
| *rac*-(2) | nein | 89.8° | 86.9° |
| *rac*-(2) | ja | 89.6° | 89.7° |
| *rac*-(15) | nein | 89.8° | 87.8 |
| *rac*-(15) | ja | 89.7° | 87.4 |
| R*-(16) | nein | 89.7° | 87.1 |
| R*-(16) | ja | 89.8° | 87.4 |

Wie aus Tabelle 1 ersichtlich ist, kann mit den erfindungsgemäßen Monomeren eine deutliche Vergrößerung des Tilts (d.h. Verringerung des Tiltwinkels) nach Polymerisation erreicht werden, insbesondere ohne Verwendung eines Photoinitiators.

## Patentansprüche

**1.** Flüssigkristall (FK-) Anzeige des PSA- (polymer sustained alignment) Typs, enthaltend eine FK-Zelle bestehend aus zwei Substraten, wobei mindestens ein Substrat lichtdurchlässig ist und mindestens ein Substrat eine Elektrodenschicht aufweist, sowie einer zwischen den Substraten befindlichen Schicht eines niedermolekularen FK-Mediums enthaltend eine oder mehrere polymerisierte Verbindungen, wobei die polymerisierte(n) Verbindung(en) erhältlich sind durch Polymerisation einer oder mehrerer polymerisierbarer Verbindungen zwischen den Substraten der FK-Zelle im FK-Medium unter Anlegen einer elektrischen Spannung, **dadurch gekennzeichnet, dass** mindestens eine der polymerisierbaren Verbindungen ein Racemat von Verbindungen enthaltend ein Biarylstrukturelement der Formel ist

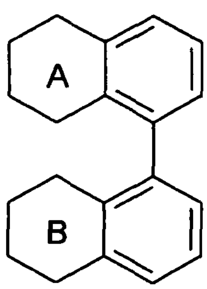

I

welches an mindestens einer Position, optional über eine organische Gruppe oder eine Abstandsgruppe, mit einer oder mehreren polymerisierbaren Gruppen verknüpft ist, und worin A und B jeweils unabhängig voneinander einen aromatischen oder ganz oder teilweise gesättigten Ring bedeuten, wobei in den einzelnen Ringen auch ein oder mehrere CH-Gruppen durch N und/oder eine oder mehrere $CH_2$-Gruppen durch O und/oder S so ersetzt sein können, dass 0- und/oder S-Atome nicht direkt miteinander verknüpft sind, und wobei die einzelnen Ringe auch

ein- oder mehrfach substituiert sein können.

2. FK-Anzeige nach Anspruch 1, **dadurch gekennzeichnet, dass** das Strukturelement der Formel I an einer oder mehreren Positionen über eine Abstandsgruppe mit einer oder mehreren polymerisierbaren Gruppen verknüpft ist.

3. FK-Anzeige nach Anspruch 1 oder 2, **dadurch gekennzeichnet, dass** das Strukturelement der Formel I an einer, zwei oder mehr als zwei Positionen direkt mit einer polymerisierbaren Gruppe verknüpft ist.

4. FK-Anzeige nach einem oder mehreren der Ansprüche 1 bis 3, **dadurch gekennzeichnet, dass** das Strukturelement der Formel I ausgewählt ist aus folgenden Formeln

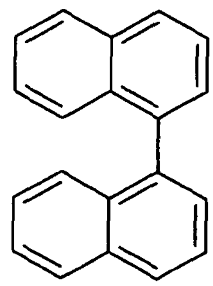

Ia

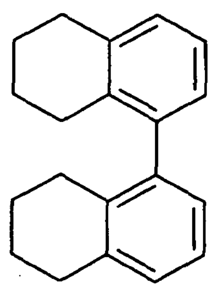

Ib

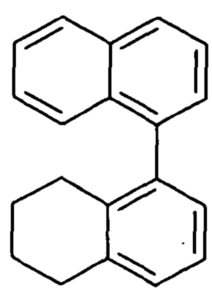

Ic

5. FK-Anzeige nach einem oder mehreren der Ansprüche 1 bis 4, **dadurch gekennzeichnet, dass** die polymerisierbaren Verbindungen aus Racematen folgender Formel ausgewählt sind

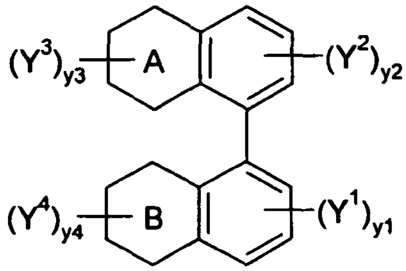

I1

worin

A und B jeweils unabhängig voneinander anelliertes Benzol, Cyclohexan oder Cyclohexen,
$Y^{1-4}$ jeweils unabhängig voneinander H, Halogen, $SF_5$, $NO_2$, eine Kohlenstoffgruppe oder Kohlenwasserstoffgruppe, wobei auch einer oder mehrere der Reste $Y^{1-4}$ mit einem benachbarten Rest $Y^{1-4}$ und/oder mit dem Biarylgrundgerüst ein aliphatisches oder aromatisches, mono- oder polycyclisches und gegebenenfalls kondensiertes Ringsystem bilden können, und worin mindestens einer der Reste $Y^{1-4}$ eine polymerisierbare Gruppe P enthält,
y1, y2 1, 2 oder 3, und
y3, y4 1, 2, 3 oder 4

bedeuten, wobei in jedem der Ringe in Formel I1 auch eine oder mehrere CH-Gruppen durch N und/oder eine oder mehrere $CH_2$-Gruppen durch O und/oder S so ersetzt sein können, dass O- und/oder S-Atome nicht direkt miteinander verknüpft sind.

**6.** FK-Anzeige nach einem oder mehreren der Ansprüche 1 bis 5, **dadurch gekennzeichnet, dass** die polymerisierbaren Verbindungen aus Racematen folgender Formel ausgewählt sind

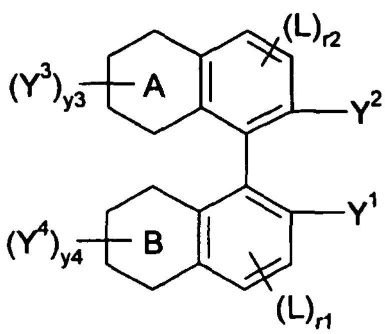

I1a

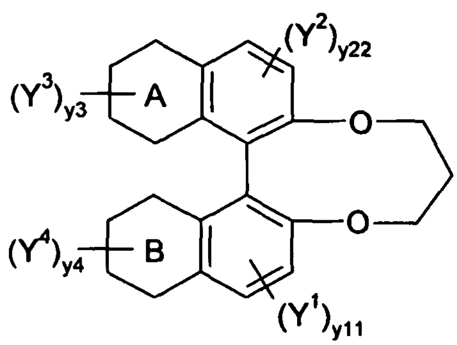

I1b

worin A, B, $Y^{1-4}$, y3 und y4 die in Anspruch 5 angegebene Bedeutung besitzen,
r1 und r2 jeweils unabhängig voneinander 0, 1 oder 2,

y11 und y22 jeweils unabhängig voneinander 0 oder 1,
L F, Cl, Br, I, -CN, $-NO_2$, -NCO, -NCS, -OCN, -SCN, $-C(=O)N(R^x)_2$,$-C(=O)Y^1$, $-C(=O)R^x$, $-N(R^x)_2$, optional substituiertes Silyl, Aryl mit 4 bis 40, vorzugsweise 6 bis 20 C Atomen, und geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin ein oder mehrere H-Atome gegebenfalls durch F oder Cl ersetzt sein können,
$R^x$ H, Halogen, eine geradkettige, verzweigte oder cyclische Alkylkette mit 1 bis 25 C-Atomen, in der auch ein oder mehrere nicht benachbarte C-Atome durch -O-, -S-, -CO-, -CO-O-, -O-CO-, -O-CO-0- ersetzt sein können, wobei auch ein oder mehrere H-Atome durch Fluor ersetzt sein können, oder eine optional substituierte Aryl-, Aryloxy-, Heteroaryl- oder Heteroaryloxygruppe mit 5 bis 40 C-Atomen, und
$Y^1$ Halogen bedeuten.

7. FK-Anzeige nach einem oder mehreren der Ansprüche 1 bis 6, **dadurch gekennzeichnet, dass** die polymerisierbaren Verbindungen aus Racematen folgender Formel ausgewählt sind

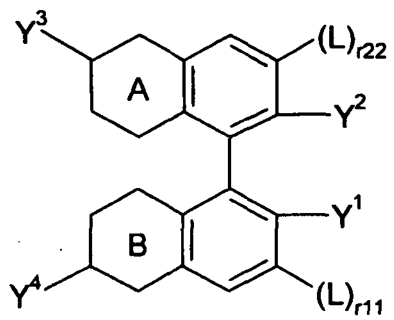

I1a1

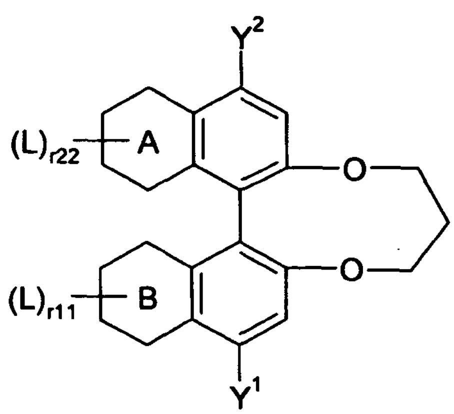

I1b1

worin $Y^{1-4}$, L, A und B die in Anspruch 5 und 6 angegebene Bedeutung besitzen und r11 und r22 0 oder 1 bedeuten.

8. FK-Anzeige nach einem oder mehreren der Ansprüche 5 bis 7, **dadurch gekennzeichnet, dass** in den Verbindungen der Formel I1, I1a, I1a1, I1b oder I1b1 einer oder mehrere der Reste $Y^{1-4}$ $-W-(Z^1-A^1)_{m1}-Sp-P$ bedeuten, worin

W O, S, $CH_2$ oder eine Einfachbindung,
Sp eine Abstandsgruppe oder eine Einfachbindung,
P eine polymerisierbare Gruppe,
$A^1$ bei jedem Auftreten gleich oder verschieden Aryl, Heteroaryl oder ganz oder teilweise gesättigtes Cycloalkyl mit 4 bis 20 C-Atomen, welches optional substituiert ist und worin auch eine oder mehrere CH-Gruppen durch N und/oder eine oder mehrere $CH_2$-Gruppen durch O und/oder S so ersetzt sein können, dass O- und/oder S-Atome nicht direkt miteinander verknüpft sind,
$Z^1$ bei jedem Auftreten gleich oder verschieden -O-, -S-, -CO- , -COO-, -OCO-, -O-COO-, $-OCH_2-$, $-CH_2O-$, $-SCH_2-$, - $CH_2S-$, $-CF_2O-$, $-OCF_2-$, $-CF_2S-$, $-SCF_2-$, $-CH_2CH_2-$, - $CF_2CH_2-$, $-CH_2CF_2-$, $-CF_2CF_2-$, -CH=CH-, -CF=CF-, -C≡C-, -CH=CH-COO-, -OCO-CH=CH-, $CR^0R^{00}$ oder eine Einfachbindung,

$R^0$ und $R^{00}$ jeweils unabhängig voneinander H oder Alkyl mit 1 bis 4 C- Atomen, und
m1 0, 1, 2, 3 oder 4

bedeuten.

**9.** FK-Anzeige nach einem oder mehreren der Ansprüche 5 bis 8, **dadurch gekennzeichnet, dass** in den Verbindungen der Formel I1, I1a, I1a1, I1b oder I1b1 einer oder mehrere der Reste $Y^{1-4}$ -W-$(Z^1$-$A^1)_{m1}$-$R^y$ bedeuten, worin W, $Z^1$, $A^1$ und m1 die in Anspruch 8 angegebene Bedeutung besitzen und

$R^y$ bei jedem Auftreten gleich oder verschieden H, L wie in Anspruch 4 definiert, geradkettiges oder verzweigtes Alkyl mit 1 bis 25 C-Atomen, welches unsubstituiert oder durch F, Cl, Br, I, CN oder P-Sp- ein- oder mehrfach substituiert ist, und worin ein mehrere nicht benachbarte $CH_2$-Gruppen jeweils unabhängig voneinander durch -C($R^x$)=C($R^x$)-, -C≡C-, -N($R^x$)-, -O-, -S-, -CO- , -CO-O-, -O-CO-, -O-CO-O- so ersetzt sein können, dass O- und/oder S-Atome nicht direkt miteinander verknüpft sind, wobei $R^x$ die in Anspruch 5 angegebene Bedeutung hat,

bedeutet, und, falls alle Reste $Y^{1-4}$ -W-$(Z^1$-$A^1)_{m1}$-$R^y$ bedeuten, einer oder mehrere der Reste $R^y$ mindestens eine Gruppe P-Sp-aufweisen.

**10.** FK-Anzeige nach einem oder mehreren der Ansprüche 1 bis 9, **dadurch gekennzeichnet, dass** die polymerisierbaren Verbindungen aus Racematen der folgenden Formeln ausgewählt sind

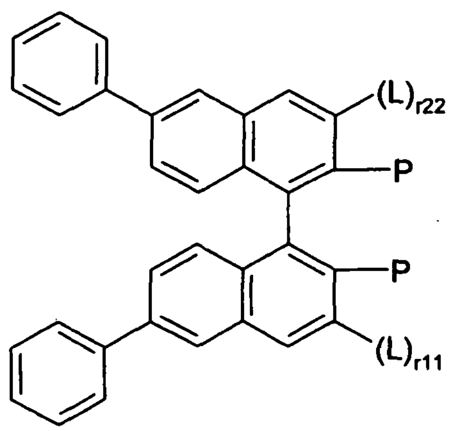

I1a1a

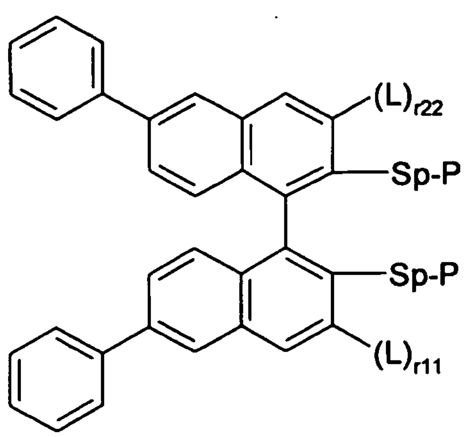

I1a1b

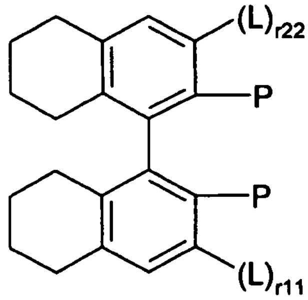

I1a1c

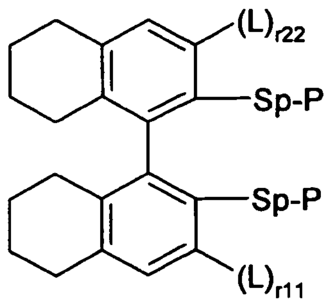

I1a1d

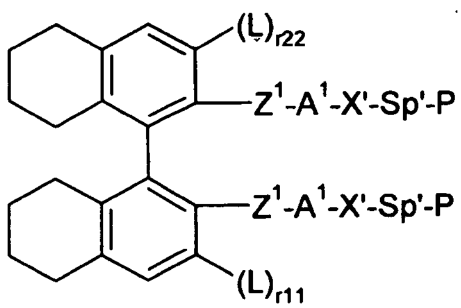

I1a1e

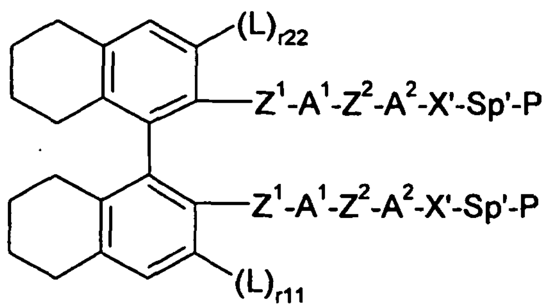

I1a1f

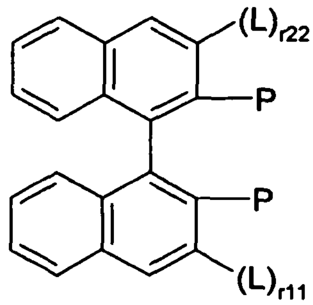

I1a1g

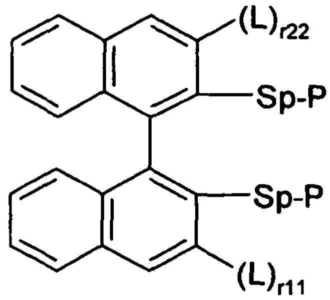

I1a1h

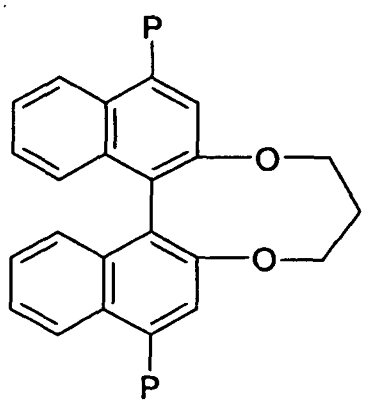

I1b1a

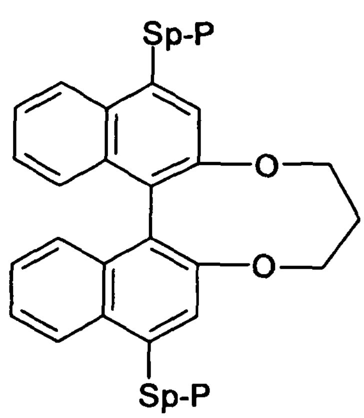

I1b1b

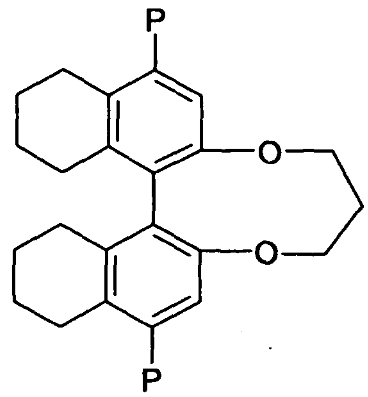

I1b1c

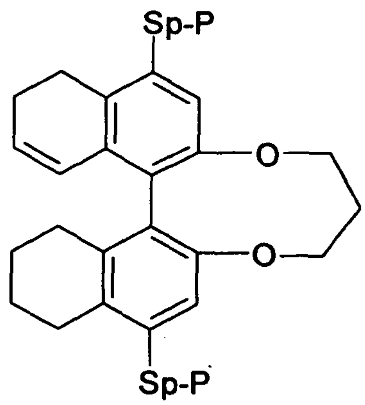

I1b1d

worin $Y^3$, $Y^4$, P, Sp, $Z^1$, $A^1$, L, r11 und r22 bei jedem Auftreten gleich oder verschieden die in Anspruch 7 und 8 angegebene Bedeutung besitzen, $Z^2$ und $A^2$ eine der für $Z^1$ bzw. $A^1$ angegebenen Bedeutungen besitzen,

Sp' Alkylen mit 1 bis 20 C-Atomen bedeutet, welches optional durch F, Cl, Br, I oder CN ein- oder mehrfach substituiert ist, und worin auch eine oder mehrere nicht benachbarte $CH_2$-Gruppen jeweils unabhängig voneinander so durch - O-, -S-, -NH-, $-NR^0-$, $-SiR^0R^{00}-$, -CO-, -COO-, -OCO-, - OCO-O-, -S-CO-, -CO-S-, $-NR^0-CO-O-$, $-O-CO-NR^0-$, $- NR^0-CO-NR^0-$, -CH=CH- oder -C≡C- ersetzt sein können, daß 0- und/oder S-Atome nicht direkt miteinander verknüpft sind,
X' -O-, -S-, -CO-, -COO-, -OCO-, -O-COO-, $-CO-NR^0-$, $-NR^0- CO-$, $-NR^0-CO-NR^0-$, $-OCH_2-$, $-CH_2O-$, $-SCH_2-$, $-CH_2S-$, $-CF_2O-$, $-OCF_2-$, $-CF_2S-$, $-SCF_2-$, $-CF_2CH_2-$, $-CH_2CF_2-$, $-CF_2CF_2-$, -CH=N-, -N=CH-, -N=N-, $-CH=CR^0-$, $-CY^2=CY^3-$, -C≡C-, -CH=CH-COO-, -OCO-CH=CH- oder eine Einfachbindung,
$R^0$ und $R^{00}$ jeweils unabhängig voneinander H oder Alkyl mit 1 bis 12 C-Atomen bedeuten, und
$Y^2$ und $Y^3$ jeweils unabhängig voneinander H, F, Cl oder CN

bedeuten.

**11.** FK-Anzeige nach einem oder mehreren der Ansprüche 1 bis 10, **dadurch gekennzeichnet, dass** das FK-Medium eine oder mehrere Verbindungen der Formel II enthält:

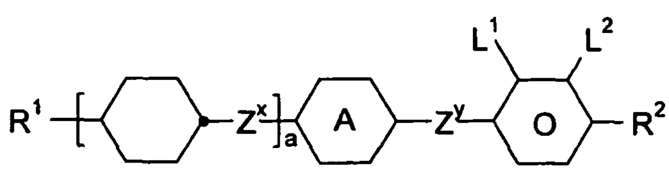

II

worin

$R^1$ und $R^2$ jeweils unabhängig voneinander Alkyl mit 1 bis 12 C- Atomen, wobei auch ein oder zwei nicht benachbarte $CH_2$- Gruppen durch -O-, -CH=CH-, -CO-, -OCO- oder -COO- so ersetzt sein können, daß O-Atome nicht direkt miteinander verknüpft sind,
$Z^x$ und $Z^y$ jeweils unabhängig voneinander $-CH_2CH_2-$, -CH=CH-, $-CF_2O-$, $-OCF_2-$, $-CH_2O-$, $-OCH_2-$, -COO-, -OCO-, $-C_2F_4-$, -CF=CF-, $-CH=CHCH_2O-$, oder eine Einfachbindung,
$L^1$ und $L^2$ jeweils unabhängig voneinander F, Cl, $OCF_3$, $CF_3$, $CH_3$, $CH_2F$ oder $CHF_2$,
a 0 oder 1 und

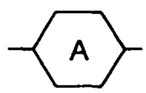 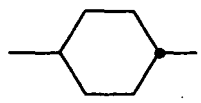 oder 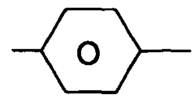

bedeuten.

**12.** FK-Anzeige nach einem oder mehreren der Ansprüche 1 bis 11, **dadurch gekennzeichnet, dass** das FK-Medium zusätzlich eine oder mehrere Verbindungen der Formel III enthält:

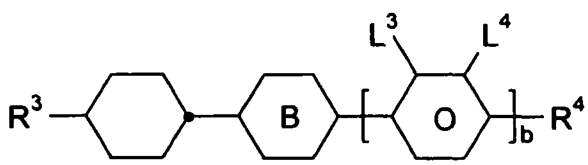 III

worin die einzelnen Reste folgende Bedeutung besitzen

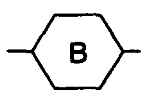 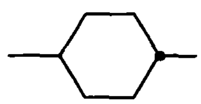, 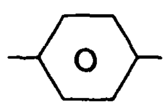 oder 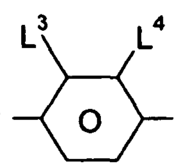

b 0 oder 1,
$L^3$ und $L^4$ jeweils unabhängig voneinander H, F oder Cl,
$R^3$ Alkenyl mit 2 bis 9 C-Atomen,
$R^4$ Alkyl mit 1 bis 12 C-Atomen, worin auch ein oder zwei nicht benachbarte $CH_2$-Gruppen durch -O-, -CH=CH-, - CO-, -OCO- oder -COO- so ersetzt sein können, daß O- Atome nicht direkt miteinander verknüpft sind, oder, falls b=0 ist und der Ring B Cyclohexylen bedeutet, auch $R^1$.

**13.** FK-Anzeige nach einem oder mehreren der Ansprüche 1 bis 12, **dadurch gekennzeichnet, dass** das FK-Medium zusätzlich eine oder mehrere Verbindungen der Formel VII enthält:

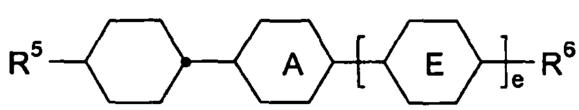 VII

worin

$R^5$ und $R^6$ unabhängig voneinander eine der für $R^1$ in Formel II angegebenen Bedeutungen haben,

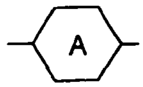 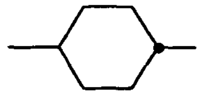 oder 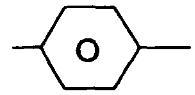

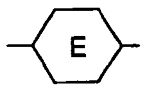 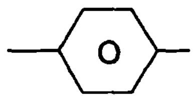 oder 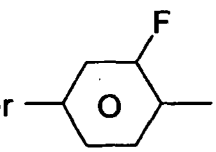,

und

e 0 oder 1 bedeuten.

**14.** FK-Medium wie in einem oder mehreren der Ansprüche 1 bis 13 definiert.

**15.** Racemat einer polymerisierbaren Verbindung enthaltend ein Biarylstrukturelement der Formel I wie in einem oder mehreren der Ansprüche 1 bis 10 definiert.

**16.** Racemat einer polymerisierbaren Verbindung gemäß Anspruch 15, **dadurch gekennzeichnet, dass** sie ein Biarylstrukturelement der Formel Ib aufweist

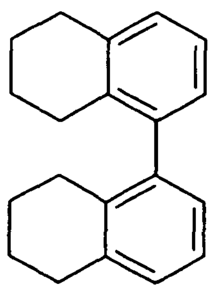

Ib

welches an mindestens einer Position, optional über eine organische Gruppe oder eine Abstandsgruppe, mit einer oder mehreren polymerisierbaren Gruppen verknüpft ist, und worin A und B jeweils unabhängig voneinander einen aromatischen oder ganz oder teilweise gesättigten Ring bedeuten, wobei in den einzelnen Ringen auch ein oder mehrere CH-Gruppen durch N und/oder eine oder mehrere $CH_2$-Gruppen durch O und/oder S so ersetzt sein können, dass 0- und/oder S-Atome nicht direkt miteinander verknüpft sind, und wobei die einzelnen Ringe auch ein- oder mehrfach substituiert sein können.

**17.** Optisch aktive polymerisierbaren Verbindung oder deren Racemat **dadurch gekennzeichnet, dass** sie ausgewählt ist aus Formel I1a1b

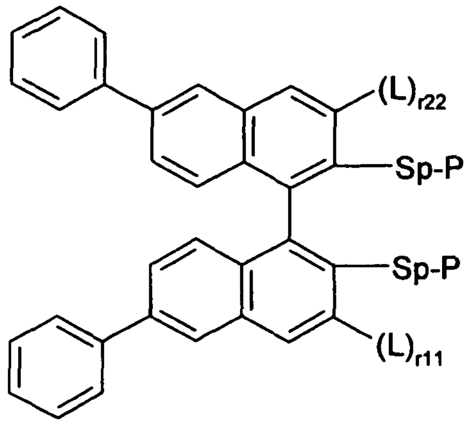

I1a1b

worin P, Sp, L, r11 und r22 die in Anspruch 10 angegebene Bedeutung besitzen.

**18.** Optisch aktive polymerisierbare Verbindung oder deren Racemat, **dadurch gekennzeichnet, dass** sie ausgewählt ist aus Formel I1b1

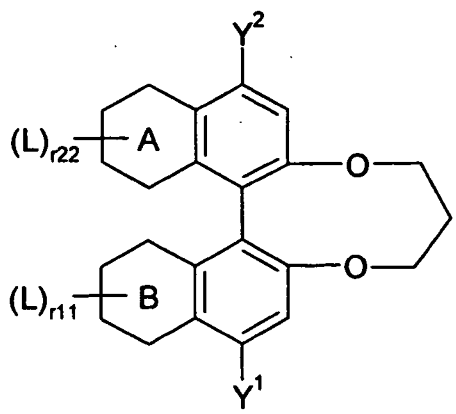

I1b1

worin A, B, L, r11 und r22 die in Anspruch 10 angegebene Bedeutung besitzen und $Y^1$ und $Y^2$ eine polymerisierbare Gruppe enthalten oder bedeuten.

**19.** FK-Anzeige gemäß einem oder mehreren der Ansprüche 1 bis 13, **dadurch gekennzeichnet, dass** sie eine PSA-VA-oder PSA-OCB-Anzeige ist.

**20.** FK-Medium enhaltend ein oder mehrere polymerisierbare Racemate oder polymeriserbare oder polymerisierte optisch aktive Verbindungen gemäss einem oder mehreren der Ansprüche 15 bis 18.

**21.** Verfahren zur Herstellung eines FK-Mediums gemäß Anspruch 20, **dadurch gekennzeichnet, dass** man eine oder mehrere niedermolekulare flüssigkristalline Verbindungen mit einer oder mehreren polymerisierbaren Verbindungen gemäß einem oder mehreren der Ansprüche 15 bis 18 und ggf. mit weiteren flüssigkristallinen Verbindungen und/oder Additiven mischt.

**Claims**

**1.** Liquid-crystal (LC) display of the PSA (polymer sustained alignment) type, comprising an LC cell consisting of two substrates, where at least one substrate is transparent to light and at least one substrate has an electrode layer, and a layer of a low-molecular-weight LC medium comprising one or more polymerised compounds which is located between the substrates, where the polymerised compound(s) is (are) obtainable by polymerisation of one or more

polymerisable compounds in the LC medium between the substrates of the LC cell with application of an electrical voltage, **characterised in that** at least one of the polymerisable compounds is a racemate of compounds containing a biaryl structural element of the formula I

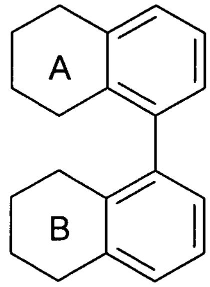

I

which is linked at at least one position to one or more polymerisable groups, optionally via an organic group or a spacer group, and in which A and B each, independently of one another, denote an aromatic or fully or partially saturated ring, where, in addition, in the individual rings, one or more CH groups may be replaced by N and/or one or more $CH_2$ groups may be replaced by O and/or S in such a way that O and/or S atoms are not linked directly to one another, and where the individual rings may also be mono- or polysubstituted.

2. LC display according to Claim 1, **characterised in that** the structural element of the formula I is linked at one or more positions to one or more polymerisable groups via a spacer group.

3. LC display according to Claim 1 or 2, **characterised in that** the structural element of the formula I is linked directly at one, two or more than two positions to a polymerisable group.

4. LC display according to one or more of Claims 1 to 3, **characterised in that** the structural element of the formula I is selected from the following formulae:

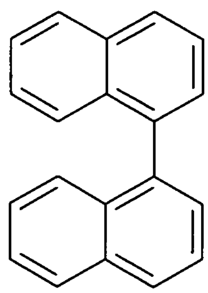

Ia

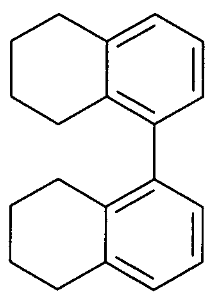

Ib

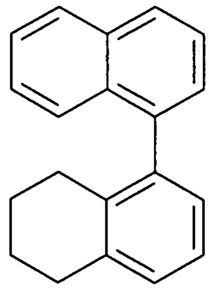

Ic

**5.** LC display according to one or more of Claims 1 to 4, **characterised in that** the polymerisable compounds are selected from racemates of the following formula:

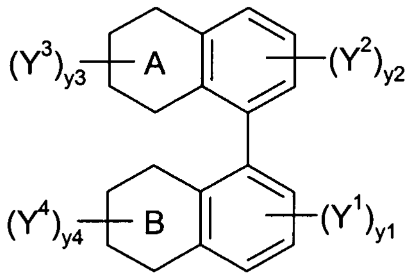

I1

in which

A and B each, independently of one another, denote fused ben- zene, cyclohexane or cyclohexene,
$Y^{1-4}$ each, independently of one another, denote H, halogen, $SF_5$, $NO_2$, a carbon group or hydrocarbon group, in which, in addition, one or more of the radicals $Y^{1-4}$ are able to form an aliphatic or aromatic, mono- or polycyclic and optionally condensed ring system with an adjacent radical $Y^{1-4}$ and/or with the biaryl skeleton and in which at least one of the radicals $Y^{1-4}$ contains a polymerisable group P,
y1, y2 denote 1, 2 or 3, and
y3, y4 denote 1, 2, 3 or 4,

where, in addition, in each of the rings in formula I1, one or more CH groups may be replaced by N and/or one or more $CH_2$ groups may be replaced by O and/or S in such a way that O and/or S atoms are not linked directly to one another.

**6.** LC display according to one or more of Claims 1 to 5, **characterised in that** the polymerisable compounds are selected from racemates of the following formulae:

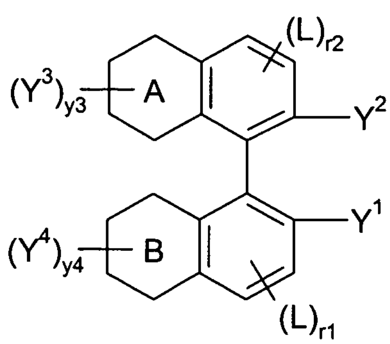

I1a

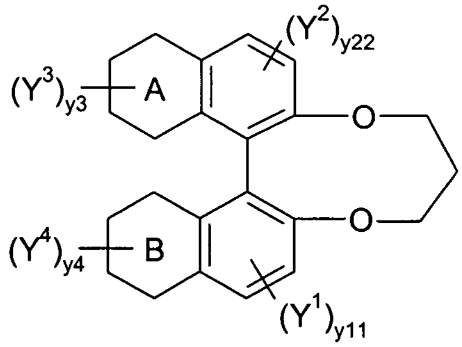

I1b

in which A, B, $Y^{1-4}$, y3 and y4 have the meaning indicated in Claim 5, r1 and r2 each, independently of one another, denote 0, 1 or 2,
y11 and y22 each, independently of one another, denote 0 or 1,
L denotes F, Cl, Br, I, -CN, $-NO_2$, -NCO, -NCS, -OCN, -SCN, $-C(=O)N(R^x)_2$, $-C(=O)Y^1$, $-C(=O)R^x$, $-N(R^x)_2$, optionally substituted silyl, aryl having 4 to 40, preferably 6 to 20, C atoms, and straight-chain or branched alkyl, alkoxy, alkylcarbonyl, alkoxycarbonyl, alkylcarbonyloxy or alkoxycarbonyloxy having 1 to 25 C atoms, in which one or more H atoms may optionally be replaced by F or Cl,
$R^x$ denotes H, halogen, a straight-chain, branched or cyclic alkyl chain having 1 to 25 C atoms, in which, in addition, one or more non-adjacent C atoms may be replaced by -O-, -S-, -CO-, -CO-O-, -O-CO-, -O-CO-O- and in which one or more H atoms may be replaced by fluorine, or an optionally substituted aryl, aryloxy, heteroaryl or heteroaryloxy group having 5 to 40 C atoms, and
$Y^1$ denotes halogen.

**7.** LC display according to one or more of Claims 1 to 6, **characterised in that** the polymerisable compounds are selected from racemates of the following formulae:

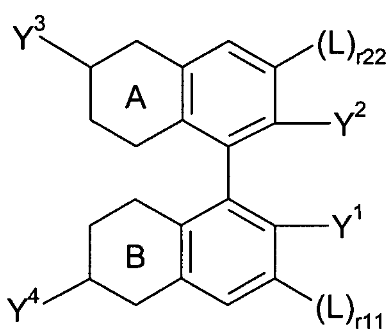

I1a1

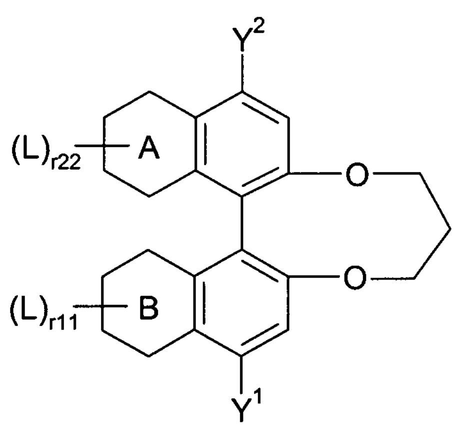

I1b1

in which $Y^{1-4}$, L, A and B have the meaning indicated in Claims 5 and 6, and r11 and r22 denote 0 or 1.

**8.** LC display according to one or more of Claims 5 to 7, **characterised in that** one or more of the radicals $Y^{1-4}$ in the compounds of the formulae I1, I1a, I1a1, I1b and I1b1 denote $-W-(Z^1-A^1)_{m1}-Sp-P$, in which

W denotes O, S, $CH_2$ or a single bond,
Sp denotes a spacer group or a single bond,
P denotes a polymerisable group,
$A^1$ on each occurrence, identically or differently, denotes aryl, heteroaryl or fully or partially saturated cycloalkyl having 4 to 20 C atoms, which is optionally substituted and in which, in addition, one or more CH groups may be re- placed by N and/or one or more $CH_2$ groups may be replaced by O and/or S in such a way that O and/or S atoms are not linked directly to one another,
$Z^1$ on each occurrence, identically or differently, denotes -O-, -S-, -CO-, -COO-, -OCO-, -O-COO-, $-OCH_2-$, $-CH_2O-$, $-SCH_2-$, $-CH_2S-$, $-CF_2O-$, $-OCF_2-$, $-CF_2S-$, $-SCF_2-$, $-CH_2CH_2-$, $-CF_2CH_2-$, $-CH_2CF_2-$, $-CF_2CF_2-$, -CH=CH-, -CF=CF-, -C≡C-, -CH=CH-COO-, -OCO-CH=CH-, $CR^0R^{00}$ or a single bond,
$R^0$ and $R^{00}$ each, independently of one another, denote H or alkyl having 1 to 4 C atoms, and
M1 denotes 0, 1, 2, 3 or 4.

**9.** LC display according to one or more of Claims 5 to 8, **characterised in that** one or more of the radicals $Y^{1-4}$ in the compounds of the formulae I1, I1a, I1a1, I1b and I1b1 denote $-W-(Z^1-A^1)_{m1}-R^4$, in which W, $Z^1$, $A^1$ and m1 have the meaning indicated in Claim 8, and

$R^4$ on each occurrence, identically or differently, denotes H, L as defined in Claim 4, straight-chain or branched alkyl having 1 to 25 C atoms, which is unsubstituted or mono- or polysubstituted by F, Cl, Br, I, CN or P-Sp- and in which one or more non-adja- cent $CH_2$ groups may each, independently of one another, be replaced by $-C(R^x)=C(R^x)-$, -C≡C-, $-N(R^x)-$, -O-, -S-, -CO-, -CO-O-, -O-CO-, -O-CO-O- in such a way that O and/or S atoms are not linked directly to one another, where $R^x$ has the meaning indicated in Claim 5,

and, if all radicals $Y^{1-4}$ denote $-W-(Z^1-A^1)_{m1}-R^4$, one or more of the radicals $R^4$ contain at least one group P-Sp-.

**10.** LC display according to one or more of Claims 1 to 9, **characterised in that** the polymerisable compounds are selected from racemates of the following formulae:

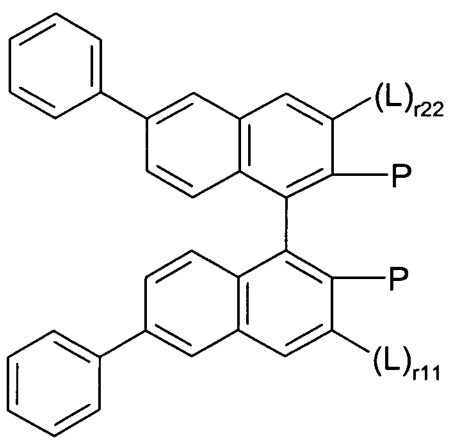

I1a1a

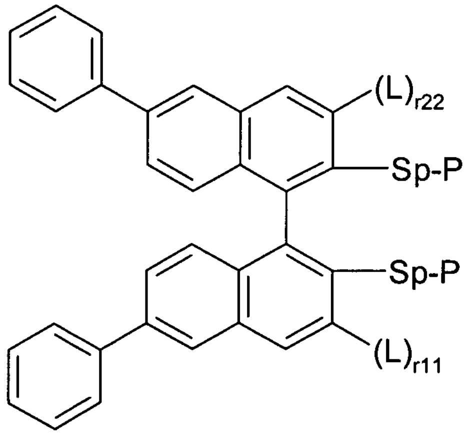

I1a1b

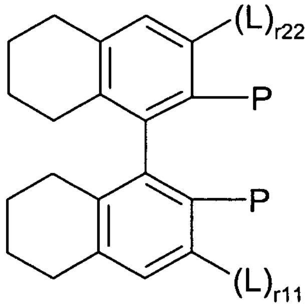

I1a1c

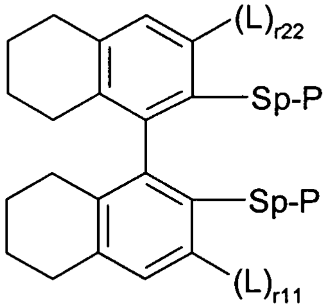

I1a1d

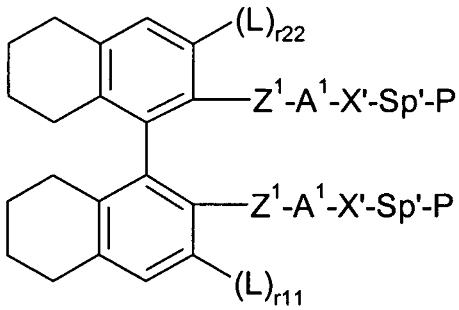

I1a1e

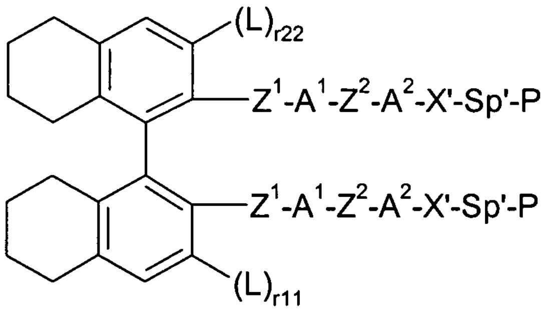

I1a1f

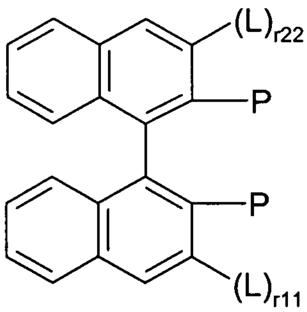

I1a1g

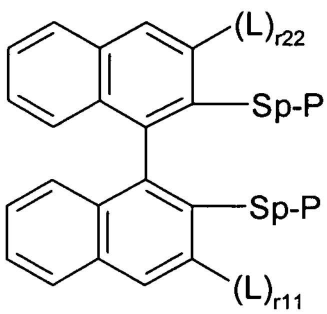

I1a1h

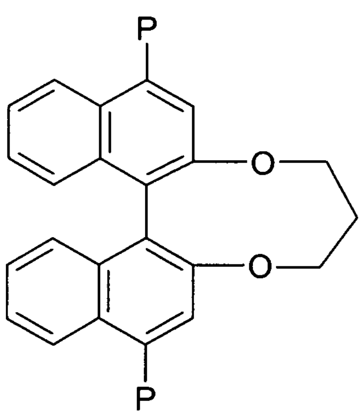

I1b1a

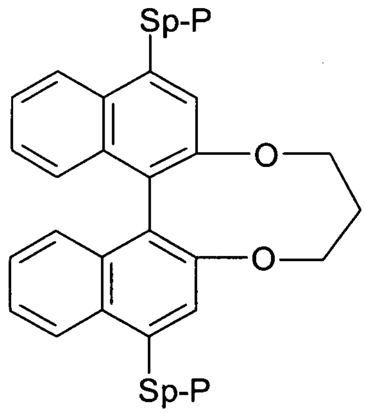

I1b1b

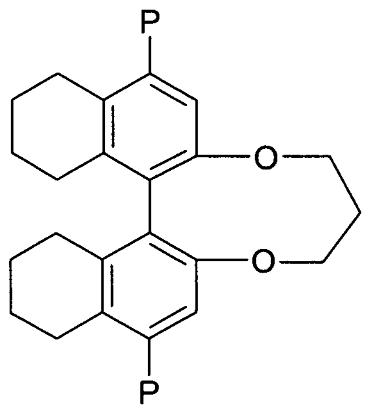

I1b1c

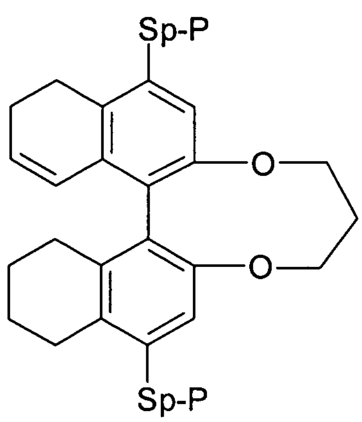

I1b1d

in which $Y^3$, $Y^4$, P, Sp, $Z^1$, $A^1$, L, r11 and r22 on each occurrence, identically or differently, have the meaning indicated in Claims 7 and 8, $Z^2$ and $A^2$ have one of the meanings indicated for $Z^1$ and $A^1$ respectively,

Sp' denotes alkylene having 1 to 20 C atoms, which is optionally mono- or polysubstituted by F, Cl, Br, I or CN and in which, in addition, one or more non-adjacent $CH_2$ groups may each, independently of one another, be replaced by -O-, -S-, -NH-, $-NR^0-$, $-SiR^0R^{00}-$, -CO-, -COO-, -OCO-, -OCO-O-, -S-CO-, -CO-S-, $-NR^0-CO-O-$, $-O-CO-NR^0-$, $-NR^0-CO-NR^0-$, -CH=CH- or -C≡C- in such a way that O and/or S atoms are not linked directly to one another,

X' denotes -O-, -S-, -CO-, -COO-, -OCO-, -O-COO-, $-CO-NR^0-$, $-NR^0-CO-$, $-NR^0-CO-NR^0$, $-OCH_2-$, $-CH_2O-$,

-$SCH_2$-, -$CH_2S$-, -$CF_2O$-, -$OCF_2$-, -$CF_2S$-, -$SCF_2$-, -$CF_2CH_2$-, -$CH_2CF_2$-, -$CF_2CF_2$-, -CH=N-, -N=CH-, -N=N-, -CH=$CR^0$-, -$CY^2$=$CY^3$, -C≡C-, -CH=CH-COO-, -OCO-CH=CH- or a single bond,
$R^0$ and $R^{00}$ each, independently of one another, denote H or alkyl having 1 to 12 C atoms, and
$Y^2$ and $Y^3$ each, independently of one another, denote H, F, Cl or CN.

**11.** LC display according to one or more of Claims 1 to 10, **characterised in that** the LC medium comprises one or more compounds of the formula II

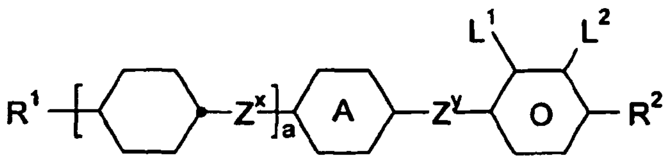

II

in which

$R^1$ and $R^2$ each, independently of one another, denote alkyl having 1 to 12 C atoms, in which, in addition, one or two non- adjacent $CH_2$ groups may be replaced by -O-, -CH=CH-, -CO-, -OCO- or -COO- in such a way that O atoms are not linked directly to one another,
$Z^x$ and $Z^y$ each, independently of one another, denote -$CH_2CH_2$-, -CH=CH-, -$CF_2O$-, -$OCF_2$-, -$CH_2O$-, -$OCH_2$-, -COO-, -OCO-, -$C_2F_4$-, -CF=CF-, -CH=$CHCH_2O$- or a single bond,
$L^1$ and $L^2$ each, independently of one another, denote F, Cl, $OCF_3$, $CF_3$, $CH_3$, $CH_2F$ or $CHF_2$,
a denotes 0 or 1, and

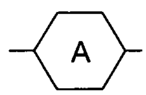

denotes

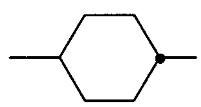

or

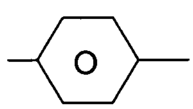

**12.** LC display according to one or more of Claims 1 to 11, **characterised in that** the LC medium additionally comprises one or more compounds of the formula III

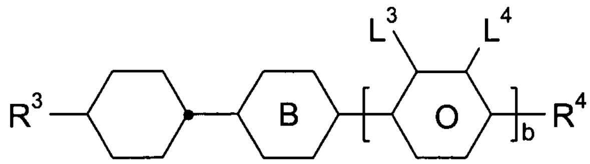

III

in which the individual radicals have the following meanings:

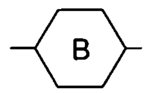

denotes

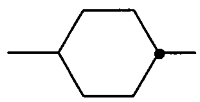 , 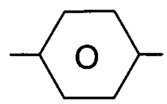

or

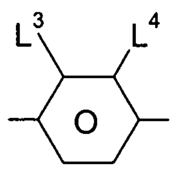

b denotes 0 or 1,

$L^3$ and $L^4$ each, independently of one another, denote H, F or Cl,

$R^3$ denotes alkenyl having 2 to 9 C atoms,

$R^4$ denotes alkyl having 1 to 12 C atoms, in which, in addi- tion, one or two non-adjacent $CH_2$ groups may be re- placed by -O-, -CH=CH-, -CO-, -OCO- or -COO- in such a way that O atoms are not linked directly to one another, or, if a = 0 and the ring B denotes cyclohexylene, $R^4$ also denotes $R^1$.

**13.** LC display according to one or more of Claims 1 to 12, **characterised in that** the LC medium additionally comprises one or more compounds of the formula VII

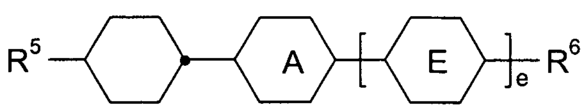

VII

in which

$R^5$ and $R^6$, independently of one another, have one of the mean- ings indicated for $R^1$ in formula II,

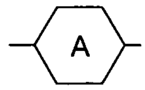

denotes

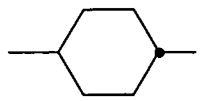

or

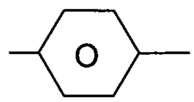

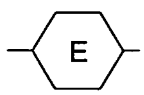

denotes

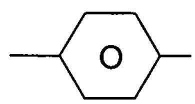

or

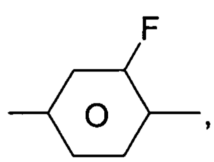,

and
e denotes 0 or 1.

**14.** LC medium as defined in one or more of Claims 1 to 13.

**15.** Racemate of a polymerisable compound containing a biaryl structural element of the formula I as defined in one or more of Claims 1 to 10.

**16.** Racemate of a polymerisable compound according to Claim 15, **characterised in that** it contains a biaryl structural element of the formula Ib

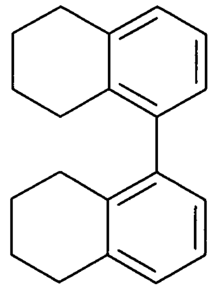

Ib

which is linked at at least one position to one or more polymerisable groups, optionally via an organic group or a spacer group, and in which A and B each, independently of one another, denote an aromatic or fully or partially saturated ring, where, in addition, in the individual rings, one or more CH groups may be replaced by N and/or one or more $CH_2$ groups may be replaced by O and/or S in such a way that O and/or S atoms are not linked directly to one another, and where the individual rings may also be mono- or polysubstituted.

**17.** Optically active polymerisable compound or racemate thereof, **characterised in that** it is selected from formula I1a1b

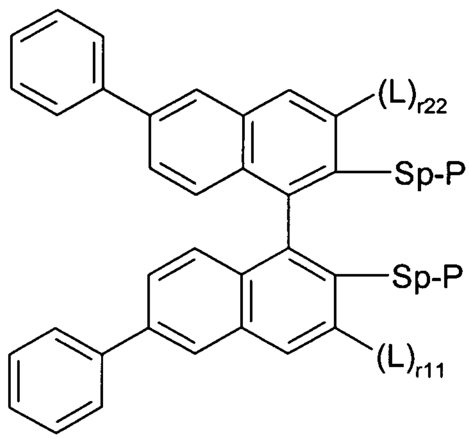

I1a1b

in which P, Sp, L, r11 and r22 have the meaning indicated in Claim 10.

**18.** Optically active polymerisable compound or racemate thereof, **characterised in that** it is selected from formula I1b1

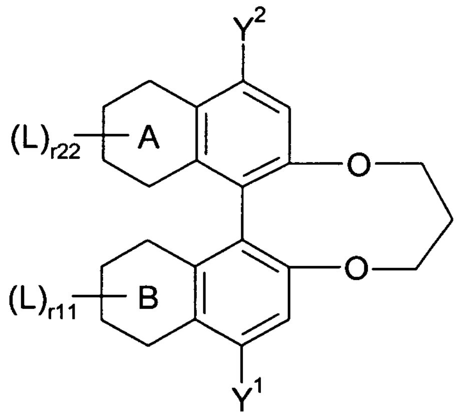

I1b1

in which A, B, L, r11 and r22 have the meaning indicated in Claim 10, and $Y^1$ and $Y^2$ contain or denote a polymerisable group.

**19.** LC display according to one or more of Claims 1 to 13, **characterised in that** it is a PSA-VA or PSA-OCB display.

**20.** LC medium comprising one or more polymerisable racemates or polymerisable or polymerised optically active compounds according to one or more of Claims 15 to 18.

**21.** Process for the preparation of an LC medium according to Claim 20, **characterised in that** one or more low-molecular-weight liquid-crystalline compounds are mixed with one or more polymerisable compounds according to one or more of Claims 15 to 18 and optionally with further liquid-crystalline compounds and/or additives.

**Revendications**

**1.** Affichage à cristaux liquides (LC) du type PSA (alignement soutenu par polymère), comprenant une cellule LC constituée de deux substrats, dans lequel au moins un substrat est transparent à la lumière et au moins un substrat a une couche d'électrode, et une couche d'un milieu LC de poids moléculaire faible comprenant un ou plusieurs composés polymérisés qui est située entre les substrats, dans lequel le(s) composé(s) polymérisé(s) peut (peuvent) être obtenu(s) par polymérisation d'un ou plusieurs composés polymérisables dans le milieu LC entre les substrats de la cellule LC avec application d'une tension électrique, **caractérisé en ce qu'**au moins un des composés polymérisables est un racémate de composés contenant un élément structurel biaryle de la formule I

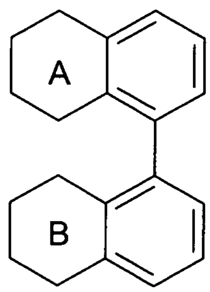

I

qui est lié en au moins une position à un ou plusieurs groupes polymérisables, optionnellement via un groupe organique ou un groupe espaceur, et dans lequel A et B chacun, indépendamment l'un de l'autre, représentent un cycle aromatique ou complètement ou partiellement saturé, où, en addition, dans les cycles individuels, un ou plusieurs groupes CH peuvent être remplacés par N et/ou un ou plusieurs groupes $CH_2$ peuvent être remplacés par O et/ou S de telle sorte que des atomes de O et/ou S ne soient pas liés directement l'un à l'autre, et où les cycles individuels peuvent également être mono- ou polysubstitués.

**2.** Affichage LC selon la revendication 1, **caractérisé en ce que** l'élément structurel de la formule I est lié en une ou plusieurs positions à un ou plusieurs groupes polymérisables via un groupe espaceur.

**3.** Affichage LC selon la revendication 1 ou 2, **caractérisé en ce que** l'élément structurel de la formule I est lié directement en une, deux ou plus de deux positions à un groupe polymérisable.

**4.** Affichage LC selon une ou plusieurs des revendications 1 à 3, **caractérisé en ce que** l'élément structurel de la formule I est choisi parmi les formules suivantes :

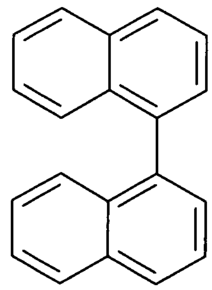

Ia

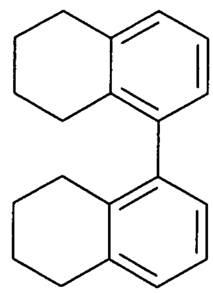

Ib

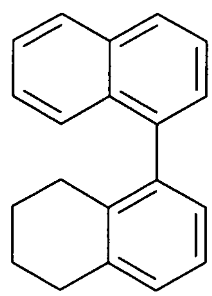

Ic

**5.** Affichage LC selon une ou plusieurs des revendications 1 à 4, **caractérisé en ce que** les composés polymérisables sont choisis parmi des racémates de la formule suivante:

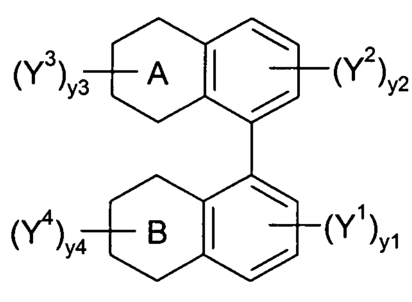

I1

dans laquelle

A et B chacun, indépendamment l'un de l'autre, représentent benzène, cyclohexane ou cyclohexène fusionné,
$Y^{1-4}$ chacun, indépendamment l'un de l'autre, représentent H, halogène, $SF_5$, $NO_2$, un groupe carbone ou un groupe hydrocarbone, dans lequel, en addition, un ou plusieurs des radicaux $Y^{1-4}$ sont capables de former un

systèmes de cycle aliphatique ou aromatique, mono- ou polycyclique et optionnellement condensé avec un radical adjacent $Y^{1-4}$ et/ou avec le squelette biaryle et dans lequel au moins un des radicaux $Y^{1-4}$ contient un groupe polymérisable P,
y1, y2 représentent 1, 2 ou 3, et
y3, y4 représentent 1, 2, 3 ou 4,

où, en addition, dans chacun des cycles dans la formule 11, un ou plusieurs groupes CH peuvent être remplacés par N et/ou un ou plusieurs groupes $CH_2$ peuvent être remplacés par O et/ou S de telle sorte que des atomes de O et/ou S ne soient pas liés directement l'un à l'autre.

6. Affichage LC selon une ou plusieurs des revendications 1 à 5, **caractérisé en ce que** les composés polymérisables sont choisis parmi des racémates des formules suivantes :

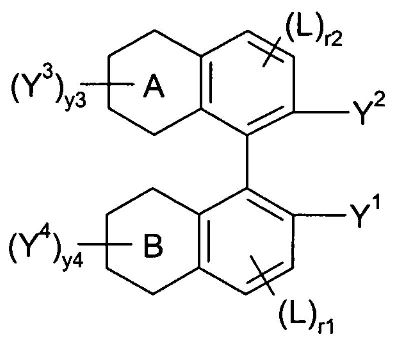

I1a

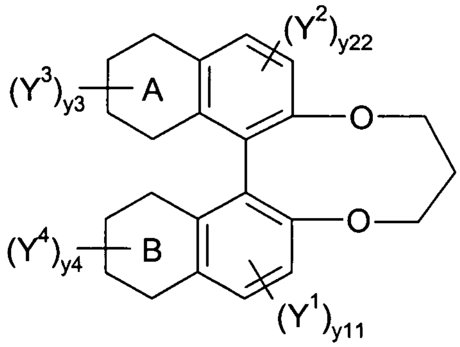

I1b

dans lesquelles A, B, $Y^{1-4}$, y3 et y4 ont la signification indiquée dans la revendication 5,
r1 et r2 chacun, indépendamment l'un de l'autre, représentent 0, 1 ou 2,
y11 et y22 chacun, indépendamment l'un de l'autre, représentent 0 ou 1,
L représente F, Cl, Br, I, -CN, $-NO_2$, -NCO, -NCS, -OCN, -SCN, $-C(=O)N(R^x)_2$, $-C(=O)Y^1$, $-C(=O)R^x$, $-N(R^x)_2$, silyle, aryle optionnellement substitué ayant 4 à 40, de préférence 6 à 20, atomes de C, et alkyle, alcoxy, alkylcarbonyle, alcoxycarbonyle, alkylcarbonyloxy ou alcoxycarbonyloxy en chaîne droite ou ramifié ayant 1 à 25 atomes de C, dans lequel un ou plusieurs atomes de H peuvent optionnellement être remplacés par F ou Cl,
$R^x$ représente H, halogène, une chaîne alkyle en chaîne droite, ramifiée ou cyclique ayant 1 à 25 atomes de C, dans lequel, en addition, un ou plusieurs atomes de C non adjacents peuvent être remplacés par -O-, -S-, -CO-, -CO-O-, -O-CO-, -O-CO-O- et dans lequel un ou plusieurs atomes de H peuvent être remplacés par fluor, ou un groupe aryle, aryloxy, hétéroaryle ou hétéroaryloxy optionnellement substitué ayant 5 à 40 atomes de C, et
$Y^1$ représente halogène.

7. Affichage LC selon une ou plusieurs des revendications 1 à 6, **caractérisé en ce que** les composés polymérisables sont choisis parmi des racémates des formules suivantes :

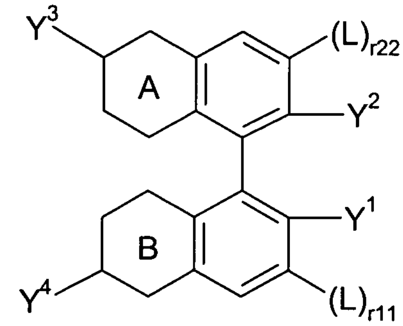

I1a1

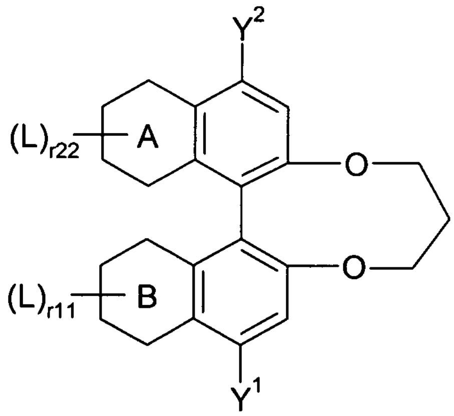

I1b1

dans lesquelles $Y^{1-4}$, L, A et B ont la signification indiquée dans les revendications 5 et 6, et r11 et r22 représentent 0 ou 1.

**8.** Affichage LC selon une ou plusieurs des revendications 5 à 7, **caractérisé en ce qu'**un ou plusieurs des radicaux $Y^{1-4}$ dans les composés des formules I1, I1a, I1a1, I1b et I1b1 représentent $-W-(Z^1-A^1)_{m1}-Sp-P$, dans lequel

W représente O, S, $CH_2$ ou une liaison simple,
Sp représente un groupe espaceur ou une liaison simple,
P représente un groupe polymérisable,
$A^1$ à chaque occurrence, de façon identique ou différente, représente aryle, hétéroaryle ou cycloalkyle complètement ou partiellement saturé ayant 4 à 20 atomes de C, qui est optionnellement substitué et dans lequel, en addition, un ou plusieurs groupes CH peuvent être remplacés par N et/ou un ou plusieurs groupes $CH_2$ peuvent être rempla- cés par O et/ou S de telle sorte que des atomes de O et/ou S ne soient pas liés directement l'un à l'autre,
$Z^1$ à chaque occurrence, de façon identique ou différente, représente -O-, -S-, -CO-, -COO-, -OCO-, -O-COO-, $-OCH_2-$, $-CH_2O-$, $-SCH_2-$, $-CH_2S-$, $-CF_2O-$, $-OCF_2-$, $-CF_2S-$, $-SCF_2-$, $-CH_2CH_2-$, $-CF_2CH_2-$, $-CH_2CF_2-$, $-CF_2CF_2-$, -CH=CH-, -CF=CF-, -C≡C-, -CH=CH-COO-, -OCO- CH=CH-, $CR^0R^{00}$ ou une liaison simple,
$R^0$ et $R^{00}$ chacun, indépendamment l'un de l'autre, représentent H ou alkyle ayant 1 à 4 atomes de C, et
M1 représente 0, 1, 2, 3 ou 4.

**9.** Affichage LC selon une ou plusieurs des revendications 5 à 8, **caractérisé en ce qu'**un ou plusieurs des radicaux $Y^{1-4}$ dans les composés des formules I1, I1a, I1a1, I1b et I1b1 représentent $-W-(Z^1-A^1)_{m1}-R^4$, dans lequel W, $Z^1$, $A^1$ et m1 ont la signification indiquée dans la revendication 8, et

$R^4$ à chaque occurrence, de façon identique ou différente, repré- sente H, L comme défini dans la revendication 4, alkyle en chaîne droite ou ramifié ayant 1 à 25 atomes de C, qui est non substitué ou mono- ou polysubstitué par F, Cl, Br, I, CN ou P-Sp- et dans lequel un ou plusieurs groupes $CH_2$ non adjacents peuvent chacun,

indépendamment l'un de l'autre, être remplacés par -C($R^x$)=C($R^x$)-, -C≡C-, -N($R^x$)-, -O-, -S-, -CO-, -CO-O-, -O-CO-, -O-CO-O- de telle sorte que des atomes de O et/ou S ne soient pas liés directement l'un à l'autre, où $R^x$ a la signification indiquée dans la revendication 5,

et, si tous les radicaux $Y^{1-4}$ représentent -W-($Z^1$-$A^1$)$_{m1}$-$R^4$, un ou plusieurs des radicaux $R^4$ contiennent au moins un groupe P-Sp-.

**10.** Affichage LC selon une ou plusieurs des revendications 1 à 9, **caractérisé en ce que** les composés polymérisables sont choisis parmi des racémates des formules suivantes:

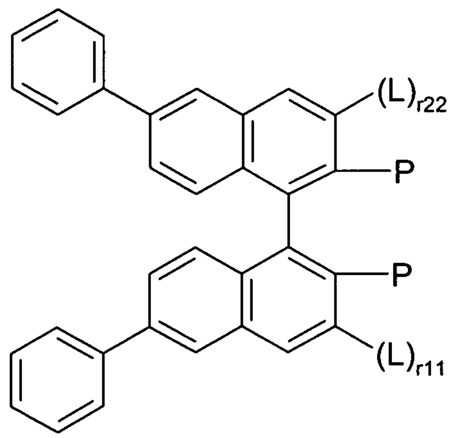

I1a1a

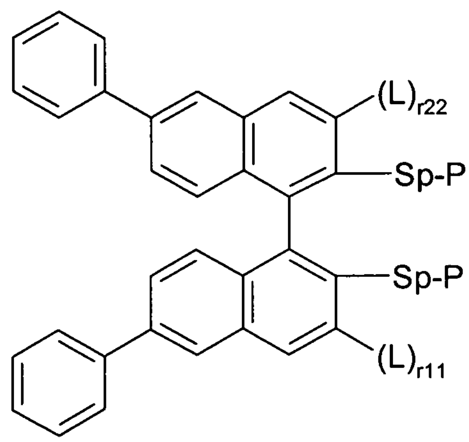

I1a1b

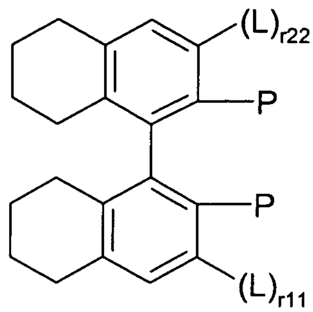

I1a1c

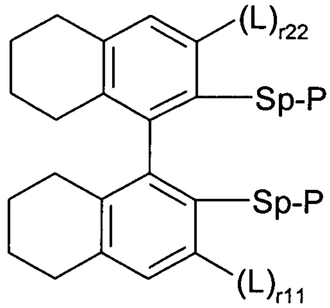

I1a1d

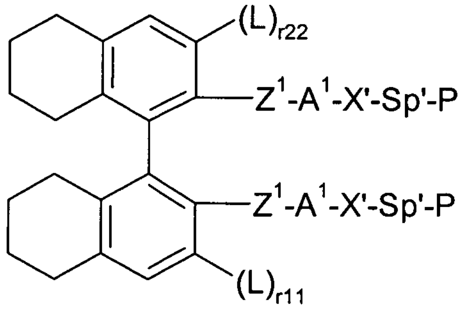

I1a1e

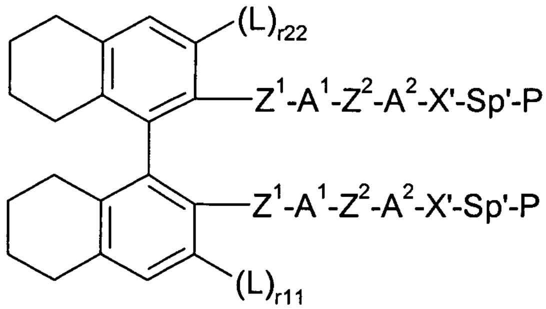

I1a1f

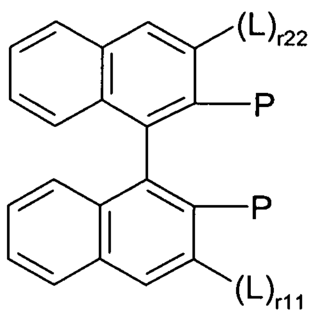

I1a1g

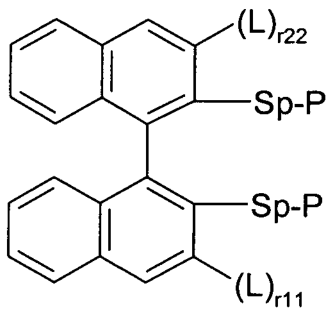

I1a1h

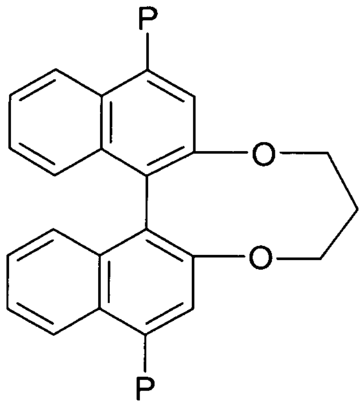

I1b1a

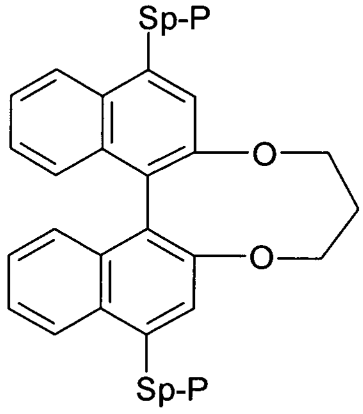

I1b1b

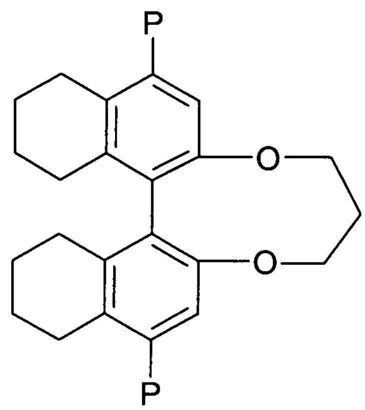

I1b1c

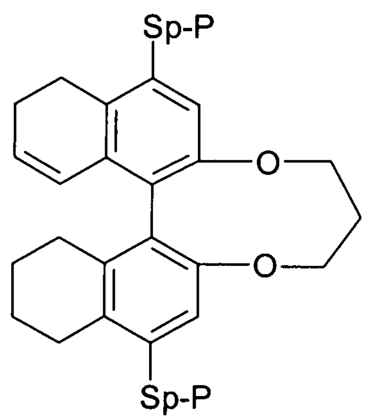

I1b1d

dans lesquelles $Y^3$, $Y^4$, P, Sp, $Z^1$, $A^1$, L, r11 et r22 à chaque occurrence, de façon identique ou différente, ont la signification indiquée dans les revendications 7 et 8, $Z^2$ et $A^2$ ont une des significations indiquées respectivement pour $Z^1$ et $A^1$,

Sp' représente alkylène ayant 1 à 20 atomes de C, qui est optionnellement mono- ou polysubstitué par F, Cl, Br, I ou CN et dans lequel, en addition, un ou plusieurs groupes $CH_2$ non adjacents peuvent chacun, indépendamment l'un de l'autre, être remplacés par -O-, -S-, -NH-, $-NR^0-$, $-SiR^0R^{00}-$, -CO-, -COO-, -OCO-, -OCO-O-, -S-CO-, -CO-S-, $-NR^0-CO-O-$, $-O-CO-NR^0-$, $-NR^0-CO-NR^0-$, -CH=CH- ou -C≡C- de telle sorte que des atomes de O et/ou S ne soient pas liés directement l'un à l'autre,
X' représente -O-, -S-, -CO-, -COO-, -OCO-, -O-COO-, $-CO-NR^0-$, $-NR^0-CO-$, $-NR^0-CO-NR^0-$, $-OCH_2-$, $-CH_2O-$, $-SCH_2-$, $-CH_2S-$, $-CF_2O-$, $-OCF_2-$, $-CF_2S-$, $-SCF_2-$, $-CF_2CH_2-$, $-CH_2CF_2-$, $-CF_2CF_2-$, -CH=N-, -N=CH-, -N=N-, $-CH=CR^0-$, $-CY^2=CY^3-$, -C≡C-, -CH=CH-COO-, -OCO-CH=CH- ou une liaison simple,
$R^0$ et $R^{00}$ chacun, indépendamment l'un de l'autre, représentent H ou alkyle ayant 1 à 12 atomes de C, et
$Y^2$ et $Y^3$ chacun, indépendamment l'un de l'autre, représentent H, F, Cl ou CN.

**11.** Affichage LC selon une ou plusieurs des revendications 1 à 10, **caractérisé en ce que** le milieu LC comprend un ou plusieurs composés de la formule II

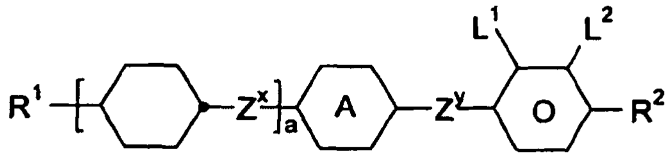

II

dans laquelle

$R^1$ et $R^2$ chacun, indépendamment l'un de l'autre, représentent alkyle ayant 1 à 12 atomes de C, dans lequel, en addition, un ou deux groupes $CH_2$ non adjacents peuvent être rem- placés par -O-, -CH=CH-, -CO-, -OCO- ou -COO- de telle sorte que des atomes de O ne soient pas liés directement l'un à l'autre,
$Z^x$ et $Z^y$ chacun, indépendamment l'un de l'autre, représentent $-CH_2CH_2-$, -CH=CH-, $-CF_2O-$, $-OCF_2-$, $-CH_2O-$, $-OCH_2-$, -COO-, -OCO-, $-C_2F_4-$, -CF=CF-, $-CH=CHCH_2O-$ ou une liaison simple,
$L^1$ et $L^2$ chacun, indépendamment l'un de l'autre, représentent F, Cl, $OCF_3$, $CF_3$, $CH_3$, $CH_2F$ ou $CHF_2$,
a représente 0 ou 1, et

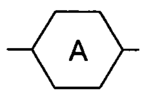

représente

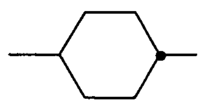

ou

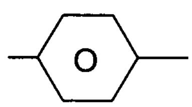

**12.** Affichage LC selon une ou plusieurs des revendications 1 à 11, **caractérisé en ce que** le milieu LC comprend additionnellement un ou plusieurs composés de la formule III

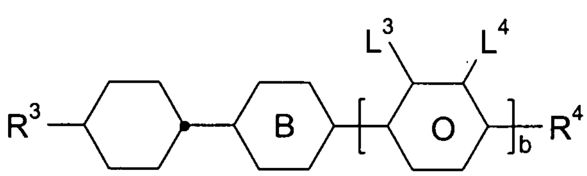

III

dans laquelle les radicaux individuels ont les significations suivantes :

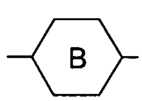

représente

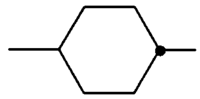
,
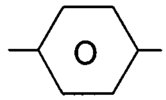

ou

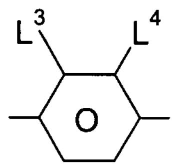

b représente 0 ou 1,
$L^3$ et $L^4$ chacun, indépendamment l'un de l'autre, représentent H, F ou Cl,
$R^3$ représente alkényle ayant 2 à 9 atomes de C,
$R^4$ représente alkyle ayant 1 à 12 atomes de C, dans lequel, en addition, un ou deux groupes $CH_2$ non adjacents peu- vent être remplacés par -O-, -CH=CH-, -CO-, -OCO- ou -COO- de telle sorte que des atomes de O ne soient pas liés directement l'un à l'autre, ou, si a = 0 et le cycle B représente cyclohexylène, $R^4$ représente également $R^1$.

**13.** Affichage LC selon une ou plusieurs des revendications 1 à 12, **caractérisé en ce que** le milieu LC comprend additionnellement un ou plusieurs composés de la formule VII

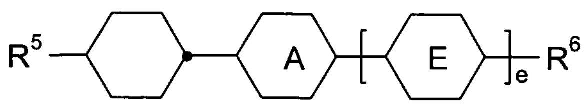
VII

dans laquelle

$R^5$ et $R^6$, indépendamment l'un de l'autre, ont une des significa- tions indiquées pour $R^1$ dans la formule II,

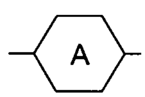

représente

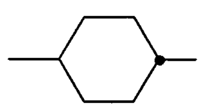

ou

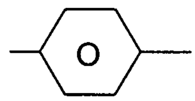

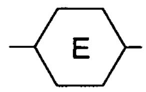

représente

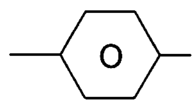

ou

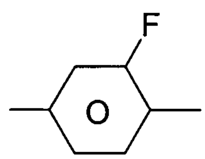
,

et
et
e représente 0 ou 1.

**14.** Milieu LC comme défini dans une ou plusieurs des revendications 1 à 13.

**15.** Racémate d'un composé polymérisable contenant un élément structurel biaryle de la formule I comme défini dans une ou plusieurs des revendications 1 à 10.

**16.** Racémate d'un composé polymérisable selon la revendication 15, **caractérisé en ce qu'**il contient un élément structurel biaryle de la formule Ib

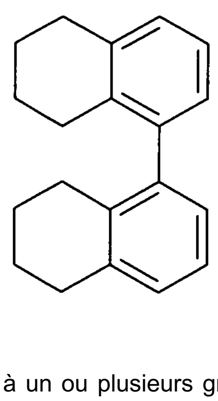
Ib

qui est lié en au moins une position à un ou plusieurs groupes polymérisables, optionnellement via un groupe organique ou un groupe espaceur, et dans lequel A et B chacun, indépendamment l'un de l'autre, représentent un cycle aromatique ou complètement ou partiellement saturé, où, en addition, dans les cycles individuels, un ou plusieurs groupes CH peuvent être remplacés par N et/ou un ou plusieurs groupes $CH_2$ peuvent être remplacés

par O et/ou S de telle sorte que des atomes de O et/ou S ne soient pas liés directement l'un à l'autre, et où les cycles individuels peuvent également être mono- ou polysubstitués.

**17.** Composé polymérisable optiquement actif ou racémate de celui-ci, **caractérisé en ce qu'**il est choisi parmi la formule I1a1b

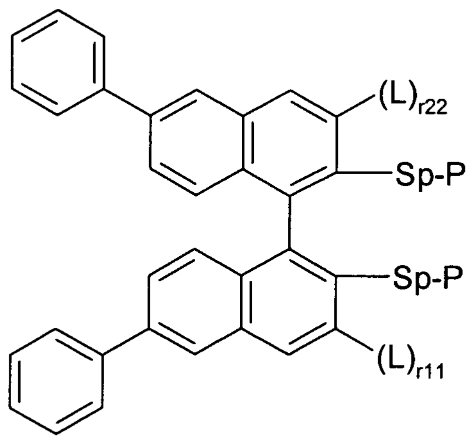

I1a1b

dans laquelle P, Sp, L, r11 et r22 ont la signification indiquée dans la revendication 10.

**18.** Composé polymérisable optiquement actif ou racémate de celui-ci, **caractérisé en ce qu'**il est choisi parmi la formule I1b1

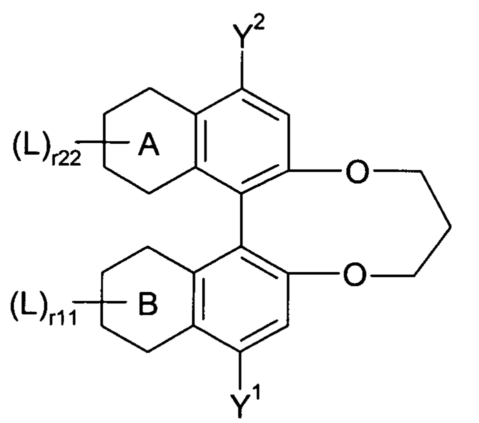

I1b1

dans laquelle A, B, L, r11 et r22 ont la signification indiquée dans la revendication 10, et $Y^1$ et $Y^2$ contiennent ou représentent un groupe polymérisable.

**19.** Affichage LC selon une ou plusieurs des revendications 1 à 13, **caractérisé en ce qu'**il est un affichage PSA-VA ou PSA-OCB.

**20.** Milieu LC comprenant un ou plusieurs racémates polymérisables ou composés optiquement actifs polymérisables ou polymérisés selon une ou plusieurs des revendications 15 à 18.

**21.** Procédé pour la préparation d'un milieu LC selon la revendication 20, **caractérisé en ce qu'**un ou plusieurs composés cristallins liquides à poids moléculaire faible sont mélangés avec un ou plusieurs composés polymérisables selon une ou plusieurs des revendications 15 à 18 et optionnellement avec d'autres composés cristallins liquides et/ou additifs.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 10036847 A
- EP 1170626 A2
- EP 1378557 A1
- EP 1498468 A1
- US 20040191428 A1
- US 20060066793 A1
- US 20060103804 A1
- US 2006054859 A1
- WO 0294805 A1
- WO 0234739 A
- US 2003006398 A1
- JP 2005015473 A
- EP 1215195 A
- US 5780629 A
- EP 1249483 A1
- WO 02034739 A1
- WO 02006195 A1
- WO 02094805 A1
- US 7060200 B1
- US 20060172090 A1
- DE 4342280 A1
- DE 19520704 A1
- GB 2328436 A
- GB 2398569 A
- GB 2298202 A
- EP 0964035 A1
- US 2005179005 A1
- JP 2001066431 A
- JP 2005170934 A
- JP 2005 A
- JP 171235 A
- EP 1306418 A1
- DE 10224046 A1
- DE 2209127 A
- DE 2240864 A
- DE 2321632 A
- DE 2338281 A
- DE 2450088 A
- DE 2637430 A
- DE 2853728 A
- EP 0364538 A
- EP 0122389 A
- DE 2636684 A
- DE 3321373 A


### In der Beschreibung aufgeführte Nicht-Patentliteratur


- **T.-J- CHEN et al.** *Jpn. J. Appl. Phys.,* 2006, vol. 45, 2702-2704
- **S. H. KIM ; L.-C- CHIEN.** *Jpn. J. Appl. Phys.,* 2004, vol. 43, 7643-7647
- *Pure Appl. Chem.,* 2001, vol. 73 (5), 888
- **C. TSCHIERSKE ; G. PELZL ; S. DIELE.** *Angew. Chem.,* 2004, vol. 116, 6340-6368
- **HALLER et al.** *Mol. Cryst. Liq. Cryst.,* 1973, vol. 24, 249-258
- Merck Liquid Crystals, Physical Properties of Liquid Crystals. Merck KGaA, November 1997
- **S. ZHENG ; D. Y. SOGAH.** *Polymer Preprints,* 2001, vol. 42 (1), 452-453
- **D.J. CRAM et al.** *J. Org. Chem.,* 1981, vol. 46, 393-406
- **M. NOJI ; M. NAKAJIMA ; K. KOGA.** *Tetrahedron Lett.,* 1994, vol. 35, 7983-7984
- **M. S. NEWMAN ; V. SANKARAN ; D. R. OLSON.** *J. Am. Chem. Soc.,* 1976, vol. 98, 3237-3241